# EUROPEAN PATENT APPLICATION

(11) **EP 4 265 644 A1**
(43) Date of publication of application: **25.10.2023**
(21) Application number: 21905000.2
(22) Date of filing: 25.06.2021
(51) Int. Cl.: C07K 16/46, C12N 15/13, C12P 21/00, A61K 39/395, A61P 35/00, A61P 35/02, A61P 37/02, A61P 31/12, A61P 25/28, A61P 13/12, A61P 19/08

(54) **BISPECIFIC ANTIBODY FOR SPECIFICALLY NEUTRALIZING TGF-? SIGNAL OF HELPER T CELL, AND PHARMACEUTICAL COMBINATION AND USE THEREOF**

(30) Priority: 18.12.2020 CN 202011502521
(71) Applicant: Shenzhen Majory Biotechnology., Ltd., Shenzhen, Guangdong 518101 (CN)
(72) Inventor: CHENG, Hanbing, Shenzhen, Guangdong 518101 (CN)
(74) Representative: Canzler & Bergmeier Patentanwälte Partnerschaft mbB
(86) International application number: PCT/CN2021/102428
(87) International publication number: WO 2022/127066

(57) **Abstract**

The present invention relates to the technical field of biology, and particularly relates to a bispecific antibody for specifically neutralizing a TGF-β signal of a helper T cell. The bispecific antibody at least comprises a first protein chain and a second protein chain, wherein the first protein chain comprises a part or all of the structure of a first protein functional region, and/or a part or all of the structure of a second protein functional region. The first protein functional region targets CD4 and the second protein functional region targets TGFβ1. The optimized anti-CD4/anti-TGF-β1 bifunctional antibody can efficiently neutralize a TGF-β signal of a helper T cell with high affinity, avidity and specificity. Specifically, the optimized anti-CD4/anti-TGF-β1 bifunctional antibody, represented in the form of IgG-(L)-ScFv, can inhibit the TGF-β signal pathway of the helper T cell with a higher neutralizing efficacy, and can achieve the same cancer inhibiting effect with a lower drug concentration, so that the bifunctional antibody becomes an ideal candidate for therapeutic application.

## Description

### FIELD OF DISCLOSURE

The present invention relates to biotechnology, in particular, to a bispecific antibody for specifically neutralizing TGF-β signal of helper T cell, and pharmaceutical composition and use thereof.

### DESCRIPTION OF RELATED ARTS

Tumors, especially malignant tumors, are a serious threat to human health nowadays, with a fatality ranking the second among all diseases. In recent years, with the deterioration of environment and increased stress in life, the incidence of malignant tumors has been scaled up year by year. Moreover, the treatment effects of malignant tumors are poor, for example, a high late-stage metastasis rate and a bleak prognosis. At present, conventional therapies such as radiotherapy, chemotherapy, targeted therapy, or surgery may alleviate the disease and prolong the survival period to a certain extent, but these methods still have major limitations, which are reflected not only in the therapeutic effects, but also in the failure to provide effective therapeutic schemes for patients with malignant tumors, such as pancreatic cancer.

TGF-β was first identified in 1981 (Robert et al., PNAS, 78: 5339-5343. (1981)). Transforming growth factor beta (TGF-β) is a multifunctional cytokine originally named for its ability to transform normal fibroblasts into cells that grow without anchorage dependence.

The TGF-β family generally includes TGF-β1, TGF-β2, and TGF-β3, where human TGF-β has a high degree of conservation compared with mouse TGF-β. Human TGF-β1 is only one amino acid different from mouse TGF-β1, and human TGF-β2 is three amino acids different from mouse TGF-β2. Human TGF-β3 is identical to mouse TGF-β3.

TGF-β with biological activity usually exists in the form of a dimer. After it binds to TGF-β Type II receptor (TGF-βRII) on the cell surface, it will further bind to TGF-β Type I receptor (TGF-βRI) nearby to form a hexamer. Subsequently, the activity of TGF-βRII serine/threonine kinase will be activated due to TGF-β binding. At this time, TGF-βRII will phosphorylate some key serine on TGF-βRI, thereby activating the serine/threonine kinase of TGF-βRI. This phosphorylation process will continue downstream to phosphorylate the adjacent regulatory SMAD (R-SMAD) which mainly includes SMAD2, 3, 9, etc., and is usually immobilized in the cell membrane by SARA protein. Phosphorylated R-SMAD shows a high affinity for cytoplasmic free Co-SMAD (such as SMAD4), forming a R-SAMD-Co-SMAD polymer. It will enter the nucleus, bind to DNA together with transcription factors, and initiate the expression of downstream genes (Sporn et al., Science, 233: 532 (1986)).

However, TGF-β does not directly act on the receptors on the cell surface. Under normal circumstances, only a small amount of free TGF-β can bind to receptors, and most TGF-β bind to latency-associated peptide (LAP) to result in no activity. There are four forms of TGF-β. Only when TGF-β-LAP polymer is cut by protease (secreted by tumor cells) in tumor microenvironment and in the form of a free state, TGF-β can be activated. A general review of TGF-β and its effects can be seen in the related literatures (Joan Massague, TGF-β in Cancer. Cell, 134 (2): 215-230 (2008)).

TGF-β is an important cytokine. TGF-β signal pathway regulates cell growth, differentiation, and apoptosis, and is an indispensable part for maintaining normal cellular functions. However, TGF-β can manipulate tumor microenvironment and has carcinogenicity. Malignant tumor cells secrete a large amount of TGF-β protein, which accelerates the growth and spread of cancer cells on the one hand, and makes cancer cells escape the attack from the immune system on the other hand.

TGF-β plays a dual role in the occurrence and development of tumors. It was found in the initial studies that TGF-β inhibits the proliferation of hematopoietic and epithelial cells and promotes apoptosis. Subsequent experiments have indicated that TGF-β negatively regulates the *in vitro* proliferation of various epithelial tumor cells (such as gastric cancer, lung cancer, colon cancer, liver cancer, kidney cancer, and prostate cancer), i.e., any protein mutation in its signaling pathway confers a selective growth advantage to tumor cells and lead to the occurrence of tumor. Due to its proliferation-inhibiting property, TGF-β has ever been considered the most promising drug target for cancer treatment. However, with further research, it has been found that TGF-β can promote tumor invasion and metastasis in tumor progression. Therefore, some scholars proposed that TGF-β is a "double-edged sword" for the occurrence and development of tumors (Yu Qingxiang, Research Progress of Transforming Growth Factor β and Tumor Metastasis, World Chinese Journal of Digestology, 14 (25): 2538-2541 (2016)).

The effect of TGF-β on tumor growth is reflected not only in the direct effect on tumor cells, but also in inhibiting the immune system to kill cancer cells. TGF-β can induce regulatory T cells (Treg) and inhibit effector T cells. In addition, TGF-β can directly act on helper T cells, B cells, NK cells, macrophages, and dendritic cells (DC), and inhibit their immune activity (Eduard Battle & Joan Massague, Transforming Growth Factor-β Signaling in Immunity and Cancer. Immunity, 50(4):924-940 (2019)).

Based on the role of TGF-β in tumor growth, many antibodies or small molecular compounds have been developed to inhibit the TGF-β signal pathway. However, in the past 20 years, none of these TGF-β-targeting drugs has achieved clinical success, where small-molecule chemical inhibitors targeting the TGF-β pathway have great toxicity, for example, some TGF-βRI small-molecule chemical inhibitors have strong cardiotoxicity. Compared with small-molecule chemical inhibitors, antibody molecules that neutralize TGF-β activity are not prone to penetrating into cells and show little clinical effectiveness although they have great advantages in safety.

CAT192 is a human IgG4 Monoclonal Antibody, which mainly neutralizes the activity of TGF-β1 and has no neutralization ability to TGF-β2 and TGF-β3. The antibody was originally isolated by Cambridge Antibody Technology (CAT) through phage display technology. In 2000, CAT signed a cooperation agreement with Genzyme Corporation in the United States to jointly develop TGF-β antibody. In 2004, CAT and Genzyme jointly announced that CAT192 was safe at all dosages in Phase I/II trials for the treatment of scleroderma, but showed no effectiveness. The failure in the clinical efficacy trial led to CAT192 being abandoned by Genzyme and replaced by another TGF-β neutralizing antibody (i.e., fresolimumab). At present, the clinical effectiveness studies of fresolimumab in the treatment of malignant tumors remain slow.

The diversity and complexity of TGF-β pathway regulation may bring about the failure of drug development of TGF-β pathway inhibitors. On the one hand, due to the dual effects of TGF-β on tumor cells and the heterogeneity of tumor cells, inhibiting TGF-β has little effect on tumor growth. On the other hand, TGF-β also has an inhibitory effect on immune cells, thereby helping tumor cells escape the surveillance of the immune system. The drugs that inhibit TGF-β can relieve the inhibition of TGF-β on immune cells to a certain extent, thereby inhibiting tumors, however, its effect may be compromised by the consumption of drugs by a large number of tumor cells.

TGF-β1 antibody is modified by genetic engineering to selectively inhibit the TGF-β signal pathway on helper T cell (CD4 positive T cell), thereby relieving the inhibition of TGF-β on immune cells and restoring their anti-tumor effect.

Bifunctional antibody, also known as bispecific antibody, is a specific antibody targeting two different antigens at the same time, which can be produced by immune sorting and purification. In addition, it can also be obtained by genetic engineering. Genetic engineering has comparable flexibility and advantages in the optimization of binding sites, evaluation of synthetic forms, and protein yield. At present, it has been proved that there are more than 45 kinds of bispecific antibody frameworks. Many developed bispecific antibodies are in the form of IgG-ScFv, that is, Morrison mode. The IgG-ScFv form, which is similar to the natural-occuring IgG format, has been verified to be ideal for bifunctional antibody for its advantages in antibody engineering, expression, and purification.

### SUMMARY OF THE PRESENT INVENTION

The inventor learned lessons from previous clinical failures of TGF-β antibodies. By designing an anti-CD4/anti-TGF-β1 bifunctional antibody, the antibody neutralizing TGF-β signal also targets the helper T cell (CD4 positive T cell), thereby achieving the anti-tumor function, which is not available for traditional TGF-β1 antibodies.

The present invention is intended to provide a bispecific antibody for specifically neutralizing TGF-β signal of helper T cell, and pharmaceutical composition and use thereof for addressing the deficiencies of existing technology. Through gene engineering technology, the TGF-β1 neutralizing antibody and immune cell-targeting antibody are integrated into a bifunctional antibody, thereby selectively blocking the TGF-β signal of the immune cells. This type of bifunctional antibody can fully eliminate the inhibition of TGF-β on immune cells, thereby achieving its anticancer functions to a greater extent.

To achieve the above and other related objects, a first aspect of the present invention provides a bispecific antibody. The bispecific antibody at least includes a first polypeptide chain and a second polypeptide chain,
wherein the first polypeptide chain includes partial or complete structure of a first function domain, and/or, partial or complete structure of a second function domain;
wherein the first function domain targets CD4, and is an anti-CD4 antibody or an antigen-binding fragment thereof,
wherein the second function domain targets transformation growth factor-β1 (TGF-β1), and is an anti-TGF-β1 antibody or an antigen-binding fragment thereof.

A second aspect of the present invention provides an isolated nucleic acid molecule that encodes the aforementioned bispecific antibody.

A third aspect of the present invention provides a vector, including the aforementioned isolated nucleic acid molecule.

A fourth aspect of the present invention provides a host cell, wherein the host cell includes the aforementioned isolated nucleic acid molecule, or the aforementioned vector.

A fifth aspect of the present invention provides a preparation method for the aforementioned bispecific antibody, including the following steps: culturing the host cell as described above under a condition suitable for expression of the bispecific antibody, and extracting the bispecific antibody from a cell culture.

A sixth aspect of the present invention provides a conjugation, including the bispecific antibody and a conjugate reagent, wherein the bispecific antibody is the aforementioned bispecific antibody, and the conjugate reagent is a detectable mark. Preferably, the conjugate reagent is selected from a chemical molecule, a fluorescent substance, a luminescent substance, a colored substance, and an enzyme.

A seventh aspect of the present invention provides a pharmaceutical composition, including a therapeutically effective amount of the aforementioned bispecific antibody, host cell, or conjugation. Optionally, the pharmaceutical composition also includes pharmaceutically acceptable excipients.

An eighth aspect of the present invention provides a use of the aforementioned bispecific antibody or the conjugation in the preparation of a medicament for prevention or treatment of a disease or disorder, wherein the disease or disorder is selected from the group consisting of cancer, immune-mediated diseases, fibrotic diseases, viral infectious diseases, neurodegenerative diseases, bone remodeling, kidney diseases, and a combination thereof.

A ninth aspect of the present invention provides the use of the aforementioned bispecific antibody or the conjugation in the preparation of a medicament for prevention or treatment of a malignant tumor, wherein the malignant tumor is selected from the group consisting of colon cancer, rectal cancer, lung cancer, kidney cancer, breast cancer, ovarian cancer, prostate cancer, bladder cancer, pancreatic cancer, gastrointestinal cancer, brain cancer, liver cancer, melanoma, blood cancer, and a combination thereof.

As described above, the bispecific antibody that selectively blocks TGF-β signal of helper T cells, pharmaceutical composition, and use thereof have the following beneficial effects:

the bispecific antibody in the present invention has different bispecific antibody combination forms and corresponding affinity, avidity, neutralizing efficacy, and anti-tumor effects. The bifunctional antibody which binds and neutralizes TGF-β1 with high affinity, avidity, and specificity disclosed in the present invention can efficiently block the TGF-β signal in helper T cells at lower serum concentration, thereby achieving better antitumor effects.

The bifunctional antibodies CT101, CT102, CT103, CT104, CT105, CT106, CT107, CT108, CT109, CT110, CT111, and CT112 in the present invention can efficiently and specifically bind to CD4 and TGF-β1, and can effectively block the interaction between TGF-β1 and TGF-βRII, thereby specifically eliminating the immunosuppression of TGF-β on helper T cells.

CT102 achieves the strongest inhibition effects on activation of the TGF-β signal in helper T cells, with an IC₅₀ up to 2 pM, followed by CT101, with an IC₅₀ up to 15 pM.

CT102 provided in the present invention is in a bispecific antibody form that has the strongest activity for neutralizing TGF-β1. The IC₅₀ of this anti-CD4/anti-TGF-β1 bispecific antibody in IgG-(L)-ScFv form reaches pM level, which is 10 or even 100 times more than that of bispecific antibodies in other forms.

Through the MC38 colon cancer xenograft tumor model, the inventor found that the anti-CD4/anti-TGF-β1 bifunctional antibody CT102 in the form of IgG-(L)-ScFv can achieve the best anticancer effect even at very low concentrations.

CT102 is capable of:
effectively binding the CD4 molecules on the surfaces of human helper T cells and relieving the immunosuppression of TGF-β of helper T cells;
effectively activating helper T cells, thereby activating a series of immune cells and inhibiting tumor growth;
having the potential of preparing drugs for preventing and treating malignant tumors such as liver cancer, lung cancer, breast cancer, pancreatic cancer, kidney cancer, cervical cancer, ovarian cancer, lymphoma, gastric cancer, colon cancer, and rectal cancer.

The bispecific antibody CT101 in the present invention can specifically bind to CD4 and helper T cells, additionally, CT101 can also bind to TGF-β1, and can effectively block the interaction between TGF-β1 and TGFβRII, thereby specifically relieving the immunosuppression of TGF-β on helper T cells. CT101 can be used for preparing drugs for preventing and treating malignant tumors such as liver cancer, lung cancer, breast cancer, kidney cancer, cervical cancer, ovarian cancer, lymphoma cancer, colon cancer, and rectal cancer.

### BRIEF DESCRIPTION OF THE DRAWINGS

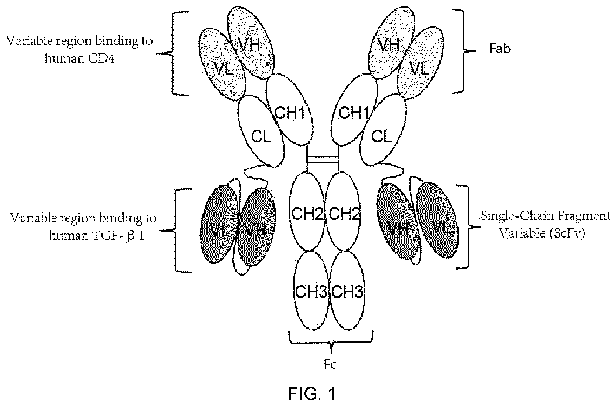

FIG. 1 shows a structural diagram of a bifunctional antibody CT102 in IgG-(L)-ScFv form.

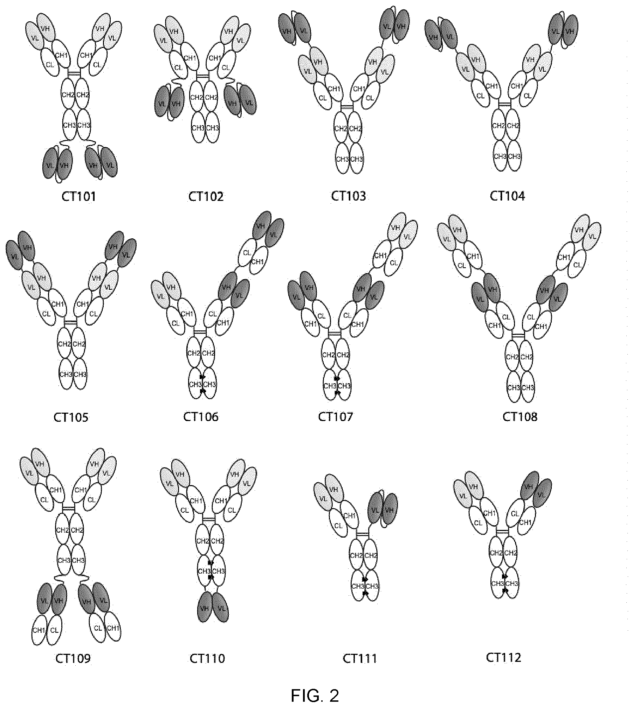

FIG. 2 shows a structural diagram of various bispecific antibody combination forms of the anti-CD4/anti-TGF-β1 bifunctional antibody.
FIG. 3 shows inhibitory effects of bivalent anti-TGF-β1 bifunctional antibody CT101 and monovalent anti-TGF-β1 bifunctional antibodies CT110, CT111, and CT112 on TGF-β signal pathway of HEK293 cells.
FIG. 4 shows inhibitory effects of bivalent anti-TGF-β1 bifunctional antibody CT101 and monovalent anti-TGF-β1 bifunctional antibodies CT110, CT111, and CT112 on TGF-β signal pathway of HEK293 cells with stable expression of human CD4 molecule.
FIG. 5 shows inhibitory effects of various bivalent anti-TGF-β1 bifunctional antibodies on TGF-β signal pathway of HEK293 cells with stable expression of human CD4 molecule.
FIG. 6 shows results of specific kinetic parameters of bivalent anti-TGF-β1 bifunctional antibodies CT101 and CT102, and monovalent anti-TGF-β1 bifunctional antibodies CT111 and CT112 when binding to CD4.
FIG. 7 shows results of specific kinetic parameters of bivalent anti-TGF-β1 bifunctional antibodies CT101 and CT102, and monovalent anti-TGF-β1 bifunctional antibodies CT111 and CT112 when binding to TGF-β1.

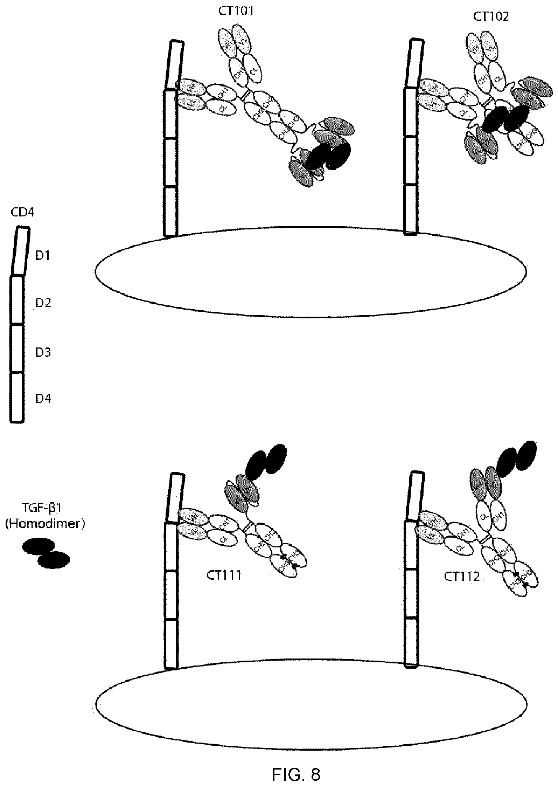

FIG. 8 shows a schematic diagram of CD4 and TGF-β1 binding of bivalent anti-TGF-β1 bifunctional antibodies CT101 and CT102, and monovalent anti-TGF-β1 bifunctional antibodies CT111 and CT112.
FIG. 9 shows a structural diagram of CT101, CT102, anti-TGF-β1 antibody, and control antibody.
FIG. 10 shows flow cytometry analysis results of binding of CT101, CT102, anti-TGF-β1 antibodies, and control antibody to CD4 T cells in humanized CD4 mice.
FIG. 11 shows pharmacokinetic assay results used to determine the half-life of the bifunctional antibodies CT101 and CT102 at a single dose of 200µg via tail vein injection.
FIG. 12 shows pharmacokinetic assay results used to determine the half-life of the bifunctional antibodies CT101 and CT102 at a single dose of 20µg via tail vein injection.
FIG. 13 shows inhibitory effect of treatment with 200µg per dose (4 doses in total, once every 5 days) of antibodies on MC38 colon cancer xenograft tumor, where the antibodies used for therapy include control antibody, anti-TGF-β1 antibody, and bifunctional antibodies CT101 and CT102.
FIG. 14 shows inhibitory effect of treatment with 20µg per dose (4 doses in total, once every 5 days) of antibodies on MC38 colon cancer xenograft tumor, where the antibodies used for therapy include control antibody, anti-TGF-β1 antibody, and bifunctional antibodies CT101 and CT102.
FIG. 15 shows inhibitory effects of different antibodies on EMT-6 breast cancer xenograft tumor, where the antibodies used for therapy include control IgG, anti-TGFβ antibody, anti-CD8/anti-TGFβ bifunctional antibody, anti-CD64/anti-TGFβ bifunctional antibody, and anti-CD4/anti-TGFβ bifunctional antibody.
FIG. 16 shows a structural diagram of the bifunctional antibody CT101 in IgG-ScFv form (Morrison mode).
FIG. 17 shows an amino acid sequence of the bifunctional antibody CT101 in IgG-ScFv form (Morrison mode), where the underlined amino acids correspond to CDR regions on the heavy or light chain of the antibody.
FIG. 18 shows SDS-PAGE analysis results of the bifunctional antibody CT101 (which is in IgG-ScFv form), where the left shows electrophoresis results of reduced protein, and the right shows electrophoresis results of non-reduced protein.
FIG. 19 shows high performance liquid chromatography (HPLC) analysis results of the bifunctional antibody CT101 (which is in IgG-ScFv form) after flowing through molecular sieve.
FIG. 20 shows results of specific kinetic parameters of the bifunctional antibody CT101 when binding to CD4.
FIG. 21 shows results of specific kinetic parameters of the bifunctional antibody CT101 when binding to TGFβ1.
FIG. 22 shows binding constants EC₅₀ of the bifunctional antibody CT101 and anti-TGF-β1 antibody CAT192 when binding to TGF-β1, which is obtained through indirect ELISA assay.
FIG. 23 shows binding constants EC₅₀ of the bifunctional antibody CT101 and anti-TGF-β1 antibody Ibalizumab when binding to CD4, which is obtained through indirect ELISA assay.
FIG. 24 shows activities of the bifunctional antibody CT101 and anti-TGF-β1 antibody CAT192 when competing with TGFβRII for binding to TGF-β1, which is obtained through competitive ELISA assay.
FIG. 25 shows binding constants EC₅₀ of the bifunctional antibody CT101 and anti-CD4 antibody Ibalizumab when binding to CD4 protein on the surface of HEK293-CD4 cells, which is obtained through FACS flow cytometry.
FIG. 26 shows inhibitory effects of the bifunctional antibody CT101 and anti-TGF-β1 antibody CAT192 on TGF-β signal pathway of HEK293 cells
FIG. 27 shows inhibitory effects of the bifunctional antibody CT101 and anti-TGF-β1 antibody CAT192 on TGF-β signal pathway of HEK293 cells with stable expression of human CD4 molecule
FIG. 28 shows inhibitory effects of different antibodies on MC38 colon cancer xenograft tumor, where the antibodies used for therapy include control IgG, anti-TGF-β1 antibody CAT192, anti-PD-L1 antibody atezolizumab, combination of atezolizumab with CAT192, and the bifunctional antibody CT101.

### DETAILED DESCRIPTION OF THE PREFERRED EMBODIMENTS

Embodiments of the present invention are illustrated by the following specific examples. Those skilled in the art can easily understand other advantages and effects of the present invention based on the contents disclosed by this description. The present invention may also be implemented or applied by other different embodiments, and the details of this description can also be modified or altered based on different viewpoints and applications without deviating from the spirit of the present invention.

Prior to further describing specific embodiments of the present invention, it should be understood that the protection scope of the present invention is not limited to the specific embodiments described below, it should also be understood that the terms used in embodiments of the present invention are intended for describing specific embodiments rather than limiting the protection scope of the present invention. In the description and claims of the present invention, the singular forms "one", "a", and "this" also include the plural forms unless otherwise specifically stated.

When a numerical range is given in the embodiments, it shall be understood that, unless otherwise stated in the present invention, the two endpoints of each numerical range and any value between the two endpoints can be selected. Unless otherwise defined, all technical and scientific terms used herein have the same meaning as commonly understood by one of ordinary skill in the art. In addition to the specific methods, equipment, and materials used in the embodiments, any methods, equipment, and materials similar or equivalent to those specified in the embodiments can be used to implement this present invention, based on the understanding of the prior art by those skilled in the art and the description of the present invention.

The bispecific antibody presented in an embodiment of the present invention includes at least a first polypeptide chain and a second polypeptide chain,
wherein the first polypeptide chain includes partial or complete structure of a first function region, and/or, partial or complete structure of a second function region,
wherein the first function region targets CD4, and is an anti-CD4 antibody or an antigen-binding fragment thereof,
wherein the second function region targets TGF-β1, and is an anti-TGF-β1 antibody or an antigen-binding fragment thereof.

The heavy chain variable region (VH) in the anti-CD4 antibody includes a variable heavy chain complementarity determining region (HCDR1), a variable heavy chain complementarity determining region 2 (HCDR2), and a variable heavy chain complementarity determining region 3 (HCDR3). The light chain variable region (VL) in the anti-CD4 antibody includes a variable light chain complementarity determining region (LCDR1), a variable light chain complementarity determining region 2 (LCDR2), and a variable light chain complementarity determining region 3 (LCDR3).

The heavy chain variable region (VH) of the first function region includes HCDR1-HCDR3 having the amino acid sequence shown as SEQ ID NO:53-SEQ ID NO:55, and the light chain variable region (VL) includes LCDR1-LCDR3 having the amino acid sequence shown as SEQ ID NO:56-SEQ ID NO:58.

The light chain variable region (VL) in the anti-TGF-β1 antibody includes a variable light chain complementarity determining region (LCDR1), a variable light chain complementarity determining region 2 (LCDR2), and a variable light chain complementarity determining region 3 (LCDR3), and the heavy chain variable region (VH) in the anti-TGF-β1 antibody includes a variable heavy chain complementarity determining region (HCDR1), a variable heavy chain complementarity determining region 2 (HCDR2), and a variable heavy chain complementarity determining region 3 (HCDR3).

The heavy chain variable region (VH) of the second function region includes HCDR1 having the amino acid sequence shown as SEQ ID NO:59, HCDR2 having the amino acid sequence shown as SEQ ID NO:60, and HCDR3 having the amino acid sequence shown as any one or more of SEQ ID NO:61-SEQ ID NO:63, and the light chain variable region (VL) includes LCDR1-LCDR3 having the amino acid sequence shown as SEQ ID NO:65-SEQ ID NO:67.

In some embodiments of the present invention, the anti-CD4 antibody or antigen-binding fragment thereof is selected from the group consisting of Fab, Fab', F(ab')2, Fd, Fv, dAb, complementarity determining region, ScFv, humanized antibody, chimeric antibody, and diabody.

In some embodiments of the present invention, the anti-TGF-β1 antibody or antigen-binding fragment thereof is selected from the group consisting of Fab, Fab', F(ab')2, Fd, Fv, dAb, complementarity determining region, ScFv, humanized antibody, chimeric antibody, and diabody.

In some embodiments of the present invention, the first function region is an immunoglobulin (IgG) including a VH and a VL, wherein the VH includes the amino acid sequence shown as SEQ ID NO:1, and the VL includes the amino acid sequence shown as SEQ ID NO:2.

In some embodiments of the present invention, the second function region is an ScFv or Fab including a VH and a VL, wherein the VH includes the amino acid sequence shown as any one of SEQ ID NO:3-5, and the VL includes the amino acid sequence shown as SEQ ID NO:6.

In some embodiments of the present invention, the first function region is the immunoglobulin (IgG) including a VH and a VL, wherein the VH includes the amino acid sequence shown as SEQ ID NO:76, and the VL includes the amino acid sequence shown as SEQ ID NO:78; and, the second function region is the ScFv or Fab including a VH and a VL, wherein the VH includes the amino acid sequence shown as SEQ ID NO:80, and the VL includes the amino acid sequence shown as SEQ ID NO:82.

In some embodiments of the present invention, the form of the bispecific antibody is not limited to IgG-ScFv/Fab, and the bispecific antibody may be in other bispecific antibody forms.

In some embodiments of the present invention, the number of the first and second function regions is independently 1, 2, or more.

In some embodiments of the present invention, the first and second function regions are directly connected or linked by a peptide linker. Preferably, the peptide linker is (GGGGS)m, wherein m is an integer, such as 1, 2, 3, 4, 5, or 6. Specifically, the peptide linker may be shown as SEQ ID NO:7.

In some embodiments of the present invention, the constant region of the immunoglobin is from human antibody. Preferably, the constant region of the immunoglobin is selected from the group consisting of constant regions of IgG1, IgG2, IgG3, and IgG4.

In some embodiments of the present invention, the first polypeptide chain, from N-terminal to C-terminal, has a formula of:
light chain variable region-light chain constant region-(peptide linker)m-heavy chain variable region-(peptide linker)n-light chain variable region;
and the second polypeptide chain, from N-terminal to C-terminal, has a formula of:
heavy chain variable region-heavy chain constant region-hinge-Fc region,
wherein the peptide linker is GGGGS, m is 0 or a positive integer, and n is 0 or a positive integer.

In some embodiments of the present invention, the light chain variable region-light chain constant region in the first polypeptide chain interacts with the heavy chain variable region-heavy chain constant region in the second polypeptide chain to form a polypeptide chain group, wherein two such polypeptide chain groups form a homodimer.

In some embodiments of the present invention, the VL at the N-terminal of the first polypeptide chain includes the amino acid sequence shown as SEQ ID NO:2, and the VH of the first polypeptide chain includes the amino acid sequence shown as any one of SEQ ID NO:3-5; and the VL at the C-terminal of the first polypeptide chain includes the amino acid sequence shown as SEQ ID NO:6, and the VH of the second polypeptide chain includes the amino acid sequence shown as SEQ ID NO:1.

In some embodiments of the present invention, the first polypeptide chain includes the amino acid sequence shown as SEQ ID NO:15, and the second polypeptide chain includes the amino acid sequence shown as SEQ ID NO:16.

In some embodiments of the present invention, the first polypeptide chain includes the amino acid sequence shown as SEQ ID NO: 13, and the second polypeptide chain includes the amino acid sequence shown as SEQ ID NO:14.

In some embodiments of the present invention, the first polypeptide chain includes the amino acid sequence shown as SEQ ID NO: 17, and the second polypeptide chain includes the amino acid sequence shown as SEQ ID NO:18.

In some embodiments of the present invention, the first polypeptide chain includes the amino acid sequence shown as SEQ ID NO:19, and the second polypeptide chain includes the amino acid sequence shown as SEQ ID NO:20.

In some embodiments of the present invention, the first polypeptide chain includes the amino acid sequence shown as SEQ ID NO:21, and the second polypeptide chain includes the amino acid sequence shown as SEQ ID NO:22.

In some embodiments of the present invention, the first polypeptide chain includes the amino acid sequence shown as SEQ ID NO:23, and the second polypeptide chain includes the amino acid sequence shown as SEQ ID NO:24. The bispecific antibody further includes a third polypeptide chain and a fourth polypeptide chain, where the third polypeptide chain includes the amino acid sequence shown as SEQ ID NO:25, and the fourth polypeptide chain includes the amino acid sequence shown as SEQ ID NO:26.

In some embodiments of the present invention, the first polypeptide chain includes the amino acid sequence shown as SEQ ID NO:27, and the second polypeptide chain includes the amino acid sequence shown as SEQ ID NO:28. The bispecific antibody further includes a third polypeptide chain and a fourth polypeptide chain, where the third polypeptide chain includes the amino acid sequence shown as SEQ ID NO:29, and the fourth polypeptide chain includes the amino acid sequence shown as SEQ ID NO:30.

In some embodiments of the present invention, the first polypeptide chain includes the amino acid sequence shown as SEQ ID NO:31, and the second polypeptide chain includes the amino acid sequence shown as SEQ ID NO:32. The bispecific antibody further includes a third polypeptide chain, where the third polypeptide chain includes the amino acid sequence shown as SEQ ID NO:33.

In some embodiments of the present invention, the first polypeptide chain includes the amino acid sequence shown as SEQ ID NO:34, and the second polypeptide chain includes the amino acid sequence shown as SEQ ID NO:35. The bispecific antibody further includes a third polypeptide chain, where the third polypeptide chain includes the amino acid sequence shown as SEQ ID NO:36.

In some embodiments of the present invention, the first polypeptide chain includes the amino acid sequence shown as SEQ ID NO:37, and the second polypeptide chain includes the amino acid sequence shown as SEQ ID NO:38. The bispecific antibody further includes a third polypeptide chain, where the third polypeptide chain includes the amino acid sequence shown as SEQ ID NO:39.

In some embodiments of the present invention, the first polypeptide chain includes the amino acid sequence shown as SEQ ID NO:40, and the second polypeptide chain includes the amino acid sequence shown as SEQ ID NO:41. The bispecific antibody further includes a third polypeptide chain, where the third polypeptide chain includes the amino acid sequence shown as SEQ ID NO:42.

In some embodiments of the present invention, the first polypeptide chain includes the amino acid sequence shown as SEQ ID NO:43, and the second polypeptide chain includes the amino acid sequence shown as SEQ ID NO:44. The bispecific antibody further includes a third polypeptide chain and a fourth polypeptide chain, where the third polypeptide chain includes the amino acid sequence shown as SEQ ID NO:45, and the fourth polypeptide chain includes the amino acid sequence shown as SEQ ID NO:46.

The bispecific antibody in some embodiments of the present invention binds to human CD4 and TGF-β1 molecules selectively.

In some embodiments of the present invention, the constant region of the immunoglobin is from human antibody; preferably, the constant region of the immunoglobin is selected from the group consisting of IgG1, IgG2, IgG3, and IgG4.

In some embodiments of the present invention, the constant region of heavy chain of the immunoglobulin is human IgG1 chain C region or human IgG4 chain C region, and the constant region of light chain is human Ig kappa chain C region.

In some embodiments of the present invention, the constant region of the immunoglobin is humanized, for example, the constant region of heavy chain is IgG4 chain C region, ACCESSION:P01861, and the constant region of light chain is Ig kappa chain C region, ACCESSION:P01834.

In some embodiments of the present invention, the constant region of light chain includes the amino acid sequence shown as SEQ ID NO:9.

In some embodiments of the present invention, the constant region of heavy chain includes the amino acid sequence shown as SEQ ID NO:10.

In some embodiments of the present invention, the hinge region includes the amino acid sequence shown as SEQ ID NO:11.

In some embodiments of the present invention, the Fc region includes the amino acid sequence shown as SEQ ID NO:12.

In some embodiments of the present invention, the first and second function regions are directly connected or linked by a peptide linker.

Preferably, the peptide linker is (GGGGS)m, wherein m is an integer, such as 1, 2, 3, 4, 5, or 6, and GGGGS (SEQ ID NO: 8) is a constituent unit of the peptide linker.

In some embodiments of the present invention, the number of the first and second function regions is independently 1, 2, or more.

In some embodiments of the present invention, the number of the immunoglobulin is 1, the number of the ScFv is 2, and preferably the two ScFvs are identical.

In some embodiments of the present invention, the immunoglobulin is IgG, IgA, IgD, IgE, or IgM, preferably, is IgG, such as IgG1, IgG2, IgG3, or IgG4.

In some embodiments of the present invention, the ScFv is linked to the C-terminal of heavy chain of immunoglobulin. Since immunoglobulin has two heavy chains, one immunoglobulin molecule is linked with two ScFv molecules. Preferably, the two ScFvs are identical.

In some embodiments of the present invention, there are two ScFvs, one end of each ScFv is linked to the C-terminal or N-terminal of the two heavy chains of the immunoglobulin, respectively.

In some embodiments of the present invention, a disulfide bond exists between the VH and VL of ScFv. The method for introducing disulfide bonds between VH and VL of an antibody is well known in the field, such as United States Patent Application US5747654; Rajagopal et al., Prot.Engin.10(1997)1453-1459; Reiter et al., Nature Biotechnology 14(1996)1239-1245; Webber et al., Molecular Immunology 32(1995)249-258; Reiter et al., Immunity 2(1995)281-287; Reiter et al., JBC 269(1994)18327-18331; Reiter et al., Inter. J. of Cancer 58(1994)142-149; or Reiter et al., Cancer Res. 54(1994)2714-2718; which are incorporated into this application by reference.

In some embodiments of the present invention, the bispecific antibody binds to human CD4 and/or TGF-β1 with a Kd less than 10⁻⁹ M, the Kd value is acquired through the Biacore Molecular Interaction System.

In some embodiments of the present invention, the bispecific antibody neutralizes human TGF-β1 with an IC₅₀ less than 5 nM, where IC₅₀ value is acquired through TGF-β1 luciferase assay.

In some embodiments of the present invention, the bispecific antibody neutralizes human TGF-β1 with the IC₅₀ less than 5 pM, where IC₅₀ value is acquired through TGF-β1 luciferase assay.

Another aspect of the present invention relates to an isolated nucleic acid molecule, encoding any of the bispecific antibodies of the present invention.

The present invention also provides a vector, including the isolated nucleic acid molecule in the present invention.

The present invention also provides a host cell, including the isolated nucleic acid molecule in the present invention, or the vector in the present invention.

Another aspect of the present invention relates to a method for preparing the bispecific antibody in the present invention, including culturing the host cell as described above under a suitable condition, and recovering the bispecific antibody from a cell culture.

Another aspect of the present invention provides a conjugation, including the bispecific antibody and a conjugate reagent, wherein the bispecific antibody is any bispecific antibody in the present invention, and the conjugate reagent is a detectable mark. Preferably, the conjugate reagent is a small molecule compound, a fluorescent substance, a luminescent substance, a colored substance, or an enzyme.

Another aspect of the present invention provides a pharmaceutical composition, including any bispecific antibody or the conjugation in the present invention, wherein the pharmaceutical composition also includes pharmaceutically acceptable excipients.

The bispecific antibody or pharmaceutical composition in the present invention can be formulated into any dosage form known in the pharmaceutical field, such as tablets, pills, suspensions, emulsions, solutions, gels, capsules, powders, granules, elixirs, lozenges, suppositories, injections (including injection liquid, sterile powders for injection, and concentrated solutions for injection), inhalants, and sprays. The preferred dosage form depends on the intended mode of administration and therapeutic use. The pharmaceutical composition in the present invention should be sterile and stable under production and storage conditions. A preferred dosage form is injection. Such injection may be a sterile injection solution. For example, sterile injection solutions can be prepared by the following methods: adding the necessary dose of the bispecific antibody in the present invention in a suitable solvent, optionally adding other desired ingredients (including but not limited to, PH modulators, surfactants, adjuvants, ionic strength enhancers, isotonic agents, preservatives, diluents, or any combination thereof), and filtrating and sterilizing. In addition, the sterile injection solution may be prepared as a sterile freeze-dried powder (e.g. by vacuum drying or freeze-drying) for easy storage and use. Such sterile freeze-dried powders can be dispersed in suitable carriers (such as sterile and pyrogen-free water) prior to use. In addition, the bispecific antibody in the present invention may present as a unit dose in the pharmaceutical composition to facilitate administration. In certain embodiments, the unit dose is at least 1mg, at least 5mg, at least 10mg, at least 15mg, at least 20mg, at least 25mg, at least 30mg, at least 45mg, at least 50mg, at least 75mg, or at least 100mg. When the pharmaceutical composition is in liquid (e.g. an injection liquid) dosage form, it may include the bispecific antibody in the present invention at a concentration of at least 0.1 mg/ml, such as at least 0.25 mg/ml, at least 0.5 mg/ml, at least 1mg/ml, at least 2.5 mg/ml, at least 5mg/ml, at least 8mg/ml, at least 10mg/ml, at least 15mg/ml, at least 25mg/ml, at least 50mg/ml, at least 75mg/ml, or at least 100mg/ml.

Bispecific antibodies or pharmaceutical compositions in the present invention may be administered by any suitable method known in the field, including but not limited to, oral administration, oral cavity, sublingual, eyeball, local, parenteral, rectal, intrathecal, intracisternal, groin, bladder, local (e.g., powder, ointment, or drops), or nasal cavity. However, for many therapeutic uses, the preferred route/mode of administration is parenteral administration (e.g. intravenous, subcutaneous, intraperitoneal, intramuscular injection). The technical persons shall understand that the route and/or method of administration varies depending on the intended purpose. In a preferred embodiment, the bispecific antibodies or pharmaceutical compositions in the present invention are administered by intravenous infusion or injection.

The bispecific antibodies or pharmaceutical compositions in the present invention can be used alone or in combination, and can also be used in combination with other pharmaceutically active agents (such as tumor chemotherapy drugs). These additional pharmaceutically active agents can be administered before, simultaneously, or after administration of the bispecific antibody or pharmaceutical composition of the present invention.

In the present invention, the administration regimen can be adjusted to obtain the optimal intended response (e.g. therapeutic or prophylactic response). For example, a single dose, multiple doses in a period of time, or reduced or increased doses proportioned to the urgency of the treatment situation may be adopted.

Another aspect of the present invention provides a use of the aforementioned bispecific antibody or the conjugation of the present invention in the preparation of drugs for preventing and/or treating diseases or disorders, where the diseases or disorders are selected from the group consisting of cancer, immune-mediated diseases, fibrotic diseases, viral infectious diseases, neurodegenerative diseases, bone remodeling, kidney diseases, and a combination thereof.

Another aspect of the present invention provides a use of the aforementioned bispecific antibody or the conjugation of the present invention in the preparation of drugs for preventing and/or treating malignant tumor, where the tumor is selected from the group consisting of colon cancer, rectal cancer, lung cancer (such as Non-small-cell carcinoma), liver cancer, ovarian cancer, skin cancer, glioma, melanoma, kidney tumor, prostate cancer, pancreatic cancer, bladder cancer, gastrointestinal cancer, breast cancer, brain cancer, and leukemia.

Another aspect of the present invention provides a method of preventing and/or treating malignant tumors, including administering an effective amount of the aforementioned bispecific antibody or the conjugation in the present invention to a subject in need thereof, wherein the malignant tumors are selected from the group consisting of colon cancer, rectal cancer, lung cancer, liver cancer, ovarian cancer, skin cancer, glioma, melanoma, kidney cancer, prostate cancer, bladder cancer, gastrointestinal cancer, breast cancer, brain cancer, and leukemia.

Typical non-limiting ranges for therapeutically or prophylactically effective amounts of bispecific antibodies in the present invention are 0.02-50mg/kg, e.g. 0.1-50 mg/kg, 0.1-25mg/kg, or 1-10mg/kg. It should be noted that the dosage may vary depending on the type and severity of the symptoms to be treated. Additionally, those skilled in this art understand that for any particular patient, a particular administration regimen should be adjusted over time based on patient needs and professional evaluation provided by a physician. The dosage ranges given herein are for illustrative purposes only and do not limit the use or scope of the pharmaceutical compositions in the present invention.

In the present invention, the subjects may be rodents, such as mice, or primates, such as human and cynomolgus monkeys.

Any bispecific antibody or conjugation in the present invention may be used for prevention or treatment of malignant tumor, where the malignant tumor is selected from the group consisting of colon cancer, rectal cancer, lung cancer, liver cancer, ovarian cancer, melanoma, kidney cancer, pancreatic cancer, skin cancer, glioma, prostate cancer, bladder cancer, gastrointestinal cancer, breast cancer, brain cancer, and leukemia.

Therapeutic antibody, especially monoclonal antibody (mAb) has achieved favorable curative effects in the treatment of many diseases. The traditional experimental methods to obtain these therapeutic antibodies are to immunize animals with antigens, obtain antigen-targeting antibodies in immunized animals, and improve antibodies with low affinity to antigens by affinity maturation methods. However, these methods require a lot of time and effort, and most of the time the specific epitopes on the antigen may not be targeted by the antibodies.

The variable regions of light chain and heavy chain determine the binding of antigens, and the variable region of each chain includes three highly variable regions, which are known as complementarity determining regions (CDRs). CDRs of heavy chain (H) include HCDR1, HCDR2, and HCDR3, and CDRs of light chain (L) includes LCDR1, LCDR2, and LCDR3, which are named by Kabat et al., seen in Sequences of Proteins of Immunological Interest, Fifth Edition (1991), vol. 1-3, NIH Publication 91-3242, Bethesda Md.

The amino acid sequence of the CDR region of the monoclonal antibody sequence in (1)-(13) below is analyzed by means of techniques well known to those skilled in the art, for example through the VBASE2 database. The results are as follows:
(1) The first function region of anti-CD4/anti-TGF-β1 bifunctional antibody:
   the VH includes the amino acid sequence shown as SEQ ID NO:1, and the VL includes the amino acid sequence shown as SEQ ID NO:2.

The three VH CDRs include the amino acid sequences as follows:
HCDR1:GYTFTSYVIH(SEQ ID NO:53),
HCDR2:YINPYNDGTDYDEKFKG(SEQ ID NO:54),
HCDR3:EKDNYATGAWFA (SEQ ID NO:55).

The three VL CDRs include the amino acid sequences as follows:
LCDR1:KSSQSLLYSTNQKNYLA(SEQ ID NO:56),
LCDR2:WASTRES(SEQ ID NO:57),
LCDR3:QQYYSYRT (SEQ ID NO:58).

(2) The second function region of anti-CD4/anti-TGF-β1 bifunctional antibody:
the VH includes the amino acid sequence shown as any one of SEQ ID NO:3-5, and the VL includes the amino acid sequence shown as SEQ ID NO:6.

The three VH CDRs include the amino acid sequences as follows:
HCDR1:SYGMH(SEQ ID NO:59),
HCDR2:VISYDGSIKYYADSVKG(SEQ ID NO:60),
HCDR3:TGEYSGYDTDPQYS(SEQ ID NO:61) or TGEYSGYDTSGVEL(SEQ ID NO:62) or TGFYSGYDTPASPD(SEQ ID NO:63),
HCDR3 consensus sequence: TGX1YSGYDTX2X3X4X5X6(SEQ ID NO:64),
wherein X1 may be any amino acid (preferably E or F).

The three VL CDRs include the amino acid sequences as follows:
LCDR1:RSSQGIGDDLG(SEQ ID NO:65),
LCDR2:GTSTLQS(SEQ ID NO:66),
LCDR3:LQDSNYPLT(SEQ ID NO:67).

In the present invention, unless otherwise stated, the scientific and technical terms used herein have meanings commonly understood by those skilled in the art. Moreover, the laboratory procedures of cell culture, molecular genetics, nucleic acid chemistry, and immunology used in the present invention are all routine steps widely used in corresponding fields. Meanwhile, in order to better understand the present invention, definitions, and explanations of related terms are provided below.

As used herein, when referring to the amino acid sequence of the TGF-β1 protein (GenBank ID: NP_000651.3), it includes the full length of the TGF-β1 protein. However, those skilled in the art understand that mutations or variations (including but not limited to substitutions, deletions, and/or additions) may occur naturally or artificially in the amino acid sequence of the TGF-β1 protein without affecting its biological function. Thereby, in the present invention, the term "TGF-β1 protein" includes all such sequences, including natural or artificial variants thereof. In one embodiment of the present invention, the amino acid sequence of the TGF-β1 protein is shown in the underlined portion of SEQ ID NO: 51.

As used herein, when referring to the amino acid sequence of the CD4 protein (cluster differentiation 4, NCBI GenBank:NP_000607.1), it includes the full length of the CD4 protein, and also includes the extracellular fragment of CD4, i.e., the soluble CD4 molecule (soluble CD4, sCD4). However, it will be understood by those technical persons in the field that mutations or variations (including but not limited to substitutions, deletions, and/or additions) may occur naturally or artificially in the amino acid sequence of the CD4 protein without affecting its biological function. Thereby, in the present invention, the term "CD4 protein" includes all such sequences, including natural or artificial variants thereof. In one embodiment of the present invention, the amino acid sequence of the extracellular fragment sCD4 of CD4 is shown in the underlined portion of SEQ ID NO: 49.

As used herein, the term EC₅₀ refers to concentration for 50% of maximal effect, which means the concentration causing 50% of maximal effect.

As used herein, the term IC₅₀ refers to concentration for 50% of maximal inhibition, which means the concentration causing 50% of maximal inhibition.

As used herein, the term "antibody" refers to an immunoglobulin molecule that typically includes two pairs of polypeptide chains (each pair has a "light" (L) chain and a "heavy" (H) chain). Generally speaking, a heavy chain can be understood as a polypeptide chain with a larger molecular weight in an antibody, while a light chain refers to a polypeptide chain with a smaller molecular weight in an antibody. Light chains can be classified into κ and λ types. The heavy chains can generally be classified into µ, δ, γ, α, or ε, and the isotypes of antibodies are defined as IgM, IgD, IgG, IgA, and IgE, respectively. Within the light and heavy chains, the variable and constant regions are connected by a "J" region with about 12 or more amino acids, and the heavy chain also contains a "D" region with about 3 or more amino acids. Each heavy chain includes a VH and CH. The CH includes three domains (CH1, CH2, and CH3). Each light chain includes a VL and a CL. The CL includes a domain. The constant regions of the antibody can mediate the binding of immunoglobulins to host tissues or factors, including various cells of the immune system (e.g. effector cells) and the first component (C1q) of the classical complement system. The VH and VL can also be subdivided into highly variable regions (known as complementarity determination regions (CDR)) interspersed with more conservative regions known as framework regions (FR). Each VH and VL includes three CDRs and four FRs arranged from amino terminal to carboxyl terminal in the following order: FR1, CDR1, FR2, CDR2, FR3, CDR3, FR4. The variable regions of each heavy chain/light chain pair form antibody binding sites respectively. The distribution of amino acids to each region or domain follows definitions in Kabat Sequences of Proteins of Immunological Interest (National Institutes of Health, Bethesda,Md. (1987 and 1991)), Chothia&Lesk (1987) J.Mol.Biol.196:901-917, or Chothia et al., (1989) Nature 342:878-883. In particular, the heavy chain may also include more than 3 CDRs, for example, 6, 9, or 12 CDRs. For example, in the bifunctional antibody in the present invention, the heavy chain may include the heavy chain of the IgG antibody with its C-terminal linking to ScFv of another antibody, in which the heavy chain includes 9 CDRs. The term "antibody" is not limited by any particular method for producing antibodies. For example, it includes, in particular, recombinant antibodies, monoclonal antibodies, and polyclonal antibodies. The antibodies may be antibodies with different isotypes, such as IgG (e.g. IgG1, IgG2, IgG3, or IgG4 subtypes), IgA1, IgA2, IgD, IgE, or IgM antibodies.

As used herein, the term "antigen-binding fragment" of an antibody refers to a polypeptide including a fragment of a full-length antibody that retains the ability to specifically bind to the same antigen to which the full-length antibody binds, and/or competes with the full-length antibody for specific binding to the antigen, which is also referred to as an "antigen-binding part". Please refer to Fundamental Immunology, Ch.7 (Paul,W.,ed., 2nd Edition, Raven Press,N.Y.(1989), which is incorporated herein by reference as an entirety for all purposes. Antigen-binding fragments of antibodies can be produced by recombinant DNA techniques or by enzymatic or chemical cleavage of intact antibodies. In some cases, antigen-binding fragments include Fab, Fab', F(ab')2, Fd, Fv, dAb, and complementary determining region (CDR) fragments, ScFv, chimeric antibody, diabody, or polypeptides including at least a portion of antibodies sufficient to impart specific antigen binding ability to the polypeptide.

As used herein, the term "Fd fragment" means an antibody fragment including VH and CH domains, the term "Fv fragment" means an antibody fragment including single VL and VH domains of the antibody, the term "dAb fragment" means an antibody fragment including VH domain (Ward et al., Nature 341:544-546(1989)), the term "Fab fragment" means an antibody fragment including VL, VH, CL, and CH domains, and the term "F (ab') 2 fragment" means an antibody fragment including two Fab fragments linked by a disulfide bridge in the hinge region.

In some cases, the antigen-binding fragment of the antibody is an ScFv in which the VL and VH domains form a univalent molecule and a single polypeptide chain by a linker (see, e.g. Bird et al., Science 242:423-426(1988) and Huston et al., Proc.Natl.Acad.Sci.USA 85:5879-5883(1988)). Such ScFv molecules may have a general structure: NH2-VL-linker-VH-COOH or NH2-VH-linker-VL-COOH. Suitable existing linkers include repeated GGGGS amino acid sequences or variants thereof. For example, a linker with amino acid sequence (GGGGS)4 may be used, and variants thereof may also be used (Holliger et al., (1993), Proc.Natl.Acad.Sci.USA 90:6444-6448). Other linkers that may be used in the present invention are described in Alfthan et al., (1995), Protein Eng.8:725-731, Choi et al., (2001), Eur.J.Immunol.31:94-106, Hu et al., (1996), Cancer Res.56:3055-3061, and Kipriyanov et al., (1999), J.Mol.Biol.293:41-56, and Roovers et al., (2001), Cancer Immunol.

In some cases, the antigen-binding fragment of antibody is a diabody, that is, a bivalent antibody in which the VH and VL domains are expressed on a single polypeptide chain. A too-short linker is employed, which disables pairing between two domains of the same chain and forces the domains to pair with complementary domains of another chain, thus producing two antigen-binding sites (see, e.g. Holliger P. et al., Proc.Natl.Acad.Sci. USA 90:6444-6448(1993), and Poljak R.J. et al., Structure 2:1121-1123(1994)).

Antigen-binding fragments of antibodies (e.g. antibody fragments described above) may be obtained from a given antibody using conventional techniques known to those skilled in the art (e.g. recombinant DNA techniques or enzymatic or chemical cleavage method), and antigen-binding fragments of antibodies may be specifically screened in the same manner as for intact antibodies.

As used herein, the term "antibody" includes not only an intact antibody but also antigen-binding fragments, unless otherwise specified.

As used herein, the terms "mono antibody" and "monoclonal antibody" refer to an antibody or a fragment of an antibody from a group of highly homologous antibody molecules, that is, a group of identical antibody molecules except for natural mutations that may occur spontaneously. Monoclonal antibody shows high specificity to a single epitope on an antigen. Polyclonal antibody is relative to monoclonal antibody. It usually includes at least two or more different antibodies, which can identify different epitopes on an antigen. Generally, monoclonal antibodies can be obtained using the hybridoma technique first reported by Kohler et al. (Nature,256:495, 1975), and can also be obtained by adopting recombinant DNA techniques (referring to U.S.P 4,816,567, etc.).

As used herein, the term "chimeric antibody" refers to an antibody that, a part of its light chain or/and heavy chain originates from an antibody (which may originate from a specific species or belong to a specific antibody class or subclass), and another part of the light chain or/and heavy chain originates from another antibody (which may originate from the same or different species or belong to the same or different antibody class or subclass). It still retains binding activity to the target antigen in any case (U.S.P 4,816,567 to Cabilly et al.; Morrison et al., Proc.Natl.Acad.Sci.USA,81:6851 6855(1984)).

As used herein, the term "humanized antibody" refers to an antibody or antibody fragment resulting from the replacement of all or part of the CDR domains of a human immunoglobulin (receptor antibody) with the CDR domains of a non-human antibody (donor antibody), where the donor antibody may be a non-human (e.g. mouse, rat, or rabbit) antibody with the desired specificity, affinity, or reactivity. In addition, some amino acid residues in the framework region (FR) of receptor antibody may also be replaced by amino acid residues of corresponding non-human antibodies or by amino acid residues of other antibodies to further improve or optimize the performance of antibodies. For more details on humanized antibodies, please refer to Jones et al., Nature, 321:522 525(1986); Reichmann et al., Nature, 332:323 329(1988); Presta, Curr. Op.Struct.Biol.,2:593 596(1992); and Clark, Immunol. Today 21:397 402(2000) etc.

As used herein, the term "epitope" refers to a site on an antigen that is specifically targeted to by an immunoglobulin or antibody. "Epitope" is also referred to as "antigenic determinant" in this field. An epitope or antigenic determinant usually includes chemically active surface groups of molecules such as amino acids, carbohydrate, or sugar side chains, and has specific three-dimensional structural characteristics and specific charge characteristics. For example, an epitope typically has a unique spatial conformation including at least 3, 4, 5, 6, 7, 8, 9, 10, 11, 12, 13, 14, or 15 consecutive or discontinuous amino acids, which may be "linear" or "conformational." Please refer to, for example, Epitope Mapping Protocols in Methods in Molecular Biology, Vol. 66, G.E. Morris, Ed. (1996). In a linear epitope, all the points of interaction between a protein and an interacting molecule (e.g. an antibody) exist linearly along the primary amino acid sequence of the protein. In a conformational epitope, the interaction points exist on amino acid residues of the protein separated from each other.

As used herein, the terms "isolating" and "isolated" refer to those artificially obtained from a natural state. If a certain "isolated" substance or component appears in nature, it may be that its natural environment has changed, or the substance has been separated from the natural environment, or both. For example, some polynucleotides or polypeptides naturally exist in a living animal, and the same polynucleotides or polypeptides with high purity separated from this natural state are known as isolated. The terms "isolating" and "isolated" do not exclude the mixing of artificial or synthetic substances, nor the presence of other impurities that do not affect the activity of substances.

As used herein, the term "vector" refers to a nucleic acid vehicle in which a polynucleotide can be inserted. When a vector can express the protein encoded by the inserted polynucleotide, it is known as an expression vector. Vectors can be introduced into host cells through transformation, transduction, or transfection, so that the genetic material elements carried by vectors can be expressed in host cells. Vectors are known to those skilled in the art, including but not limited to plasmid; phagemid; cosmid; artificial chromosomes, such as yeast artificial chromosome (YAC), bacterial artificial chromosome (BAC), or P1 derived artificial chromosome (PAC); bacteriophage, such as λ bacteriophage or M13 bacteriophage; and animal virus. Animal viruses that can be used as vectors include, but are not limited to, retroviruses (including lentivirus), adenoviruses, adeno-associated viruses, herpesviruses (e.g. herpes simplex virus), poxviruses, baculoviruses, papillomavirus, and papovaviruses (e.g. SV40). A vector may include a variety of elements controlling expression, including but not limited to promoter sequence, transcription initiation sequence, enhancer sequence, selective element, and reporter gene. In addition, a vector may also contain a replication initiation site.

As used herein, the term "host cell" refers to a cell that can be used to introduce the vector, including but not limited to, prokaryotic cells (E. coli or bacillus subtilis), fungal cells (yeast cells or aspergillus fungi, etc.), insect cells (S2 fruit fly cells or Sf9), or animal cells (such as fibroblasts, CHO cells, COS cells, NSO cells, HeLa cells, BHK cells, HEK293 cells or human cells).

As used herein, the term "specific binding" refers to a non-random binding reaction between two molecules, such as a reaction between an antibody and the antigen it targets. In certain embodiments, an antibody that specifically binds to an antigen (or specific to an antigen) means that the antibody binds the antigen with an affinity (KD) less than about 10⁻⁵ M, e.g. less than about 10⁻⁶ M, 10⁻⁷ M, 10⁻⁸ M, 10⁻⁹ M, 10⁻¹⁰ M, or less. In some embodiments of the present invention, the term "target" refers to specific binding.

As used herein, the term "Kd" refers to the dissociation equilibrium constant of a particular antibody-antigen interaction, which is used to describe the binding affinity between antibody and antigen. The smaller the equilibrium dissociation constant, the more tightly the antibody binds to the antigen, and the higher affinity between the antibody and antigen. Typically, antibodies bind antigens with a dissociation equilibrium constant (KD) less than about 10⁻⁵ M, for example, less than about 10⁻⁶ M, 10⁻⁷ M, 10⁻⁸ M, 10⁻⁹ M, 10⁻¹⁰ M, or less. For example, the dissociation equilibrium constant may be determined in a Biacore apparatus using surface plasmon resonance (SPR).

As used herein, the terms "monoclonal antibody" and "mono antibody" have the same meaning and are used interchangeably; the terms "polyclonal antibody" and "poly antibody" have the same meaning and are used interchangeably; and the terms "polypeptide" and "protein" have the same meaning and are used interchangeably. And in the present invention, amino acids are generally denoted by single-letter and three-letter abbreviations well-known in the field. For example, glycine can be expressed as G or Gly, and alanine can be expressed as A or Ala.

As used herein, the term "pharmaceutically acceptable excipient" refers to vehicle and/or excipient that are pharmacologically and/or physiologically compatible with the subject and the active ingredients, which are known in the field (for example, Remington's Pharmaceutical Sciences. Edited by Gennaro AR, 19th ed. Pennsylvania: Mack Publishing Company,1995), including but not limited to: PH modulators, surfactants, adjuvant, and ionic strength enhancers. For example, PH modulators include, but are not limited to, phosphate buffers; surfactants include but are not limited to cationic, anionic, or nonionic surfactants, such as Tween-80; and ionic strength enhancers include, but are not limited to, sodium chloride.

As used herein, the term "adjuvant" refers to non-specific immune enhancers that, when delivered with or in advance of an antigen, enhance immune response of the body to the antigen or alter the type of immune response. There are many kinds of adjuvants, including but not limited to aluminum adjuvant (such as aluminum hydroxide), Freund's adjuvant (such as complete Freund's adjuvant and incomplete Freund's adjuvant), corynebacterium parvum, lipopolysaccharide, and cytokines. Freund's adjuvant is the most commonly used one in animal trials at present. Aluminum hydroxide adjuvant is widely used in clinical trials.

As used herein, the term "effective amount" refers to an amount sufficient to achieve or at least partially achieve the desired effect. Therapeutically effective amount refers to the amount sufficient to cure or at least partially prevent the disease and its complications of patients who have already suffered from the disease. Determining such an effective amount is entirely within the ability of those skilled in the art. For example, the effective amount for therapeutic use depends on the severity of the disease to be treated, the general state of the patient's immune system, the general condition of the patient (such as age, weight, and gender), the administration way of the drug, and other treatments administered concurrently.

### Preparation Example 1: Expression and Purification of Recombinant Protein CD4-His

### 1. Construction of pCMV-CD4-His plasmid

PCR amplification with CD4 human cDNA (synthesized by Genewiz) serving as a template was performed, and CD4-His fragment was purified and recovered using DNA extraction kit. Restriction enzyme digestion was performed on the recovered CD4-His fragment and the expression vector pCMV using restriction enzymes Nhel and Xbal. The recovered target gene fragments and linear expression vectors were linked by T4 ligase, and all ligated products were transformed into DH5α chemocompetent cells, which were then spread on Agar plate with Ampicillin (AMP). Properly isolated single colonies were screened for colony PCR identification, the clones identified as positive by PCR were inoculated into LB medium for culture, and bacterial solution was sent to Guangzhou Invitrogen for sequencing and verification. The sequencing results show that the insertion sequence of positive recombinants is completely correct.

### 2. Expression and purification of recombinant protein CD4-His

HEK293F cells (purchased from Invitrogen) were transfected with the recombinant plasmid pCMV-CD4-his following the protocol of lipofectamin transfection kit (purchased from Invitrogen). Five days later, the culture solution was subjected to high-speed centrifugation, supernatant concentration, and liquid exchange, the cells were transferred to Binding Buffer A (20 mM HEPES, 150 mM NaCl, pH 7.4) and loaded to HisTrap column, and the protein was linearly eluted using Elution Buffer (20 mM HEPES,150 mM NaCl, 0.5 M Immidazole, pH 7.4). Initial pure sample was transferred to Binding Buffer B (20 mM Tris-HCl, pH 9.0) using HiTrap Desalting column, loaded to HiTrap Q column, and the protein was linearly eluted using Elution Buffer B (50 mM Tris-HCl, 1M NaCl, pH 9.0). The target sample was recovered and transferred to PBS. The purified sample was added into loading buffer of the reduced protein electrophoresis for SDS-PAGE electrophoresis detection.

The fusion protein CD4-His was prepared thereby.

The amino acid sequence of CD4-His is as follows (371aa):

KKVVLGKKGDTVELTCTASQKKSIQFHWKNSNQIKILGNQGSFLT
KGPSKLNDRADSRRSLWDQGNFPLIIKNLKIEDSDTYICEVEDQKEEVQL
LVFGLTANSDTHLLQGQSLTLTLESPPGSSPSVQCRSPRGKNIQGGKTL
SVSQLELQDSGTWTCTVLQNQKKVEFKIDIVVLAFQKASSIVYKKEGEQV
EFSFPLAFTVEKLTGSGELWWQAERASSSKSWITFDLKNKEVSVKRVTQ
DPKLQMGKKLPLHLTLPQALPQYAGSGNLTLALEAKTGKLHQEVNLVVM
RATQLQKNLTCEVWGPTSPKLMLSLKLENKEAKVSKREKAVWVLNPEA
GMWQCLLSDSGQVLLESNIKVLPTWHHHHHH(SEQ ID NO:49).

**The** nucleotide sequence encoding the recombinant CD4-His protein is shown as SEQ ID NO: 50.

### Preparation Example 2: Preparation of Recombinant TGF-β1

### 1. Construction of pCMV-TGF-β1-His plasmid

PCR amplification was performed with TGF-β1 human cDNA (synthesized by Genewiz) serving as a template, and TGF-β1-His fragment was purified and recovered using DNA extraction kit. Restriction enzyme digestion was performed on the recovered TGF-β1-His fragment and the expression vector pCMV using restriction enzymes Nhel and Xbal. The recovered target gene fragments and linear expression vectors were linked by T4 ligase, and all ligated products were transformed into DH5α chemocompetent cells, which were then spread on Agar plate with Ampicillin (AMP). Properly isolated single colonies were screened for colony PCR identification, the clones identified as positive by PCR were inoculated into LB medium for culture, and bacterial solution was sent to Guangzhou Invitrogen for sequencing and verification. The sequencing results show that the insertion sequence of positive recombinants is completely correct.

### 2. Expression and purification of fusion protein TGF-β1-His

HEK293F cells (purchased from Invitrogen) were transfected with the recombinant plasmid pCMV-TGF-β1-his following the protocol of lipofectamin transfection kit (purchased from Invitrogen). Five days later, the culture solution was subjected to high-speed centrifugation, supernatant concentration, and liquid exchange, the cells were transferred to Binding Buffer A (20 mM HEPES, 150 mM NaCl, pH 7.4) and loaded to HisTrap column, and the protein was linearly eluted using Elution Buffer (20 mM HEPES,150 mM NaCl, 0.5 M Immidazole, pH 7.4). Initial pure sample was transferred to Binding Buffer B (20 mM Tris-HCl, pH 9.0) using HiTrap Desalting column, loaded to HiTrap Q column, and the protein was linearly eluted using Elution Buffer B (50 mM Tris-HCl, 1M NaCl, pH 9.0. The target sample was recovered and transferred to PBS. The purified sample was added into loading buffer of the reduced protein electrophoresis for SDS-PAGE electrophoresis detection.

The fusion protein TGF-β1-His was prepared thereby.

The amino acid sequence of TGF-β1-His is as follows (118aa):

ALDTNYCFSSTEKNCCVRQLYIDFRKDLGWKWIHEPKGYHANFC
LGPCPYIWSLDTQYSKVLALYNQHNPGASAAPCCVPQALEPLPIVYYVG
RKPKVEQLSNMIVRSCKCSHHHHHH(SEQ ID NO:51).

The nucleotide sequence encoding the recombinant TGF-β1-His protein is shown as SEQ ID NO: 52.

### Preparation Example 3: Preparation of TGF-β1 Antibody (ScFv-IgG4)

The VH and VL (derived from metelimumab) of TGF-β1 antibody were linked by (GGGGS)₃ to form an ScFv, which was then linked with Fc constant region by a hinge to form an ScFv-IgG. Fc constant region adopted Ig gamma-4 chain C region, ACCESSION:P01861. The nucleotide sequence encoding anti-TGF-β1 antibody was synthesized by Shanghai Sangon.

The nucleotide sequence encoding TGF-β1 antibody was cloned into pCMV vector, and the recombinant expression plasmid of TGF-β1 antibody was obtained.

HEK293F cells (purchased from Invitrogen) were transfected with the recombinant plasmid. Five days later, the culture solution was subjected to high-speed centrifugation, supernatant concentration, and liquid exchange. The cells were transferred to 1 x Binding Buffer (20 mM Na₃PO₄, pH 7.0) and loaded to Protein G column, and the protein was eluted to Neutralization buffer (1 M Tris-HCl, pH 9.0) using Elution Buffer (10 mM Tris-Glycine, pH 2.7). The target sample was recovered and transferred to PBS. The purified sample was added into loading buffer of the reduced protein electrophoresis for SDS-PAGE electrophoresis detection.

### TGF-β1 antibody (ScFv-IgG4) was prepared thereby.

Amino acid sequence encoding TGF-β1 antibody: (475bp)

EVQLVESGGGVVQPGRSLRLSCAASGFTFSSYGMHWVRQAPGK
ELEWVAVISYDGSIKYYADSVKGRFTISRDNSKNTLYLQMNSLRAEDTAV
YYCARTGEYSGYDTDPQYSWGQGTTVTVSSGGGGSGGGGSGGGGSE
IVLTQSPSSLSASVGDRVTITCRASQGIGDDLGWYQQKPGKAPILLIYGTS
TLQSGVPSRFSGSGSGTDFTLTINSLQPEDFATYYCLQDSNYPLTFGGG
TRLEIKSGSKYGPPCPPCPAPEFLGGPSVFLFPPKPKDTLMISRTPEVTC
VVVDVSQEDPEVQFNWYVDGVEVHNAKTKPREEQFNSTYRVVSVLTVL
HQDWLNGKEYKCKVSNKGLPSSIEKTISKAKGQPREPQVYTLPPSQEEM
TKNQVSLTCLVKGFYPSDIAVEWESNGQPENNYKTTPPVLDSDGSFFLY
SRLTVDKSRWQEGNVFSCSVMHEALHNHYTQKSLSLSLGK（SEQ ID
NO:68）.

### Preparation Example 4: Preparation of Control Antibody (ScFv-IgG4)

The VH and VL of the control antibody were linked by (GGGGS)₃ to form an ScFv, which was then linked with Fc constant region by a hinge to form an ScFv-IgG. Fc constant region adopted Ig gamma-4 chain C region, ACCESSION:P01861. The nucleotide sequence of control antibody was synthesized by Shanghai Sangon.

The nucleotide sequence encoding control antibody was cloned into pCMV vector, and the recombinant expression plasmid of control antibody was obtained.

HEK293F cells (purchased from Invitrogen) were transfected with the recombinant plasmid. Five days later, the culture solution was subjected to high-speed centrifugation, supernatant concentration, and liquid exchange. The cells were transferred to 1 x Binding Buffer (20 mM Na₃PO₄, pH 7.0) and loaded to Protein G column, and the protein was eluted to Neutralization buffer (1 M Tris-HCl, pH 9.0) using Elution Buffer (10 mM Tris-Glycine, pH 2.7). The target sample was recovered and transferred to PBS. The purified sample was added into loading buffer of the reduced protein electrophoresis for SDS-PAGE electrophoresis detection.

Amino acid sequence encoding control antibody: (475aa)

EVQLVESGGGLVQPGGSLRLSCAASGYTFTNYGMNWVRQAPGK
GLEWVGWINTYTGEPTYAADFKRRFTFSLDTSKSTAYLQMNSLRAEDTA
VYYCAKYPHYYGSSHWYFDVWGQGTLVTVSSGGGGSGGGGSGGGGS
DIQMTQSPSSLSASVGDRVTITCSASQDISNYLNWYQQKPGKAPKVLIYF
TSSLHSGVPSRFSGSGSGTDFTLTISSLQPEDFATYYCQQYSTVPWTFG
QGTKVEIKSGSKYGPPCPPCPAPEFLGGPSVFLFPPKPKDTLMISRTPEV
TCVVVDVSQEDPEVQFNWYVDGVEVHNAKTKPREEQFNSTYRVVSVLT
VLHQDWLNGKEYKCKVSNKGLPSSIEKTISKAKGQPREPQVYTLPPSQE
EMTKNQVSLTCLVKGFYPSDIAVEWESNGQPENNYKTTPPVLDSDGSFF
LYSRLTVDKSRWQEGNVFSCSVMHEALHNHYTQKSLSLSLGK（SEQ ID
NO:69）

The control antibody (ScFv-IgG4) was prepared thereby.

### Preparation Example 5: Preparation of Bifunctional Antibody CT101

The first polypeptide chain of bifunctional antibody CT101 included the light chain of Ibalizumab, and the second polypeptide chain included the heavy chain of Ibalizumab linking to anti-TGF-β1 ScFv through a (GGGGS)₃ linker. Finally, the two polypeptide chains formed IgG-(H)-ScFv.

The nucleotide sequence encoding bifunctional antibody CT101 was cloned into pCMV vector, and the recombinant expression plasmid of CT101 antibody was obtained.

HEK293F cells (purchased from Invitrogen) were transfected with the recombinant plasmid. Five days later, the culture solution was subjected to high-speed centrifugation, supernatant concentration, and liquid exchange. The cells were transferred to 1 x Binding Buffer (20 mM Na₃PO₄, pH 7.0) and loaded to Protein G column, and the protein was eluted to Neutralization buffer (1 M Tris-HCl, pH 9.0) using Elution Buffer (10 mM Tris-Glycine, pH 2.7). The target sample was recovered and transferred to PBS. The purified sample was added into loading buffer of the reduced protein electrophoresis for SDS-PAGE electrophoresis detection.

Amino acid sequence encoding the first polypeptide chain of bifunctional antibody CT101:
(219aa)

DIVMTQSPDSLAVSLGERVTMNCKSSQSLLYSTNQKNYLAWYQQ
KPGQSPKLLIYWASTRESGVPDRFSGSGSGTDFTLTISSVQAEDVAVYY
CQQYYSYRTFGGGTKLEIKGTVAAPSVFIFPPSDEQLKSGTASVVCLLNN
FYPREAKVQWKVDNALQSGNSQESVTEQDSKDSTYSLSSTLTLSKADY
EKHKVYACEVTHQGLSSPVTKSFNRGEC（SEQ ID NO:13）

Amino acid sequence encoding the second polypeptide chain of bifunctional antibody CT101: (709aa)

QVQLQQSGPEVVKPGASVKMSCKASGYTFTSYVIHWVRQKPGQ
GLDWIGYINPYNDGTDYDEKFKGKATLTSDTSTSTAYMELSSLRSEDTAV
YYCAREKDNYATGAWFAYWGQGTLVTVSSASTKGPSVFPLAPCSRSTS
ESTAALGCLVKDYFPEPVTVSWNSGALTSGVHTFPAVLQSSGLYSLSSV
VTVPSSSLGTKTYTCNVDHKPSNTKVD

KRVESKYGPPCPPCPAPEFLGGPSVFLFPPKPKDTLMISRTPEVT
CVVVDVSQEDPEVQFNWYVDGVEVHNAKTKPREEQFNSTYRVVSVLTV
LHQDWLNGKEYKCKVSNKGLPSSIEKTISKAKGQPREPQVYTLPPSQEE
MTKNQVSLTCLVKGFYPSDIAVEWESNGQPENNYKTTPPVLDSDGSFFL
YSRLTVDKSRWQEGNVFSCSVMHEALHNHYTQKSLSLSLGKGGGGSG
GGGSGGGGSEVQLVESGGGVVQPGRSLRLSCAASGFTFSSYGMHWV
RQAPGKELEWVAVISYDGSIKYYADSVKGRFTISRDNSKNTLYLQMNSL
RAEDTAVYYCARTGEYSGYDTDPQYSWGQGTTVTVSSGGGGSGGGG
SGGGGSEIVLTQSPSSLSASVGDRVTITCRASQGIGDDLGWYQQKPGKA
PILLIYGTSTLQSGVPSRFSGSGSGTDFTLTINSLQPEDFATYYCLQDSNY
PLTFGGGTRLEIK（SEQ ID NO:14）

The bifunctional antibody CT101 was prepared thereby.

### Preparation Example 6: Preparation of Bifunctional Antibody CT102

The first polypeptide chain of bifunctional antibody CT102 included the light chain of Ibalizumab linking to anti-TGF-β1 ScFv through a (GGGGS)₃ linker. The second polypeptide chain included the heavy chain of Ibalizumab. Finally, the two polypeptide chains formed IgG-(L)-ScFv.

The nucleotide sequence encoding bifunctional antibody CT102 was cloned into pCMV vector, and the recombinant expression plasmid of CT102 antibody was obtained.

HEK293F cells (purchased from Invitrogen) were transfected with the recombinant plasmid. Five days later, the culture solution was subjected to high-speed centrifugation, supernatant concentration, and liquid exchange. The cells were transferred to 1 x Binding Buffer (20 mM Na₃PO₄, pH 7.0) and loaded to Protein G column, and the protein was eluted to Neutralization buffer (1 M Tris-HCl, pH 9.0) using Elution Buffer (10 mM Tris-Glycine, pH 2.7). The target sample was recovered and transferred to PBS. The purified sample was added into loading buffer of the reduced protein electrophoresis for SDS-PAGE electrophoresis detection.

Amino acid sequence encoding the first polypeptide chain of bifunctional antibody CT102: (479aa)

DIVMTQSPDSLAVSLGERVTMNCKSSQSLLYSTNQKNYLAWYQQ
KPGQSPKLLIYWASTRESGVPDRFSGSGSGTDFTLTISSVQAEDVAVYY
CQQYYSYRTFGGGTKLEIKGTVAAPSVFIFPPSDEQLKSGTASVVCLLNN
FYPREAKVQWKVDNALQSGNSQESVTEQDSKDSTYSLSSTLTLSKADY

EKHKVYACEVTHQGLSSPVTKSFNRGECGGGGSGGGGSGGGGSEVQL
VESGGGVVQPGRSLRLSCAASGFTFSSYGMHWVRQAPGKELEWVAVI
SYDGSIKYYADSVKGRFTISRDNSKNTLYLQMNSLRAEDTAVYYCARTG
EYSGYDTDPQYSWGQGTTVTVSSGGGGSGGGGSGGGGSEIVLTQSPS
SLSASVGDRVTITCRASQGIGDDLGWYQQKPGKAPILLIYGTSTLQSGVP
SRFSGSGSGTDFTLTINSLQPEDFATYYCLQDSNYPLTFGGGTRLEIK
（SEQ ID NO:15）

Amino acid sequence encoding the second polypeptide chain of bifunctional antibody CT102: (449aa)

QVQLQQSGPEVVKPGASVKMSCKASGYTFTSYVIHWVRQKPGQ
GLDWIGYINPYNDGTDYDEKFKGKATLTSDTSTSTAYMELSSLRSEDTAV
YYCAREKDNYATGAWFAYWGQGTLVTVSSASTKGPSVFPLAPCSRSTS
ESTAALGCLVKDYFPEPVTVSWNSGALTSGVHTFPAVLQSSGLYSLSSV
VTVPSSSLGTKTYTCNVDHKPSNTKVD

KRVESKYGPPCPPCPAPEFLGGPSVFLFPPKPKDTLMISRTPEVT
CVVVDVSQEDPEVQFNWYVDGVEVHNAKTKPREEQFNSTYRVVSVLTV
LHQDWLNGKEYKCKVSNKGLPSSIEKTISKAKGQPREPQVYTLPPSQEE
MTKNQVSLTCLVKGFYPSDIAVEWESNGQPENNYKTTPPVLDSDGSFFL
YSRLTVDKSRWQEGNVFSCSVMHEALHNHYTQKSLSLSLGK（SEQ ID
NO:16）

The bifunctional antibody CT102 was prepared thereby.

### Preparation Example 7: Preparation of Bifunctional Antibody CT103

The first polypeptide chain of bifunctional antibody CT103 included the light chain of Ibalizumab, and the second polypeptide chain included the heavy chain of Ibalizumab linking to anti-TGF-β1 ScFv through a (GGGGS)₃ linker. Finally, the two polypeptide chains formed ScFv-(H)-IgG.

The nucleotide sequence encoding bifunctional antibody CT103 was cloned into pCMV vector, and the recombinant expression plasmid of CT103 antibody was obtained.

HEK293F cells (purchased from Invitrogen) were transfected with the recombinant plasmid. Five days later, the culture solution was subjected to high-speed centrifugation, supernatant concentration, and liquid exchange. The cells were transferred to 1 x Binding Buffer (20 mM Na₃PO₄, pH 7.0) and loaded to Protein G column, and the protein was eluted to Neutralization buffer (1 M Tris-HCl, pH 9.0) using Elution Buffer (10 mM Tris-Glycine, pH 2.7). The target sample was recovered and transferred to PBS. The purified sample was added into loading buffer of the reduced protein electrophoresis for SDS-PAGE electrophoresis detection.

Amino acid sequence encoding the first polypeptide chain of bifunctional antibody CT103: (219aa)

DIVMTQSPDSLAVSLGERVTMNCKSSQSLLYSTNQKNYLAWYQQ
KPGQSPKLLIYWASTRESGVPDRFSGSGSGTDFTLTISSVQAEDVAVYY
CQQYYSYRTFGGGTKLEIKGTVAAPSVFIFPPSDEQLKSGTASVVCLLNN
FYPREAKVQWKVDNALQSGNSQESVTEQDSKDSTYSLSSTLTLSKADY
EKHKVYACEVTHQGLSSPVTKSFNRGEC（SEQ ID NO:17）

Amino acid sequence encoding the second polypeptide chain of bifunctional antibody CT103: (709aa)

EVQLVESGGGVVQPGRSLRLSCAASGFTFSSYGMHWVRQAPGK
ELEWVAVISYDGSIKYYADSVKGRFTISRDNSKNTLYLQMNSLRAEDTAV
YYCARTGEYSGYDTDPQYSWGQGTTVTVSSGGGGSGGGGSGGGGSE
IVLTQSPSSLSASVGDRVTITCRASQGIGDDLGWYQQKPGKAPILLIYGTS
TLQSGVPSRFSGSGSGTDFTLTINSLQPEDFATYYCLQDSNYPLTFGGG
TRLEIKGGGGSGGGGSGGGGSQVQLQQSGPEVVKPGASVKMSCKASG
YTFTSYVIHWVRQKPGQGLDWIGYINPYNDGTDYDEKFKGKATLTSDTS
TSTAYMELSSLRSEDTAVYYCAREKDNYATGAWFAYWGQGTLVTVSSA
STKGPSVFPLAPCSRSTSESTAALGCLVKDYFPEPVTVSWNSGALTSGV
HTFPAVLQSSGLYSLSSVVTVPSSSLGTKTYTCNVDHKPSNTKVDKRVE
SKYGPPCPPCPAPEFLGGPSVFLFPPKPKDTLMISRTPEVTCVVVDVSQ
EDPEVQFNWYVDGVEVHNAKTKPREEQFNSTYRVVSVLTVLHQDWLN
GKEYKCKVSNKGLPSSIEKTISKAKGQPREPQVYTLPPSQEEMTKNQVS
LTCLVKGFYPSDIAVEWESNGQPENNYKTTPPVLDSDGSFFLYSRLTVD
KSRWQEGNVFSCSVMHEALHNHYTQKSLSLSLGK（SEQ ID NO:18）

The bifunctional antibody CT103 was prepared thereby.

### Preparation Example 8: Preparation of Bifunctional Antibody CT104

The first polypeptide chain of the bifunctional antibody CT104 included the light chain of Ibalizumab linking to anti-TGF-β1 ScFv through a (GGGGS)₃ linker. The second polypeptide chain included the heavy chain of Ibalizumab. Finally, the two polypeptide chains formed ScFv-(L)-IgG.

The nucleotide sequence encoding bifunctional antibody CT104 was cloned into pCMV vector, and the recombinant expression plasmid of CT104 antibody was obtained.

HEK293F cells (purchased from Invitrogen) were transfected with the recombinant plasmid. Five days later, the culture solution was subjected to high-speed centrifugation, supernatant concentration, and liquid exchange. The cells were transferred to 1 x Binding Buffer (20 mM Na₃PO₄, pH 7.0) and loaded to Protein G column, and the protein was eluted to Neutralization buffer (1 M Tris-HCl, pH 9.0) using Elution Buffer (10 mM Tris-Glycine, pH 2.7). The target sample was recovered and transferred to PBS. The purified sample was added into loading buffer of the reduced protein electrophoresis for SDS-PAGE electrophoresis detection.

Amino acid sequence encoding the first polypeptide chain of bifunctional antibody CT104: (479aa)

EVQLVESGGGVVQPGRSLRLSCAASGFTFSSYGMHWVRQAPGK
ELEWVAVISYDGSIKYYADSVKGRFTISRDNSKNTLYLQMNSLRAEDTAV
YYCARTGEYSGYDTDPQYSWGQGTTVTVSSGGGGSGGGGSGGGGSE
IVLTQSPSSLSASVGDRVTITCRASQGIGDDLGWYQQKPGKAPILLIYGTS
TLQSGVPSRFSGSGSGTDFTLTINSLQPEDFATYYCLQDSNYPLTFGGG
TRLEIKGGGGSGGGGSGGGGSDIVMTQSPDSLAVSLGERVTMNCKSSQ
SLLYSTNQKNYLAWYQQKPGQSPKLLIYWASTRESGVPDRFSGSGSGT
DFTLTISSVQAEDVAVYYCQQYYSYRTFGGGTKLEIKGTVAAPSVFIFPP
SDEQLKSGTASVVCLLNNFYPREAKVQWKVDNALQSGNSQESVTEQDS
KDSTYSLSSTLTLSKADYEKHKVYACEVTHQGLSSPVTKSFNRGEC

(SEQ ID NO:19)

Amino acid sequence encoding the second polypeptide chain of bifunctional antibody CT104: (449aa)

QVQLQQSGPEVVKPGASVKMSCKASGYTFTSYVIHWVRQKPGQ
GLDWIGYINPYNDGTDYDEKFKGKATLTSDTSTSTAYMELSSLRSEDTAV
YYCAREKDNYATGAWFAYWGQGTLVTVSSASTKGPSVFPLAPCSRSTS
ESTAALGCLVKDYFPEPVTVSWNSGALTSGVHTFPAVLQSSGLYSLSSV
VTVPSSSLGTKTYTCNVDHKPSNTKVD

KRVESKYGPPCPPCPAPEFLGGPSVFLFPPKPKDTLMISRTPEVT
CVVVDVSQEDPEVQFNWYVDGVEVHNAKTKPREEQFNSTYRVVSVLTV
LHQDWLNGKEYKCKVSNKGLPSSIEKTISKAKGQPREPQVYTLPPSQEE
MTKNQVSLTCLVKGFYPSDIAVEWESNGQPENNYKTTPPVLDSDGSFFL
YSRLTVDKSRWQEGNVFSCSVMHEALHNHYTQKSLSLSLGK（SEQ ID
NO:20）

The bifunctional antibody CT104 was prepared thereby.

### Preparation Example 9: Preparation of Bifunctional Antibody CT105

The first polypeptide chain of the bifunctional antibody CT105 included the light chain of Ibalizumab linking to the VL of anti-TGF-β1 antibody through a (GGGGS)₁ linker. The second polypeptide chain included the heavy chain of Ibalizumab linking to the VH of anti-TGF-β1 antibody. Finally, the two polypeptide chains formed DVD-IgG.

The nucleotide sequence encoding bifunctional antibody CT105 was cloned into pCMV vector, and the recombinant expression plasmid of CT105 antibody was obtained.

HEK293F cells (purchased from Invitrogen) were transfected with the recombinant plasmid. Five days later, the culture solution was subjected to high-speed centrifugation, supernatant concentration, and liquid exchange. The cells were transferred to 1 x Binding Buffer (20 mM Na₃PO₄, pH 7.0) and loaded to Protein G column, and the protein was eluted to Neutralization buffer (1 M Tris-HCl, pH 9.0) using Elution Buffer (10 mM Tris-Glycine, pH 2.7). The target sample was recovered and transferred to PBS. The purified sample was added into loading buffer of the reduced protein electrophoresis for SDS-PAGE electrophoresis detection.

Amino acid sequence encoding the first polypeptide chain of bifunctional antibody CT105: (331aa)

EIVLTQSPSSLSASVGDRVTITCRASQGIGDDLGWYQQKPGKAPI
LLIYGTSTLQSGVPSRFSGSGSGTDFTLTINSLQPEDFATYYCLQDSNYP
LTFGGGTRLEIKGGGGSDIVMTQSPDSLAVSLGERVTMNCKSSQSLLYS
TNQKNYLAWYQQKPGQSPKLLIYWASTRESGVPDRFSGSGSGTDFTLTI
SSVQAEDVAVYYCQQYYSYRTFGGGTKLEIKGTVAAPSVFIFPPSDEQL
KSGTASVVCLLNNFYPREAKVQWKVDNALQSGNSQESVTEQDSKDSTY
SLSSTLTLSKADYEKHKVYACEVTHQGLSSPVTKSFNRGEC（SEQ ID
NO:21）

Amino acid sequence encoding the second polypeptide chain of bifunctional antibody CT105: (449aa)

EVQLVESGGGVVQPGRSLRLSCAASGFTFSSYGMHWVRQAPGK
ELEWVAVISYDGSIKYYADSVKGRFTISRDNSKNTLYLQMNSLRAEDTAV
YYCARTGEYSGYDTDPQYSWGQGTTVTVSSGGGGSQVQLQQSGPEV
VKPGASVKMSCKASGYTFTSYVIHWVRQKPGQGLDWIGYINPYNDGTD
YDEKFKGKATLTSDTSTSTAYMELSSLRSEDTAVYYCAREKDNYATGAW
FAYWGQGTLVTVSSASTKGPSVFPLAPCSRSTSESTAALGCLVKDYFPE

PVTVSWNSGALTSGVHTFPAVLQSSGLYSLSSVVTVPSSSLGTKTYTCN
VDHKPSNTKVDKRVESKYGPPCPPCPAPEFLGGPSVFLFPPKPKDTLMI
SRTPEVTCVVVDVSQEDPEVQFNWYVDGVEVHNAKTKPREEQFNSTYR
VVSVLTVLHQDWLNGKEYKCKVSNKGLPSSIEKTISKAKGQPREPQVYT
LPPSQEEMTKNQVSLTCLVKGFYPSDIAVEWESNGQPENNYKTTPPVLD
SDGSFFLYSRLTVDKSRWQEGNVFSCSVMHEALHNHYTQKSLSLSLGK
（SEQ ID NO:22）

The bifunctional antibody CT105 was prepared thereby.

### Preparation Example 10: Preparation of Bifunctional Antibody CT106

The first polypeptide chain of bifunctional antibody CT106 included the light chain of Ibalizumab. The second polypeptide chain included a heavy chain of Ibalizumab (S354C/T366W/R409A Knob mutation was introduced into Fc region). The third polypeptide chain included two anti-TGF-β1 antibody VH and CL combinations (CrossMab format) connected in series, and an Fc constant region IgG4 (introducing Y349C/T366S/L368A/F405K/Y407V Hole mutation). The fourth polypeptide chain included anti-TGF-β1 antibody VL and CH1 combinations (crossmab format). Finally, the four polypeptide chains formed sFab-IgG.

The nucleotide sequence encoding bifunctional antibody CT106 was cloned into pCMV vector, and the recombinant expression plasmid of CT106 antibody was obtained.

HEK293F cells (purchased from Invitrogen) were transfected with the recombinant plasmid. Five days later, the culture solution was subjected to high-speed centrifugation, supernatant concentration, and liquid exchange. The cells were transferred to 1 x Binding Buffer (20 mM Na₃PO₄, pH 7.0) and loaded to Protein G column, and the protein was eluted to Neutralization buffer (1 M Tris-HCl, pH 9.0) using Elution Buffer (10 mM Tris-Glycine, pH 2.7). The target sample was recovered and transferred to PBS. The purified sample was added into loading buffer of the reduced protein electrophoresis for SDS-PAGE electrophoresis detection.

Amino acid sequence encoding the first polypeptide chain of bifunctional antibody CT106: (219aa)

DIVMTQSPDSLAVSLGERVTMNCKSSQSLLYSTNQKNYLAWYQQ
KPGQSPKLLIYWASTRESGVPDRFSGSGSGTDFTLTISSVQAEDVAVYY
CQQYYSYRTFGGGTKLEIKGTVAAPSVFIFPPSDEQLKSGTASVVCLLNN
FYPREAKVQWKVDNALQSGNSQESVTEQDSKDSTYSLSSTLTLSKADY
EKHKVYACEVTHQGLSSPVTKSFNRGEC（SEQ ID NO:23）

Amino acid sequence encoding the second polypeptide chain of bifunctional antibody CT106: (449aa)

QVQLQQSGPEVVKPGASVKMSCKASGYTFTSYVIHWVRQKPGQ
GLDWIGYINPYNDGTDYDEKFKGKATLTSDTSTSTAYMELSSLRSEDTAV
YYCAREKDNYATGAWFAYWGQGTLVTVSSASTKGPSVFPLAPCSRSTS
ESTAALGCLVKDYFPEPVTVSWNSGALTSGVHTFPAVLQSSGLYSLSSV
VTVPSSSLGTKTYTCNVDHKPSNTKVD

QVQLQQSGPEVVKPGASVKMSCKASGYTFTSYVIHWVRQKPGQ
GLDWIGYINPYNDGTDYDEKFKGKATLTSDTSTSTAYMELSSLRSEDTAV
YYCAREKDNYATGAWFAYWGQGTLVTVSSASTKGPSVFPLAPCSRSTS
ESTAALGCLVKDYFPEPVTVSWNSGALTSGVHTFPAVLQSSGLYSLSSV
VTVPSSSLGTKTYTCNVDHKPSNTKVDKRVESKYGPPCPPCPAPEFLGG
PSVFLFPPKPKDTLMISRTPEVTCVVVDVSQEDPEVQFNWYVDGVEVHN
AKTKPREEQFNSTYRVVSVLTVLHQDWLNGKEYKCKVSNKGLPSSIEKTI
SKAKGQPREPQVYTLPPCQEEMTKNQVSLWCLVKGFYPSDIAVEWESN
GQPENNYKTTPPVLDSDGSFFLYSALTVDKSRWQEGNVFSCSVMHEAL
HNHYTQKSLSLSLGK（SEQ ID NO:24）.

Amino acid sequence encoding the third polypeptide chain of bifunctional antibody CT106: (698aa)

EVQLVESGGGVVQPGRSLRLSCAASGFTFSSYGMHWVRQAPGK
ELEWVAVISYDGSIKYYADSVKGRFTISRDNSKNTLYLQMNSLRAEDTAV
YYCARTGEYSGYDTDPQYSWGQGTTVTVSGGSGTVAAPSVFIFPPSDE
QLKSGTASVVCLLNNFYPREAKVQWKVDNALQSGNSQESVTEQDSKDS
TYSLSSTLTLSKADYEKHKVYACEVTHQ

GLSSPVTKSFNRGECSGGGGSEVQLVESGGGVVQPGRSLRLSC
AASGFTFSSYGMHWVRQAPGKELEWVAVISYDGSIKYYADSVKGRFTIS
RDNSKNTLYLQMNSLRAEDTAVYYCARTGEYSGYDTDPQYSWGQGTT
VTVSGGSGTVAAPSVFIFPPSDEQLKSGTASVVCLLNNFYPREAKVQWK
VDNALQSGNSQESVTEQDSKDSTYSLSSTLTLSKADYEKHKVYACEVTH
QGLSSPVTKSFNRGECSKYGPPCPPCPAPEFLGGPSVFLFPPKPKDTLM
ISRTPEVTCVVVDVSQEDPEVQFNWYVDGVEVHNAKTKPREEQFNSTY
RVVSVLTVLHQDWLNGKEYKCKVSNKGLPSSIEKTISKAKGQPREPQVC
TLPPSQEEMTKNQVSLSCAVKGFYPSDIAVEWESNGQPENNYKTTPPVL

DSDGSFKLVSRLTVDKSRWQEGNVFSCSVMHEALHNHYTQKSLSLSLG
K（SEQ ID NO:25）

Amino acid sequence encoding the fourth polypeptide chain of bifunctional antibody CT106: (210aa)

EIVLTQSPSSLSASVGDRVTITCRSSQGIGDDLGWYQQKPGKAPI
LLIYGTSTLQSGVPSRFSGSGSGTDFTLTINSLQPEDFATYYCLQDSNYP
LTFGGGTRLEIKGSSASTKGPSVFPLAPCSRSTSESTAALGCLVKDYFPE
PVTVSWNSGALTSGVHTFPAVLQSSGLYSLSSVVTVPSSSLGTKTYTCN
VDHKPSNTKVDKRVES（SEQ ID NO:26)

The bifunctional antibody CT106 was prepared thereby.

### Preparation Example 11: Preparation of Bifunctional Antibody CT107

The first polypeptide chain of bifunctional antibody CT107 included the VL and CH1 of anti-TGF-β1 antibody that are in CrossMab format. The second polypeptide chain included an anti-TGF-β1 antibody VH and CL combination (crossmab format), and an Fc constant region IgG4 (introducing S354C/T366W/R409A Knob mutation into Fc region). The third polypeptide chain included an Ibalizumab VH and CH1 combination, an anti-TGF-β1 antibody VH and CL combination (CrossMab format), and an Fc constant region IgG4 (introducing Y349C/T366S/L368A/F405K/Y407V Hole mutation into Fc region). The fourth polypeptide chain included the light chain of Ibalizumab. Finally, the four polypeptide chains formed sFab-IgG.

The nucleotide sequence encoding bifunctional antibody CT107 was cloned into pCMV vector, and the recombinant expression plasmid of CT107 antibody was obtained.

HEK293F cells (purchased from Invitrogen) were transfected with the recombinant plasmid. Five days later, the culture solution was subjected to high-speed centrifugation, supernatant concentration, and liquid exchange. The cells were transferred to 1 x Binding Buffer (20 mM Na₃PO₄, pH 7.0) and loaded to Protein G column, and the protein was eluted to Neutralization buffer (1 M Tris-HCl, pH 9.0) using Elution Buffer (10 mM Tris-Glycine, pH 2.7). The target sample was recovered and transferred to PBS. The purified sample was added into loading buffer of the reduced protein electrophoresis for SDS-PAGE electrophoresis detection.

Amino acid sequence encoding the first polypeptide chain of bifunctional antibody CT107: (210aa)

EIVLTQSPSSLSASVGDRVTITCRSSQGIGDDLGWYQQKPGKAPI
LLIYGTSTLQSGVPSRFSGSGSGTDFTLTINSLQPEDFATYYCLQDSNYP
LTFGGGTRLEIKGSSASTKGPSVFPLAPCSRSTSESTAALGCLVKDYFPE
PVTVSWNSGALTSGVHTFPAVLQSSGLYSLSSVVTVPSSSLGTKTYTCN
VDHKPSNTKVDKRVES（SEQ ID NO:27）

Amino acid sequence encoding the second polypeptide chain of bifunctional antibody CT107: (460aa)

EVQLVESGGGVVQPGRSLRLSCAASGFTFSSYGMHWVRQAPGK
ELEWVAVISYDGSIKYYADSVKGRFTISRDNSKNTLYLQMNSLRAEDTAV

YYCARTGEYSGYDTDPQYSWGQGTTVTVSGGSGTVAAPSVFIFPPSDE
QLKSGTASVVCLLNNFYPREAKVQWKVDNALQSGNSQESVTEQDSKDS
TYSLSSTLTLSKADYEKHKVYACEVTHQ

GLSSPVTKSFNRGECSKYGPPCPPCPAPEFLGGPSVFLFPPKPK
DTLMISRTPEVTCVVVDVSQEDPEVQFNWYVDGVEVHNAKTKPREEQF
NSTYRVVSVLTVLHQDWLNGKEYKCKVSNKGLPSSIEKTISKAKGQPRE
PQVYTLPPCQEEMTKNQVSLWCLVKGFYPSDIAVEWESNGQPENNYKT
TPPVLDSDGSFFLYSALTVDKSRWQEGNVFSCSVMHEALHNHYTQKSL
SLSLGK（SEQ ID NO:28）

Amino acid sequence encoding the third polypeptide chain of bifunctional antibody CT107: (687aa)

QVQLQQSGPEVVKPGASVKMSCKASGYTFTSYVIHWVRQKPGQ
GLDWIGYINPYNDGTDYDEKFKGKATLTSDTSTSTAYMELSSLRSEDTAV
YYCAREKDNYATGAWFAYWGQGTLVTVSSASTKGPSVFPLAPCSRSTS
ESTAALGCLVKDYFPEPVTVSWNSGALTSGVHTFPAVLQSSGLYSLSSV
VTVPSSSLGTKTYTCNVDHKPSNTKVD

KRVESGGGGSEVQLVESGGGVVQPGRSLRLSCAASGFTFSSYG
MHWVRQAPGKELEWVAVISYDGSIKYYADSVKGRFTISRDNSKNTLYLQ
MNSLRAEDTAVYYCARTGEYSGYDTDPQYSWGQGTTVTVSGGSGTVA
APSVFIFPPSDEQLKSGTASVVCLLNNFYPREAKVQWKVDNALQSGNSQ
ESVTEQDSKDSTYSLSSTLTLSKADYEKHKVYACEVTHQGLSSPVTKSF
NRGECSKYGPPCPPCPAPEFLGGPSVFLFPPKPKDTLMISRTPEVTCVV
VDVSQEDPEVQFNWYVDGVEVHNAKTKPREEQFNSTYRVVSVLTVLHQ
DWLNGKEYKCKVSNKGLPSSIEKTISKAKGQPREPQVCTLPPSQEEMTK
NQVSLSCAVKGFYPSDIAVEWESNGQPENNYKTTPPVLDSDGSFKLVSR

LTVDKSRWQEGNVFSCSVMHEALHNHYTQKSLSLSLGK（SEQ ID
NO:29）

Amino acid sequence encoding the fourth polypeptide chain of bifunctional antibody CT107: (219aa)

DIVMTQSPDSLAVSLGERVTMNCKSSQSLLYSTNQKNYLAWYQQ
KPGQSPKLLIYWASTRESGVPDRFSGSGSGTDFTLTISSVQAEDVAVYY
CQQYYSYRTFGGGTKLEIKGTVAAPSVFIFPPSDEQLKSGTASVVCLLNN
FYPREAKVQWKVDNALQSGNSQESVTEQDSKDSTYSLSSTLTLSKADY
EKHKVYACEVTHQGLSSPVTKSFNRGEC（SEQ ID NO:30)

The bifunctional antibody CT107 was prepared thereby.

### Preparation Example 12: Preparation of Bifunctional Antibody CT108

The first polypeptide chain of bifunctional antibody CT108 included the light chain of Ibalizumab. The second chain included the VL and CH1 of anti-TGF-β1 antibody that are in CrossMab format. The third polypeptide chain included an Ibalizumab VH and CH1 combination, an anti-TGF-β1 antibody VH and CL combination(CrossMab format), and an Fc constant region IgG4. Finally, the three polypeptide chains formed Fab-IgG.

The nucleotide sequence encoding bifunctional antibody CT108 was cloned into pCMV vector, and the recombinant expression plasmid of CT108 antibody was obtained.

HEK293F cells (purchased from Invitrogen) were transfected with the recombinant plasmid. Five days later, the culture solution was subjected to high-speed centrifugation, supernatant concentration, and liquid exchange. The cells were transferred to 1 x Binding Buffer (20 mM Na₃PO₄, pH 7.0) and loaded to Protein G column, and the protein was eluted to Neutralization buffer (1 M Tris-HCl, pH 9.0) using Elution Buffer (10 mM Tris-Glycine, pH 2.7). The target sample was recovered and transferred to PBS. The purified sample was added into loading buffer of the reduced protein electrophoresis for SDS-PAGE electrophoresis detection.

Amino acid sequence encoding the first polypeptide chain of bifunctional antibody CT108: (219aa)

DIVMTQSPDSLAVSLGERVTMNCKSSQSLLYSTNQKNYLAWYQQ
KPGQSPKLLIYWASTRESGVPDRFSGSGSGTDFTLTISSVQAEDVAVYY
CQQYYSYRTFGGGTKLEIKGTVAAPSVFIFPPSDEQLKSGTASVVCLLNN
FYPREAKVQWKVDNALQSGNSQESVTEQDSKDSTYSLSSTLTLSKADY
EKHKVYACEVTHQGLSSPVTKSFNRGEC（SEQ ID NO:31）

Amino acid sequence encoding the second polypeptide chain of bifunctional antibody CT108: (210aa)

EIVLTQSPSSLSASVGDRVTITCRSSQGIGDDLGWYQQKPGKAPI
LLIYGTSTLQSGVPSRFSGSGSGTDFTLTINSLQPEDFATYYCLQDSNYP
LTFGGGTRLEIKGSSASTKGPSVFPLAPCSRSTSESTAALGCLVKDYFPE
PVTVSWNSGALTSGVHTFPAVLQSSGLYSLSSVVTVPSSSLGTKTYTCN
VDHKPSNTKVDKRVES（SEQ ID NO:32）

Amino acid sequence encoding the third polypeptide chain of bifunctional antibody CT108: (687aa)

QVQLQQSGPEVVKPGASVKMSCKASGYTFTSYVIHWVRQKPGQ
GLDWIGYINPYNDGTDYDEKFKGKATLTSDTSTSTAYMELSSLRSEDTAV
YYCAREKDNYATGAWFAYWGQGTLVTVSSASTKGPSVFPLAPCSRSTS
ESTAALGCLVKDYFPEPVTVSWNSGALTSGVHTFPAVLQSSGLYSLSSV
VTVPSSSLGTKTYTCNVDHKPSNTKVD

KRVESGGGGSEVQLVESGGGVVQPGRSLRLSCAASGFTFSSYG
MHWVRQAPGKELEWVAVISYDGSIKYYADSVKGRFTISRDNSKNTLYLQ
MNSLRAEDTAVYYCARTGEYSGYDTDPQYSWGQGTTVTVSGGSGTVA
APSVFIFPPSDEQLKSGTASVVCLLNNFYPREAKVQWKVDNALQSGNSQ
ESVTEQDSKDSTYSLSSTLTLSKADYEKHKVYACEVTHQGLSSPVTKSF
NRGECSKYGPPCPPCPAPEFLGGPSVFLFPPKPKDTLMISRTPEVTCVV
VDVSQEDPEVQFNWYVDGVEVHNAKTKPREEQFNSTYRVVSVLTVLHQ
DWLNGKEYKCKVSNKGLPSSIEKTISKAKGQPREPQVYTLPPSQEEMTK
NQVSLTCLVKGFYPSDIAVEWESNGQPENNYKTTPPVLDSDGSFFLYSR
LTVDKSRWQEGNVFSCSVMHEALHNHYTQKSLSLSLGK（SEQ ID
NO:33）

The bifunctional antibody CT108 was prepared thereby.

### Preparation Example 13: Preparation of Bifunctional Antibody CT109

The first polypeptide chain of bifunctional antibody CT109 included the light chain of Ibalizumab. The second chain included the VL and CH1 of anti-TGF-β1 antibody that are in CrossMab format. The third polypeptide chain included the heavy chain of Ibalizumab, and an anti-TGF-β1 antibody VH and CL combination (CrossMab format). Finally, the three polypeptide chains formed IgG-Fab.

The nucleotide sequence encoding bifunctional antibody CT109 was cloned into pCMV vector, and the recombinant expression plasmid of CT109 antibody was obtained.

HEK293F cells (purchased from Invitrogen) were transfected with the recombinant plasmid. Five days later, the culture solution was subjected to high-speed centrifugation, supernatant concentration, and liquid exchange. The cells were transferred to 1 x Binding Buffer (20 mM Na₃PO₄, pH 7.0) and loaded to Protein G column, and the protein was eluted to Neutralization buffer (1 M Tris-HCl, pH 9.0) using Elution Buffer (10 mM Tris-Glycine, pH 2.7). The target sample was recovered and transferred to PBS. The purified sample was added into loading buffer of the reduced protein electrophoresis for SDS-PAGE electrophoresis detection.

Amino acid sequence encoding the first polypeptide chain of bifunctional antibody CT109: (219aa)

DIVMTQSPDSLAVSLGERVTMNCKSSQSLLYSTNQKNYLAWYQQ
KPGQSPKLLIYWASTRESGVPDRFSGSGSGTDFTLTISSVQAEDVAVYY
CQQYYSYRTFGGGTKLEIKGTVAAPSVFIFPPSDEQLKSGTASVVCLLNN
FYPREAKVQWKVDNALQSGNSQESVTEQDSKDSTYSLSSTLTLSKADY
EKHKVYACEVTHQGLSSPVTKSFNRGEC（SEQ ID NO:34）

Amino acid sequence encoding the second polypeptide chain of bifunctional antibody CT109: (210aa)

EIVLTQSPSSLSASVGDRVTITCRSSQGIGDDLGWYQQKPGKAPI
LLIYGTSTLQSGVPSRFSGSGSGTDFTLTINSLQPEDFATYYCLQDSNYP

LTFGGGTRLEIKGSSASTKGPSVFPLAPCSRSTSESTAALGCLVKDYFPE
PVTVSWNSGALTSGVHTFPAVLQSSGLYSLSSVVTVPSSSLGTKTYTCN
VDHKPSNTKVDKRVES（SEQ ID NO:35）

Amino acid sequence encoding the third polypeptide chain of bifunctional antibody CT109: (696aa)

QVQLQQSGPEVVKPGASVKMSCKASGYTFTSYVIHWVRQKPGQ
GLDWIGYINPYNDGTDYDEKFKGKATLTSDTSTSTAYMELSSLRSEDTAV
YYCAREKDNYATGAWFAYWGQGTLVTVSSASTKGPSVFPLAPCSRSTS
ESTAALGCLVKDYFPEPVTVSWNSGALTSGVHTFPAVLQSSGLYSLSSV
VTVPSSSLGTKTYTCNVDHKPSNTKVD

KRVESKYGPPCPPCPAPEFLGGPSVFLFPPKPKDTLMISRTPEVT
CVVVDVSQEDPEVQFNWYVDGVEVHNAKTK

PREEQFNSTYRVVSVLTVLHQDWLNGKEYKCKVSNKGLPSSIEK
TISKAKGQPREPQVYTLPPSQEEMTKNQVSLTCLVKGFYPSDIAVEWES
NGQPENNYKTTPPVLDSDGSFFLYSRLTVDKSRWQEGNVFSCSVMHEA
LHNHYTQKSLSLSLGKGGGGSGGGGSGGGGSEVQLVESGGGVVQPG
RSLRLSCAASGFTFSSYGMHWVRQAPGKELEWVAVISYDGSIKYYADSV
KGRFTISRDNSKNTLYLQMNSLRAEDTAVYYCARTGEYSGYDTDPQYS
WGQGTTVTVSGGSGTVAAPSVFIFPPSDEQLKSGTASVVCLLNNFYPRE
AKVQWKVDNALQSGNSQESVTEQDSKDSTYSLSSTLTLSKADYEKHKV
YACEVTHQGLSSPVTKSFNRGEC（SEQ ID NO:36）

The bifunctional antibody CT109 was prepared thereby.

### Preparation Example 14: Preparation of Bifunctional Antibody CT110

The first polypeptide chain of the bifunctional antibody CT110 included the light chain of Ibalizumab. The second polypeptide chain included the heavy chain of Ibalizumab (introducing S354C/T366W/R409A Knob mutation in Fc region) linking to the VH of anti-TGF-β1 antibody through a (GGGGS)₁ linker. The third polypeptide chain included the heavy chain of Ibalizumab (introducing Y349C/T366S/L368A/F405K/Y407V Hole mutation in Fc region) linking to the VL of anti-TGF-β1 antibody through a (GGGGS)₁ linker. Finally, the three polypeptide chains formed IgG-Fv.

The nucleotide sequence encoding bifunctional antibody CT110 was cloned into pCMV vector, and the recombinant expression plasmid of CT110 antibody was obtained.

HEK293F cells (purchased from Invitrogen) were transfected with the recombinant plasmid. Five days later, the culture solution was subjected to high-speed centrifugation, supernatant concentration, and liquid exchange. The cells were transferred to 1 x Binding Buffer (20 mM Na₃PO₄, pH 7.0) and loaded to Protein G column, and the protein was eluted to Neutralization buffer (1 M Tris-HCl, pH 9.0) using Elution Buffer (10 mM Tris-Glycine, pH 2.7). The target sample was recovered and transferred to PBS. The purified sample was added into loading buffer of the reduced protein electrophoresis for SDS-PAGE electrophoresis detection.

Amino acid sequence encoding the first polypeptide chain of bifunctional antibody CT110: (219aa)

DIVMTQSPDSLAVSLGERVTMNCKSSQSLLYSTNQKNYLAWYQQ
KPGQSPKLLIYWASTRESGVPDRFSGSGSGTDFTLTISSVQAEDVAVYY

CQQYYSYRTFGGGTKLEIKGTVAAPSVFIFPPSDEQLKSGTASVVCLLNN
FYPREAKVQWKVDNALQSGNSQESVTEQDSKDSTYSLSSTLTLSKADY
EKHKVYACEVTHQGLSSPVTKSFNRGEC（SEQ ID NO:37）

Amino acid sequence encoding the second polypeptide chain of bifunctional antibody CT110: (577aa)

QVQLQQSGPEVVKPGASVKMSCKASGYTFTSYVIHWVRQKPGQ
GLDWIGYINPYNDGTDYDEKFKGKATLTSDTSTSTAYMELSSLRSEDTAV
YYCAREKDNYATGAWFAYWGQGTLVTVSSASTKGPSVFPLAPCSRSTS
ESTAALGCLVKDYFPEPVTVSWNSGALTSGVHTFPAVLQSSGLYSLSSV
VTVPSSSLGTKTYTCNVDHKPSNTKVD

KRVESKYGPPCPPCPAPEFLGGPSVFLFPPKPKDTLMISRTPEVT
CVVVDVSQEDPEVQFNWYVDGVEVHNAKTKPREEQFNSTYRVVSVLTV
LHQDWLNGKEYKCKVSNKGLPSSIEKTISKAKGQPREPQVYTLPPCQEE
MTKNQVSLWCLVKGFYPSDIAVEWESNGQPENNYKTTPPVLDSDGSFF
LYSALTVDKSRWQEGNVFSCSVMHEALHNHYTQKSLSLSLGKGGGGSE
VQLVESGGGVVQPGRSLRLSCAASGFTFSSYGMHWVRQAPGKELEWV
AVISYDGSIKYYADSVKGRFTISRDNSKNTLYLQMNSLRAEDTAVYYCAR
TGEYSGYDTDPQYSWGQGTTVTVSS（SEQ ID NO:38）

Amino acid sequence encoding the third polypeptide chain of bifunctional antibody CT110: (561aa)

QVQLQQSGPEVVKPGASVKMSCKASGYTFTSYVIHWVRQKPGQ
GLDWIGYINPYNDGTDYDEKFKGKATLTSDTSTSTAYMELSSLRSEDTAV
YYCAREKDNYATGAWFAYWGQGTLVTVSSASTKGPSVFPLAPCSRSTS

ESTAALGCLVKDYFPEPVTVSWNSGALTSGVHTFPAVLQSSGLYSLSSV
VTVPSSSLGTKTYTCNVDHKPSNTKVD

KRVESKYGPPCPPCPAPEFLGGPSVFLFPPKPKDTLMISRTPEVT
CVVVDVSQEDPEVQFNWYVDGVEVHNAKTKPREEQFNSTYRVVSVLTV
LHQDWLNGKEYKCKVSNKGLPSSIEKTISKAKGQPREPQVCTLPPSQEE
MTKNQVSLSCAVKGFYPSDIAVEWESNGQPENNYKTTPPVLDSDGSFK
LVSRLTVDKSRWQEGNVFSCSVMHEALHNHYTQKSLSLSLGKGGGGSE
IVLTQSPSSLSASVGDRVTITCRASQGIGDDLGWYQQKPGKAPILLIYGTS
TLQSGVPSRFSGSGSGTDFTLTINSLQPEDFATYYCLQDSNYPLTFGGG
TRLEIK（SEQ ID NO:39）

The bifunctional antibody CT110 was prepared thereby.

### Preparation Example 15: Preparation of Bifunctional Antibody CT111

The first polypeptide chain of bifunctional antibody CT111 included the light chain of Ibalizumab. The second polypeptide chain included the heavy chain of Ibalizumab (S354C/T366W/R409A Knob mutation was introduced into Fc region). The third polypeptide chain included anti-TGF-β1 ScFv linking with Fc constant region IgG4 through hinge (Y349C/T366S/L368A/F405K/Y407V Hole mutation was introduced into Fc region). Finally, the three polypeptide chains formed Fab-Fc-ScFv.

The nucleotide sequence encoding bifunctional antibody CT111 was cloned into pCMV vector, and the recombinant expression plasmid of CT111 antibody was obtained.

HEK293F cells (purchased from Invitrogen) were transfected with the recombinant plasmid. Five days later, the culture solution was subjected to high-speed centrifugation, supernatant concentration, and liquid exchange. The cells were transferred to 1 x Binding Buffer (20 mM Na₃PO₄, pH 7.0) and loaded to Protein G column, and the protein was eluted to Neutralization buffer (1 M Tris-HCl, pH 9.0) using Elution Buffer (10 mM Tris-Glycine, pH 2.7). The target sample was recovered and transferred to PBS. The purified sample was added into loading buffer of the reduced protein electrophoresis for SDS-PAGE electrophoresis detection.

Amino acid sequence encoding the first polypeptide chain of bifunctional antibody CT111: (219aa)

DIVMTQSPDSLAVSLGERVTMNCKSSQSLLYSTNQKNYLAWYQQ
KPGQSPKLLIYWASTRESGVPDRFSGSGSGTDFTLTISSVQAEDVAVYY
CQQYYSYRTFGGGTKLEIKGTVAAPSVFIFPPSDEQLKSGTASVVCLLNN
FYPREAKVQWKVDNALQSGNSQESVTEQDSKDSTYSLSSTLTLSKADY
EKHKVYACEVTHQGLSSPVTKSFNRGEC（SEQ ID NO:40）

Amino acid sequence encoding the second polypeptide chain of bifunctional antibody CT111: (449aa)

QVQLQQSGPEVVKPGASVKMSCKASGYTFTSYVIHWVRQKPGQ
GLDWIGYINPYNDGTDYDEKFKGKATLTSDTSTSTAYMELSSLRSEDTAV
YYCAREKDNYATGAWFAYWGQGTLVTVSSASTKGPSVFPLAPCSRSTS
ESTAALGCLVKDYFPEPVTVSWNSGALTSGVHTFPAVLQSSGLYSLSSV
VTVPSSSLGTKTYTCNVDHKPSNTKVD

KRVESKYGPPCPPCPAPEFLGGPSVFLFPPKPKDTLMISRTPEVT
CVVVDVSQEDPEVQFNWYVDGVEVHNAKTKPREEQFNSTYRVVSVLTV
LHQDWLNGKEYKCKVSNKGLPSSIEKTISKAKGQPREPQVYTLPPCQEE
MTKNQVSLWCLVKGFYPSDIAVEWESNGQPENNYKTTPPVLDSDGSFF
LYSALTVDKSRWQEGNVFSCSVMHEALHNHYTQKSLSLSLGK（SEQ ID
NO:41）

Amino acid sequence encoding the third polypeptide chain of bifunctional antibody CT111: (471aa)

EVQLVESGGGVVQPGRSLRLSCAASGFTFSSYGMHWVRQAPGK
ELEWVAVISYDGSIKYYADSVKGRFTISRDNSKNTLYLQMNSLRAEDTAV
YYCARTGEYSGYDTDPQYSWGQGTTVTVSSGGGGSGGGGSGGGGSE
IVLTQSPSSLSASVGDRVTITCRASQGIGDDLGWYQQKPGKAPILLIYGTS
TLQSGVPSRFSGSGSGTDFTLTINSLQPEDFATYYCLQDSNYPLTFGGG
TRLEIKYGPPCPPCPAPEFLGGPSVFLFPPKPKDTLMISRTPEVTCVVVD
VSQEDPEVQFNWYVDGVEVHNAKTKPREEQFNSTYRVVSVLTVLHQD
WLNGKEYKCKVSNKGLPSSIEKTISKAKGQPREPQVCTLPPSQEEMTKN
QVSLSCAVKGFYPSDIAVEWESNGQPENNYKTTPPVLDSDGSFKLVSRL
TVDKSRWQEGNVFSCSVMHEALHNHYTQKSLSLSLGK（SEQ ID NO:42）

The bifunctional antibody CT111 was prepared thereby.

### Preparation Example 16: Preparation of Bifunctional Antibody CT112

The first polypeptide chain of bifunctional antibody CT112 included a light chain of Ibalizumab. The second polypeptide chain included a heavy chain of Ibalizumab (S354C/T366W/R409A Knob mutation was introduced into Fc region). The third polypeptide chain included an anti-TGF-β1 antibody VH and CL combination (CrossMab format), and an Fc constant region IgG4 (S354C/T366W/R409A Knob mutation was introduced into the Fc region). The fourth polypeptide chain included the VL and CH1 of anti-TGF-β1 antibody that are in crossmab format. Finally, the four polypeptide chains formed IgG CrossMab.

The nucleotide sequence encoding bifunctional antibody CT112 was cloned into pCMV vector, and the recombinant expression plasmid of CT112 antibody was obtained.

HEK293F cells (purchased from Invitrogen) were transfected with the recombinant plasmid. Five days later, the culture solution was subjected to high-speed centrifugation, supernatant concentration, and liquid exchange. The cells were transferred to 1 x Binding Buffer (20 mM Na₃PO₄, pH 7.0), loaded to Protein G column, and the protein was eluted to Neutralization buffer (1 M Tris-HCl, pH 9.0) using Elution Buffer (10 mM Tris-Glycine, pH 2.7). The target sample was recovered and transferred to PBS. The purified sample was added into loading buffer of the reduced protein electrophoresis for SDS-PAGE electrophoresis detection.

Amino acid sequence encoding the first polypeptide chain of bifunctional antibody CT112: (219aa)

DIVMTQSPDSLAVSLGERVTMNCKSSQSLLYSTNQKNYLAWYQQ
KPGQSPKLLIYWASTRESGVPDRFSGSGSGTDFTLTISSVQAEDVAVYY
CQQYYSYRTFGGGTKLEIKGTVAAPSVFIFPPSDEQLKSGTASVVCLLNN
FYPREAKVQWKVDNALQSGNSQESVTEQDSKDSTYSLSSTLTLSKADY
EKHKVYACEVTHQGLSSPVTKSFNRGEC（SEQ ID NO:43）

Amino acid sequence encoding the second polypeptide chain of bifunctional antibody CT112: (449aa)

QVQLQQSGPEVVKPGASVKMSCKASGYTFTSYVIHWVRQKPGQ
GLDWIGYINPYNDGTDYDEKFKGKATLTSDTSTSTAYMELSSLRSEDTAV
YYCAREKDNYATGAWFAYWGQGTLVTVSSASTKGPSVFPLAPCSRSTS
ESTAALGCLVKDYFPEPVTVSWNSGALTSGVHTFPAVLQSSGLYSLSSV
VTVPSSSLGTKTYTCNVDHKPSNTKVD

KRVESKYGPPCPPCPAPEFLGGPSVFLFPPKPKDTLMISRTPEVT
CVVVDVSQEDPEVQFNWYVDGVEVHNAKTKPREEQFNSTYRVVSVLTV
LHQDWLNGKEYKCKVSNKGLPSSIEKTISKAKGQPREPQVYTLPPCQEE
MTKNQVSLWCLVKGFYPSDIAVEWESNGQPENNYKTTPPVLDSDGSFF
LYSALTVDKSRWQEGNVFSCSVMHEALHNHYTQKSLSLSLGK（SEQ ID
NO:44）

Amino acid sequence encoding the third polypeptide chain of bifunctional antibody CT112: (460aa)

EVQLVESGGGVVQPGRSLRLSCAASGFTFSSYGMHWVRQAPGK
ELEWVAVISYDGSIKYYADSVKGRFTISRDNSKNTLYLQMNSLRAEDTAV
YYCARTGEYSGYDTDPQYSWGQGTTVTVSGGSGTVAAPSVFIFPPSDE
QLKSGTASVVCLLNNFYPREAKVQWKVDNALQSGNSQESVTEQDSKDS
TYSLSSTLTLSKADYEKHKVYACEVTHQ

GLSSPVTKSFNRGECSKYGPPCPPCPAPEFLGGPSVFLFPPKPK
DTLMISRTPEVTCVVVDVSQEDPEVQFNWYVDGVEVHNAKTKPREEQF
NSTYRVVSVLTVLHQDWLNGKEYKCKVSNKGLPSSIEKTISKAKGQPRE
PQVCTLPPSQEEMTKNQVSLSCAVKGFYPSDIAVEWESNGQPENNYKT

TPPVLDSDGSFKLVSRLTVDKSRWQEGNVFSCSVMHEALHNHYTQKSL
SLSLGK（SEQ ID NO:45）

Amino acid sequence encoding the fourth polypeptide chain of bifunctional antibody CT112: (210aa)

EIVLTQSPSSLSASVGDRVTITCRSSQGIGDDLGWYQQKPGKAPI
LLIYGTSTLQSGVPSRFSGSGSGTDFTLTINSLQPEDFATYYCLQDSNYP
LTFGGGTRLEIKGSSASTKGPSVFPLAPCSRSTSESTAALGCLVKDYFPE
PVTVSWNSGALTSGVHTFPAVLQSSGLYSLSSVVTVPSSSLGTKTYTCN
VDHKPSNTKVDKRVES（SEQ ID NO:46）

The bifunctional antibody CT112 was prepared thereby.

### Preparation Example 17: Preparation of Recombinant TGF-β1

### 1. Construction of pCMV-TGF-β1-His plasmid

PCR amplification was performed with TGF-β1 human cDNA (synthesized by Genewiz) serving as a template, TGF-β1-His fragment was purified and recovered using DNA extraction kit. Restriction enzyme digestion was performed on the recovered TGF-β1-His fragment and the expression vector pCMV using restriction enzymes Nhel and Xbal. The recovered target gene fragments and linear expression vectors were linked by T4 ligase, and all ligated products were transformed into DH5α chemocompetent cells, which were then spread on Agar plate with Ampicillin (AMP). Properly isolated single colonies were screened for colony PCR identification, the clones identified as positive by PCR were inoculated into LB medium for culture, and bacterial solution was sent to Guangzhou Invitrogen for sequencing and verification. The sequencing results show that the insertion sequence of positive recombinants is completely correct.

### 2. Expression and purification of fusion protein TGF-β1-His

HEK293F cells (purchased from Invitrogen) were transfected with the recombinant plasmid pCMV-TGF-β1-his following the protocol of lipofectamin transfection kit (purchased from Invitrogen). Five days later, the culture solution was subjected to high-speed centrifugation, supernatant concentration, and liquid exchange, the cells were transferred to Binding Buffer A (20 mM HEPES, 150 mM NaCl, pH 7.4) and loaded to HisTrap column, and the protein was linearly eluted using Elution Buffer (20 mM HEPES,150 mM NaCl, 0.5 M Immidazole, pH 7.4). Initial pure sample was transferred to Binding Buffer B (20 mM Tris-HCl, pH 9.0) using HiTrap Desalting column and loaded to HiTrap Q column, and the protein was linearly eluted using Elution Buffer B (50 mM Tris-HCl, 1M NaCl, pH 9.0). The target sample was recovered and transferred to PBS. The purified sample was added into loading buffer of the reduced protein electrophoresis for SDS-PAGE electrophoresis detection.

The fusion protein TGF-β1-His was prepared thereby.

The amino acid sequence of TGF-β1-His is as follows (118aa): 
ALDTNYCFSSTEKNCCVRQLYIDFRKDLGWKWIHEPKGYHANFC
LGPCPYIWSLDTQYSKVLALYNQHNPGASAAPCCVPQALEPLPIVYYVG
RKPKVEQLSNMIVRSCKCSHHHHHH(SEQ ID NO:70),
 wherein, the underlined part is the amino acid sequence of TGF-β1.

### Nucleic acid sequence encoding TGF-β1-His

GCCCTGGATACCAACTATTGCTTCAGCTCCACAGAGAAGAACT
GCTGTGTGCGGCAGCTGTACATTGACTTTAGGAAGGACCTGGGTTGG
AAGTGGATCCACGAGCCCAAGGGCTACCATGCCAACTTCTGCCTCG
GGCCCTGCCCCTACATTTGGAGCCTGGACACGCAGTACAGCAAGGT
CCTGGCCCTGTACAACCAGCATAACCCGGGCGCCTCGGCGGCGCCG
TGCTGCGTGCCGCAGGCGCTGGAGCCGCTGCCCATCGTGTACTACG
TGGGCCGCAAGCCCAAGGTGGAGCAGCTGTCCAACATGATCGTGCG
CTCCTGCAAGTGCAGCCATCATCATCATCATCAT（SEQ ID NO:71）

### Preparation Example 18: Expression and Purification of Recombinant Protein CD4-His

### 1. Construction of pCMV-CD4-His plasmid

PCR amplification was performed with CD4 human cDNA (synthesized by Genewiz) serving as a template, CD4-His fragment was purified and recovered using DNA extraction kit. Restriction enzyme digestion was performed on the recovered CD4-His fragment and the expression vector pCMV using restriction enzymes Nhel and Xbal. The recovered target gene fragments and linear expression vectors were linked by T4 ligase, and all ligated products were transformed into DH5α chemocompetent cells, which were then spread on Agar plate with Ampicillin (AMP). Properly isolated single colonies were screened for colony PCR identification, the clones identified as positive by PCR were inoculated into LB medium for culture, and bacterial solution was sent to Guangzhou Invitrogen for sequencing and verification. The sequencing results show that the insertion sequence of positive recombinants is completely correct.

### 2. Expression and purification of recombinant protein CD4-His

HEK293F cells (purchased from Invitrogen) were transfected with the recombinant plasmid pCMV-CD4-his following the protocol of lipofectamin transfection kit (purchased from Invitrogen). Five days later, the culture solution was subjected to high-speed centrifugation, supernatant concentration, and liquid exchange, the cells were transferred Binding Buffer A (20 mM HEPES, 150 mM NaCl, pH 7.4) and loaded to HisTrap column, and the protein was linearly eluted using Elution Buffer (20 mM HEPES,150 mM NaCl, 0.5 M Immidazole, pH 7.4). Initial pure sample was transferred to Binding Buffer B (20 mM Tris-HCl, pH 9.0) using HiTrap Desalting column and loaded to HiTrap Q column, and the protein was linearly eluted using Elution Buffer B (50 mM Tris-HCl, 1M NaCl, pH 9.0). The target sample was recovered and transferred to PBS. The purified sample was added into loading buffer of the reduced protein electrophoresis for SDS-PAGE electrophoresis detection.

The fusion protein CD4-His was prepared thereby.

The amino acid sequence of CD4-His is as follows (371aa): 
KKVVLGKKGDTVELTCTASQKKSIQFHWKNSNQIKILGNQGSFLT
KGPSKLNDRADSRRSLWDQGNFPLIIKNLKIEDSDTYICEVEDQKEEVQL
LVFGLTANSDTHLLQGQSLTLTLESPPGSSPSVQCRSPRGKNIQGGKTL
SVSQLELQDSGTWTCTVLQNQKKVEFKIDIVVLAFQKASSIVYKKEGEQV
EFSFPLAFTVEKLTGSGELWWQAERASSSKSWITFDLKNKEVSVKRVTQ
 
DPKLQMGKKLPLHLTLPQALPQYAGSGNLTLALEAKTGKLHQEVNLVVM
RATQLQKNLTCEVWGPTSPKLMLSLKLENKEAKVSKREKAVWVLNPEA
GMWQCLLSDSGQVLLESNIKVLPTWHHHHHH(SEQ ID NO: 72),
 wherein, the underlined part is the amino acid sequence of CD4.

Nucleic acid sequence encoding CD4-His

AAGAAAGTGGTGCTGGGCAAAAAAGGGGATACAGTGGAACTG
ACCTGTACAGCTTCCCAGAAGAAGAGCATACAATTCCACTGGAAAAA
CTCCAACCAGATAAAGATTCTGGGAAATCAGGGCTCCTTCTTAACTAA
AGGTCCATCCAAGCTGAATGATCGCGCTGACTCAAGAAGAAGCCTTT
GGGACCAAGGAAACTTTCCCCTGATCATCAAGAATCTTAAGATAGAA
GACTCAGATACTTACATCTGTGAAGTGGAGGACCAGAAGGAGGAGGT
GCAATTGCTAGTGTTCGGATTGACTGCCAACTCTGACACCCACCTGC
TTCAGGGGCAGAGCCTGACCCTGACCTTGGAGAGCCCCCCTGGTAG
TAGCCCCTCAGTGCAATGTAGGAGTCCAAGGGGTAAAAACATACAGG
GGGGGAAGACCCTCTCCGTGTCTCAGCTGGAGCTCCAGGATAGTGG
CACCTGGACATGCACTGTCTTGCAGAACCAGAAGAAGGTGGAGTTCA
AAATAGACATCGTGGTGCTAGCTTTCCAGAAGGCCTCCAGCATAGTC
TATAAGAAAGAGGGGGAACAGGTGGAGTTCTCCTTCCCACTCGCCTT
TACAGTTGAAAAGCTGACGGGCAGTGGCGAGCTGTGGTGGCAGGCG
GAGAGGGCTTCCTCCTCCAAGTCTTGGATCACCTTTGACCTGAAGAA
CAAGGAAGTGTCTGTAAAACGGGTTACCCAGGACCCTAAGCTCCAGA
TGGGCAAGAAGCTCCCGCTCCACCTCACCCTGCCCCAGGCCTTGCC
TCAGTATGCTGGCTCTGGAAACCTCACCCTGGCCCTTGAAGCGAAAA
CAGGAAAGTTGCATCAGGAAGTGAACCTGGTGGTGATGAGAGCCACT
CAGCTCCAGAAAAATTTGACCTGTGAGGTGTGGGGACCCACCTCCCC
TAAGCTGATGCTGAGTTTGAAACTGGAGAACAAGGAGGCAAAGGTCT

CGAAGCGGGAGAAGGCGGTGTGGGTGCTGAACCCTGAGGCGGGGA
TGTGGCAGTGTCTGCTGAGTGACTCGGGACAGGTCCTGCTGGAATC
CAACATCAAGGTTCTGCCCACATGGCATCATCATCATCATCAT（SEQ
ID NO:73）

### Preparation Example 19: Expression and Purification of Fusion Protein TGFβRII-hFc

### 1. Synthesis of gene TGFβRII-hFc:

Fusion design was performed for the amino acid corresponding to the extracellular region TGFβRII-ECD of gene transforming growth factor beta receptor II (TGFβRII, NCBIGenBank:NP_001020018.1), Thrombin enzyme digestion sites, and Fc polypeptide fragment (hFc) of human IgG (SEQ ID NO:74). GENEWIZ was entrusted to synthesize the corresponding coding nucleic acid sequence (SEQ ID NO: 75).

TGFβRII: Transforming Growth Factor Beta Receptor II, NCBIGenBank:NP_001020018.1.

hFc: Ig gamma-1 chain C region, ACCESSION: P01857, 106-330.

Amino acid sequence of fusion protein TGFβRII-hFc:(370aa) 
TIPPHVQKSVNNDMIVTDNNGAVKFPQLCKFCDVRFSTCDNQKS
CMSNCSITSICEKPQEVCVAVWRKNDENITLETVCHDPKLPYHDFILEDA
ASPKCIMKEKKKPGETFFMCSCSSDECNDNIIFSEEYNTSNPDLVPRGSD
KTHTCPPCPAPELLGGPSVFLFPPKPKDTLMISRTPEVTCVVVDVSHEDP
EVKFNWYVDGVEVHNAKTKPREEQYNSTYRVVSVLTVLHQDWLNGKEY
KCKVSNKALPAPIEKTISKAKGQPREPQVYTLPPSREEMTKNQVSLTCLV
 
KGFYPSDIAVEWESNGQPENNYKTTPPVLDSDGSFFLYSKLTVDKSRW
QQGNVFSCSVMHEALHNHYTQKSLSLSPGK(SEQ ID NO:74),
 wherein, the double underline shows the ECD of TGFβRII; the wavy line shows Thrombin enzyme digestion sites, and the single underline shows hFc part.

### Nucleic acid sequence encoding the fusion protein TGFβRII-hFc: (1110bp).

ACGATCCCACCGCACGTTCAGAAGTCGGTTAATAACGACATGA
TAGTCACTGACAACAACGGTGCAGTCAAGTTTCCACAACTGTGTAAAT
TTTGTGATGTGAGATTTTCCACCTGTGACAACCAGAAATCCTGCATGA
GCAACTGCAGCATCACCTCCATCTGTGAGAAGCCACAGGAAGTCTGT
GTGGCTGTATGGAGAAAGAATGACGAGAACATAACACTAGAGACAGT
TTGCCATGACCCCAAGCTCCCCTACCATGACTTTATTCTGGAAGATGC
TGCTTCTCCAAAGTGCATTATGAAGGAAAAAAAAAAGCCTGGTGAGA
CTTTCTTCATGTGTTCCTGTAGCTCTGATGAGTGCAATGACAACATCA
TCTTCTCAGAAGAATATAACACCAGCAATCCTGACCTGGTGCCGAGG
GGAAGCGACAAAACTCACACATGCCCACCGTGCCCAGCACCTGAACT
CCTGGGGGGACCGTCAGTCTTCCTCTTCCCCCCAAAACCCAAGGACA
CCCTCATGATCTCCCGGACCCCTGAGGTCACATGCGTGGTGGTGGA
CGTGAGCCACGAAGACCCTGAGGTCAAGTTCAACTGGTACGTGGAC
GGCGTGGAGGTGCATAATGCCAAGACAAAGCCGCGGGAGGAGCAGT
ACAACAGCACGTACCGTGTGGTCAGCGTCCTCACCGTCCTGCACCA
GGACTGGCTGAATGGCAAGGAGTACAAGTGCAAGGTCTCCAACAAA
GCCCTCCCAGCCCCCATCGAGAAAACCATCTCCAAAGCCAAAGGGC
AGCCCCGAGAACCACAGGTGTACACCCTGCCCCCATCCCGGGAGGA
GATGACCAAGAACCAGGTCAGCCTGACCTGCCTGGTCAAAGGCTTCT

ATCCCAGCGACATCGCCGTGGAGTGGGAGAGCAATGGGCAGCCGGA
GAACAACTACAAGACCACGCCTCCCGTGCTGGACTCCGACGGCTCC
TTCTTCCTCTACAGCAAGCTCACCGTGGACAAGAGCAGGTGGCAGCA
GGGGAACGTCTTCTCATGCTCCGTGATGCATGAGGCTCTGCACAACC
ACTACACGCAGAAGAGCCTCTCCCTGTCTCCGGGTAAA (SEQ ID
NO:75)

### 2. Construction of pCMV-TGFβRII-hFc plasmid

The TGFβRII-hFc encoding gene synthesized by GENEWIZ was cloned into the pCMV expression vector (owned by the Company) to obtain pCMV-TGFβRII-hFc plasmid.

### 3. Transfection HEK293F cell with recombinant plasmid pCMV-TGFβRII-hFc

HEK293F cells (purchased from ThermoFisher Scientific) were transfected with the recombinant plasmid pCMV-TGFβRII-hFc following the protocol of lipofectamin transfection kit (purchased from Invitrogen).

### 4. SDS-PAGE electrophoresis detection of TGFβRII-hFc protein

Five days after the transfection, the culture solution was subjected to high-speed centrifugation, microporous membrane vacuum filtration, and purification with ProteinA/G column to obtain TGFβRII-hFc fusion protein samples. Some samples were added to loading buffer of the reduced protein electrophoresis for SDS-PAGE electrophoresis detection.

The fusion protein TGFβRII-hFc was prepared thereby.

### Preparation Example 20: Preparation of anti-CD4 Antibody Ibalizumab

The amino acid sequence of the VH and VL of the marketed CD4 monoclonal antibody Trogarzo (Ibalizumab) may refer to United States patent US005871732. GenScript was entrusted to synthesize the nucleic acid sequence encoding the VH and VL.

Amino acid sequence of Ibalizumab VH: (122aa)

QVQLQQSGPEVVKPGASVKMSCKASGYTFTSYVIHWVRQKPGQ
GLDWIGYINPYNDGTDYDEKFKGKATLTSDTSTSTAYMELSSLRSEDTAV
YYCAREKDNYATGAWFAYWGQGTLVTVSS (SEQ ID NO:76)

Nucleic acid sequence encoding Ibalizumab VH: (366bp)

CAGGTGCAACTGCAACAGTCCGGACCCGAAGTCGTGAAACCA
GGAGCTTCCGTGAAGATGAGCTGCAAAGCATCCGGATACACCTTCAC
CAGCTACGTGATCCACTGGGTGAGGCAGAAACCTGGCCAGGGCCTG
GACTGGATCGGCTACATCAACCCTTACAACGACGGAACCGACTACGA
CGAGAAATTCAAAGGCAAAGCTACCCTGACCAGCGACACCAGCACCT
CCACTGCTTACATGGAGCTGTCCAGCCTGAGGTCCGAAGACACCGCT
GTGTACTACTGCGCTAGGGAGAAGGACAACTACGCTACCGGCGCTT
GGTTCGCCTACTGGGGCCAGGGAACCCTGGTGACCGTGTCCAGC
(SEQ ID NO:77)

Amino acid sequence of Ibalizumab VL: (112aa)

DIVMTQSPDSLAVSLGERVTMNCKSSQSLLYSTNQKNYLAWYQQ
KPGQSPKLLIYWASTRESGVPDRFSGSGSGTDFTLTISSVQAEDVAVYY
CQQYYSYRTFGGGTKLEIK (SEQ ID NO:78)

Nucleic acid sequence encoding Ibalizumab VL: (336bp)

GACATCGTGATGACCCAGTCCCCTGACTCCCTGGCTGTGTCC
CTGGGCGAACGGGTCACCATGAACTGCAAATCTTCCCAGTCCCTGCT
GTACTCCACCAACCAGAAGAACTACCTGGCTTGGTACCAGCAGAAAC
CTGGCCAGTCCCCCAAACTGCTCATCTACTGGGCTTCCACCAGGGAA
AGCGGCGTGCCTGACAGATTCTCCGGAAGCGGCAGCGGAACCGACT
TCACCCTGACCATCTCCAGCGTGCAGGCTGAAGACGTGGCTGTCTAC
TACTGCCAGCAGTACTACAGCTACAGGACCTTCGGCGGAGGCACCA
AGCTGGAGATCAAG (SEQ ID NO:79)

The CH employed Ig gamma-4 chain C region, ACCESSION: P01861; the CL employed Ig kappa chain C region, ACCESSION:P01834.

The heavy chain cDNA and light chain cDNA of Ibalizumab were cloned into pCMV vectors respectively to obtain the recombinant expression plasmids encoding Ibalizumab.

HEK293F cells were transfected with the recombinant plasmids. HEK293F cell culture solution was purified and then tested.

Anti-CD4 monoclonal antibody Trogarzo (Ibalizumab) was prepared thereby.

### Preparation Example 21: Preparation and Detection of anti-TGF-β1 Antibody CAT192

Amino acid sequence of antibody CAT192 VH (123aa)

EVQLVESGGGVVQPGRSLRLSCAASGFTFSSYGMHWVRQAPGK
ELEWVAVISYDGSIKYYADSVKGRFTISRDNSKNTLYLQMNSLRAEDTAV
YYCARTGEYSGYDTDPQYSWGQGTTVTVSS (SEQ ID NO:80)

Nucleic acid sequence of antibody CAT192 VH (369bp)

GAGGTCCAGCTGGTGGAGTCTGGGGGAGGCGTGGTCCAGCC
TGGGAGGTCCCTGAGACTCTCCTGTGCAGCCTCTGGATTCACCTTCA
GTAGCTATGGCATGCACTGGGTCCGCCAGGCTCCAGGCAAGGAGCT
GGAGTGGGTGGCAGTTATATCATATGATGGAAGTATTAAATACTATGC
AGACTCCGTGAAGGGCCGATTCACCATCTCCAGAGACAATTCCAAGA
ACACGCTGTATCTGCAAATGAACAGCCTGAGAGCTGAGGACACGGCT
GTGTATTACTGTGCGCGAACTGGTGAATATAGTGGCTACGATACGGA
CCCCCAGTACTCCTGGGGGCAAGGGACCACGGTCACCGTCTCCTCA
(SEQ ID NO:81)

Amino acid sequence of the antibody CAT192 VL: (107aa)

EIVLTQSPSSLSASVGDRVTITCRSSQGIGDDLGWYQQKPGKAPI
LLIYGTSTLQSGVPSRFSGSGSGTDFTLTINSLQPEDFATYYCLQDSNYP
LTFGGGTRLEIK (SEQ ID NO:82)

Nucleic acid sequence of the antibody CAT192 VL: (321bp)

GAAATTGTGCTGACTCAGTCTCCATCCTCCCTGTCTGCATCTG
TAGGAGACAGAGTCACCATCACTTGCCGGTCAAGTCAGGGCATTGGA
GATGATTTGGGCTGGTATCAGCAGAAGCCAGGGAAAGCCCCTATCCT
CCTGATCTATGGTACATCCACTTTACAAAGTGGGGTCCCGTCAAGGT

TCAGCGGCAGTGGATCTGGCACAGATTTCACTCTCACCATCAACAGC
CTGCAGCCTGAAGATTTTGCAACTTATTACTGTCTACAAGATTCCAAT
TACCCGCTCACTTTCGGCGGAGGGACACGACTGGAGATTAAA (SEQ
ID NO:83)

The anti-TGF-β1 antibody CAT192 was prepared thereby.

### Embodiment 1: Comparison of Inhibition Effects of Bivalent Bifunctional Antibody CT101 and Monovalent Bifunctional Antibodies CT110, CT111, and CT112 on Activation of TGF-β1-induced HEK293 Cell TGF-β/Smad Signal

HEK293 cells were inoculated to a 24-well plate at a concentration of 1 x 10⁵/ml and 500 µL/well, cultured at an incubator under 37°C and 5% CO₂ for 24 hours, and then transfected with TGF-β/Smad promoter luciferase reporter gene plasmid (pSMAD-Luc) and renilla luciferase control plasmid (pRL-TK). 24 hours after transfection, CT101, CT110, CT111, and CT112 antibodies with different concentrations were added and cultured for 12 hours. After 12 hours, the culture medium was discarded and the cells in each well were lysed with 100µL 1 x Passive Lysis Buffer (purchased from Promega). 70 µL of lysate was placed into an ELISA plate, 30 µL of substrate 1 solution was added, the ELISA plate was immediately put into the ELISA reader, and Luminescence was selected to read the value of each well of the ELISA plate as An. 30 µL of substrate 2 solution was added, the ELISA plate was immediately put into the ELISA reader, and Luminescence was selected to read the value of each well of the ELISA plate as Bn. The data was analyzed and processed with SoftMax Pro. The ratio of An/Bn was the activation fold of TGF-β/SMAD signal pathway.

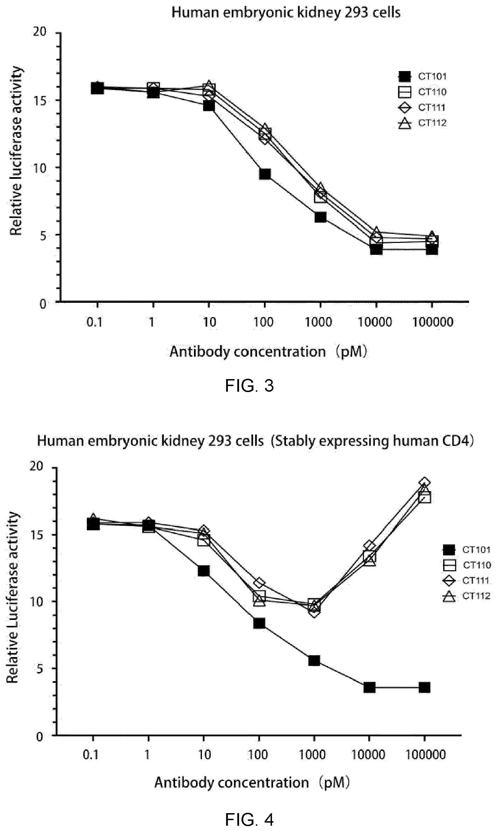

The results are shown in FIG. 3. The results show that antibodies CT101, CT110, CT111, and CT112 can effectively inhibit the activation of TGF-β/SMAD signal pathway induced by TGF-β1 in a dose-dependent manner, at the same dose, the pharmacological activity of CT101 on the inhibition of TGF-β/Smad signal pathway induced by TGF-β1 is slightly superior to that of CT110, CT111, and CT112. The IC₅₀ of CT101, CT110, CT111, and CT112 are about 250, 750, 800, and 1000 pM, respectively.

### Embodiment 2: Comparison of Inhibition Effects of Bivalent Bifunctional Antibody CT101 and Monovalent Bifunctional Antibodies CT110, CT111, and CT112 on Activation of TGF-β1-induced HEK293-CD4 Cell TGF-β/Smad Signal

HEK293-CD4 cells were inoculated to a 24-well plate at a concentration of 1 x 10⁵/ml and 500 µL/well, cultured at an incubator under 37°C and 5%CO₂ for 24 hours, and then transfected with TGF-β/Smad promoter luciferase reporter gene plasmid (pSMAD-Luc) and rellina luciferase control plasmid (pRL-TK). 24 hours after transfection, CT101, CT110, CT111, and CT112 antibodies with different concentrations were added and cultured for 12 hours. After 12 hours, the culture medium was discarded and the cells in each well were lysed with 100µL 1 x Passive Lysis Buffer (purchased from Promega). 70 µL of lysate was placed into an ELISA plate, 30 µL of substrate 1 solution was added, the ELISA plate was immediately put into the ELISA reader, and Luminescence was selected to read the value of each well of the ELISA plate as An. 30 µL of substrate 2 solution was added, the ELISA plate was immediately put into the ELISA reader, and Luminescence was selected to read the value of each well of the ELISA plate as Bn. The data was analyzed and processed with SoftMax Pro. The ratio of An/Bn was the activation fold of TGF-β/SMAD signal pathway.

The results are shown in FIG. 4. The results show that only antibody CT101 can effectively inhibit the activation of TGF-β/Smad signal pathway induced by TGF-β1, and the inhibition effect of CT101 is in a dose-dependent manner. The monovalent bifunctional antibody CT110, CT111, and CT112 can partially inhibit the activity of TGF-β1 in a dose-dependent manner within the concentration range of 0.1-1000 PM, however, increase the activity of TGF-β1 in a dose-dependent manner when the concentration is above 1000 pM. The IC₅₀ value of CT101 for effectively inhibiting TGF-β1-induced activation of TGF-β/Smad signal pathway in HEK93-CD4 cells is 15 pM.

### Embodiment 3: Comparison of Inhibition Effects of Bifunctional Antibodies CT101, CT102, CT103, CT104, CT105, CT106, CT107, CT108, and CT109 on Activation of TGF-β1-induced HEK293-CD4 Cell TGF-β/Smad Signal

HEK293-CD4 cells were inoculated to a 24-well plate at a concentration of 1 x 10⁵/ml and 500 µL/well, cultured at an incubator under 37°C and 5%CO₂ for 24 hours, and then transfected with TGF-β/Smad promoter luciferase reporter gene plasmid (pSMAD-Luc) and renilla luciferase control plasmid (pRL-TK). 24 hours after transfection, bifunctional antibodies CT101, CT102, CT103, CT104, CT105, CT106, CT107, CT108, and CT109 with different concentrations were added and cultured for 12 hours. After 12 hours, the culture medium was discarded and the cells in each well were lysed with 100 µL 1 x Passive Lysis Buffer (purchased from Promega). 70 µL of lysate was placed into an ELISA plate, 30 µL of substrate 1 solution was added, the ELISA plate was immediately put into the ELISA reader, and Luminescence was selected to read the value of each well of the ELISA plate as An. 30 µL of substrate 2 solution was added, the ELISA plate was immediately put into the ELISA reader, and Luminescence was selected to read the value of each well of the ELISA plate as Bn. The data was analyzed and processed with SoftMax Pro. The ratio of AN/BN was the activation fold of TGF-β/SMAD signal pathway.

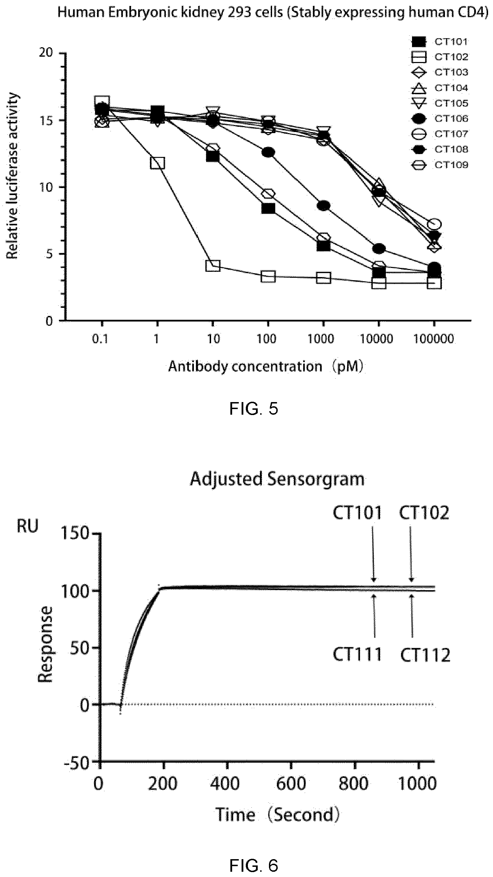

The results are shown in FIG. 5. The results show that bifunctional antibodies CT101, CT102, CT103, CT104, CT105, CT106, CT107, CT108, and CT109 can effectively inhibit the activation of TGF-β/SMAD signal pathway induced by TGF-β1 in a dose-dependent manner, at the same dose, the pharmacological activity of CT102 on the inhibition of TGF-β/Smad signal pathway induced by TGF-β1 is significantly superior to that of other antibodies, with an IC₅₀ value below 2 pM. The IC₅₀ values of other bifunctional antibodies CT101, CT103, CT104, CT105, CT106, CT107, CT108, and CT109, which effectively inhibit the activation of TGF-β/Smad signal pathway induced by TGF-β1, are 15 pM, 2500 pM, 2400 pM, 2500 pM, 300 pM, 2600 pM, 2700 pM, and 50 pM, respectively.

### Embodiment 4: Determination of Kinetics Parameters of Antibodies CT101, CT102, CT111, and CT112

### 1. Determination of kinetics parameters of bifunctional antibodies CT101, CT102, CT111, and CT112 binding to CD4

The affinity constants of CT101, CT102, CT111, and CT112 with human CD4 were measured using Biacore system for molecular interaction. HBS-EP served as buffer, human CD4 was immobilized on the surface of CM5 chip by Bicaore standard amino coupling. Binding antibodies CT101, CT102, CT111, and CT112 to human CD4 was performed, where the concentration of antibodies CT101, CT102, CT111, and CT112 was 250 nM, the flow rate was 30 µl/min, the binding duration was 120 s, and the dissociation duration was 880 s. The chip was regenerated by using 10 mM glycine, where PH was 2.7, the flow rate was 30 µl/min, and the duration was 120 s. The affinity constants were obtained by data fitting analysis with a 1:1 model. Data was collected with Biacore Control 2.0 software and analyzed using Biacore T200 Evaluation 2.0 software. The kinetics parameters of antibodies CT101, CT102, CT111, and CT112 binding to human CD4 are shown in Table 1, and the test results of specific kinetic parameters of antibodies CT101, CT102, CT111, and CT112 binding to human CD4 are shown in FIG. 6.

**Table 1: Kinetics parameters of antibodies CT101, CT102, CT111, and CT112 binding to human CD4**

| Antibody name | Kon (10⁴/Ms) | Koff (10⁻⁵/s) | Kd (Koff/Kon, nM) |
|---|---|---|---|
| CT101 | 3.8 | 0.03 | 0.01 |
| CT102 | 4.0 | 0.03 | 0.01 |
| CT103 | 2.9 | 0.09 | 0.03 |
| CT104 | 3.0 | 0.09 | 0.03 |

Kd is the affinity constant; Kon is the binding rate of antigen and antibody; Koff is the dissociation rate of antigen and antibody; Kd= Koff/Kon.

The results show that bivalent antibodies CT101 and CT102 show high affinities with antigen CD4, which are higher than those of monovalent antibodies CT111 and CT112.

2. Determination of kinetics parameters of antibodies CT101, CT102, CT111, and CT112 binding to TGF-β1

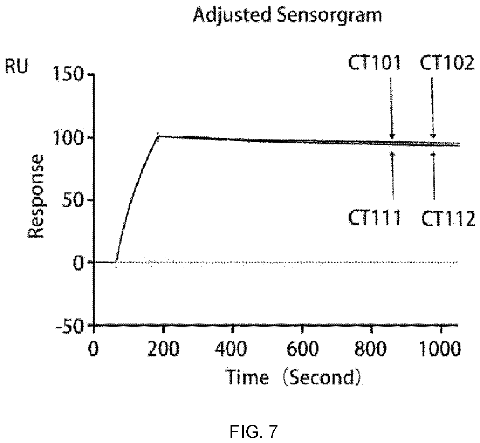

The affinity constants of CT101, CT102, CT111, and CT112 with human TGF-β1 were measured with a Biacore molecular interaction meter. HBS-EP served as buffer, human TGF-β1 was immobilized on the surface of CM5 chip by Bicaore standard amino coupling. Binding of antibodies CT101, CT102, CT111, and CT112 to human TGF-β1 was performed, where the concentration of antibodies CT101, CT102, CT111, and CT112 was 250 nM, the flow rate was 30 µl/min, the binding duration was 120 s, and the dissociation duration was 880 s. The chip was regenerated by using 10 mM glycine, where PH was 2.7, the flow rate was 30 µl/min, and the duration was 120 s. The affinity constants were obtained by data fitting analysis with a 1:1 model. Data was collected with Biacore Control 2.0 software and analyzed using Biacore T200 Evaluation 2.0 software. The kinetics parameters of antibodies CT101, CT102, CT111, and CT112 binding to human TGF-β1 are shown in Table 2, and the test results of specific kinetic parameters of antibodies CT101, CT102, CT111, and CT112 binding to human TGF-β1 are shown in FIG. 7.

Table 2: Kinetics parameters of antibodies CT101, CT102, CT111, and CT112 binding to TGF-β1

| Antibody name | Kon (10⁴/Ms) | Koff (10⁻⁵/s) | Kd (Koff/Kon, nM) |
|---|---|---|---|
| CT101 | 2.8 | 2.1 | 0.75 |
| CT102 | 2.6 | 1.9 | 0.73 |
| CT111 | 2.9 | 5.8 | 2.00 |
| CT112 | 3.0 | 5.9 | 1.97 |

The results show that bivalent antibodies CT101 and CT102 show high affinities with antigen TGF-β1, which are higher than those of univalent antibodies CT111 and CT112.

### Embodiment 5: Binding of CT101, CT102, TGF-β1 antibodies and control antibody to helper T cells from humanized CD4 mice

Human CD4 molecules were specifically expressed on CD4 helper T cells from humanized CD4 mice. Flow cytometry was used to determine the specific binding ability of antibodies to CD4 helper T cells *in vivo.*

200 µg of CT101, CT102, TGF-β1 antibodies and control antibody were injected into humanized CD4 mice via tail vein. The mice were killed by euthanasia 4 hours later, the lymph nodes of the mice were taken out, crushed with a homogenizer, and filtered the tissue fragments. The cells were collected, placed on ice, and incubated with the antibody mixture for half an hour. The antibody mixture was composed of FITC-conjugated rat anti-mouse CD45 IgG (1:400), APC-conjugated rat anti-mouse TCRβ IgG (1:200), APC-Cy7-conjugated rat anti-mouse CD4 IgG (1:200), PE-Cy7-conjugated rat anti-mouse CD8 IgG (1:200), and PE-conjugated goat anti human Fc IgG (1:400), which was diluted with 1 x FACS buffer (1% BSA, 2mM EDTA, 0.1% sodium azide). The cells were washed with PBS, 200 µL of PBS was added to resuspend the cells, and fluorescence signals (MFI, mean fluorescent intensity) were detected by flow cytometry.

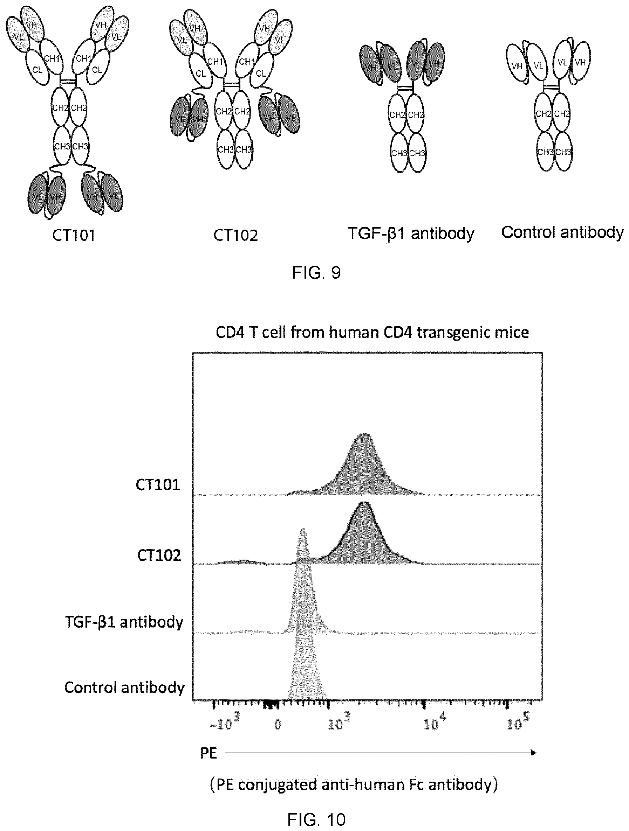

Results are shown in FIG. 10. Antibodies CT101 and CT102 can effectively attach to helper T cells by binding to CD4 molecules, thus specifically inhibiting the activation of TGF-β signal pathway on helper T cells. However, TGF-β1 antibody and control antibody can't bind to CD4 molecule, so that they can't attach to helper T cells.

### Embodiment 6: Metabolism of CT101 and CT102 in humanized CD4 mice under high-dose intravenous injection

In order to detect the metabolism of CT101 and CT102 in humanized CD4 mice under high-dose intravenous injection, 200 µg of CT101 or CT102 antibody was injected into humanized CD4 mice aged 8-10 weeks through the tail vein, blood samples were collected to detect the serum concentration of the antibodies every 24 hours.

### Determining the concentrations of antibodies CT101 and CT102 in serum by ELISA as follows:

The ELISA plate was coated with CD4-His and incubated at 37°C for 3 hours. After rinsing the plate, 1%BSA was used for blocking for one hour. After rinsing the plate, diluted serum was added and incubated at 37°C for 60 minutes. After rinsing the plate, enzyme-labeled donkey anti-human IgG secondary antibody working solution was added and incubated at 37°C for 30 minutes. After rinsing the plate, TMB developing solution was added to develop for 5 minutes in a dark place, and termination solution was added to terminate the chromogenic reaction. The ELISA plate was immediately put into an ELISA reader, and the optical wavelength of 450 nm was selected to read the OD value of each well of the ELISA plate. The data was analyzed and processed with SoftMax Pro software.

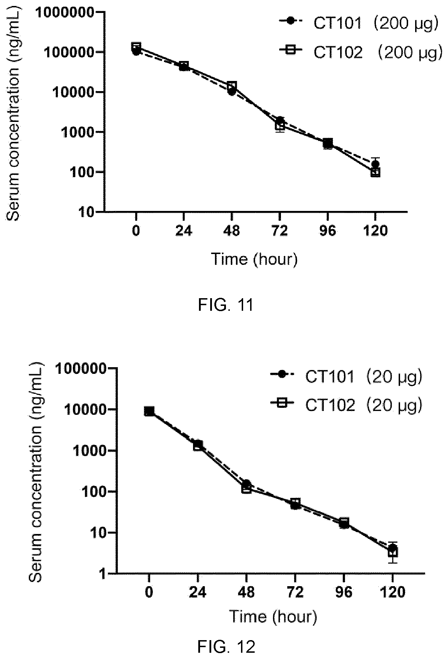

The results of metabolism of CT101 and CT102 in humanized CD4 mice under high-dose intravenous injection are shown in FIG. 11. Curve fitting with the time after antibody injection as the X-axis and serum concentration as Y-axis was performed. The calculated half-life period t1/2 of antibodies CT101 and CT102 are 60 hours.

### Embodiment 7: Metabolism of CT101 and CT102 in humanized CD4 mice under low-dose intravenous injection

In order to detect the metabolism of CT101 and CT102 in humanized CD4 mice under low-dose intravenous injection, 20 µg of CT101 or CT102 antibody was injected into humanized CD4 mice aged 8-10 weeks through the tail vein, blood samples were collected to detect the serum concentration of the antibodies every 24 hours.

Determining the concentrations of antibodies CT101 and CT102 in serum by ELISA as follows:

The ELISA plate was coated with CD4-His and incubated at 37°C for 3 hours. After rinsing the plate, 1%BSA was used for blocking for one hour. After rinsing the plate, diluted serum was added and incubated at 37°C for 60 minutes. After rinsing the plate, enzyme-labeled donkey anti-human IgG secondary antibody working solution was added and incubated at 37°C for 30 minutes. After rinsing the plate, TMB developing solution was added to develop for 5 minutes in a dark place, and termination solution was added to terminate the chromogenic reaction. The ELISA plate was immediately put into an ELISA reader, and the optical wavelength of 450nm was selected to read the OD value of each well of the ELISA plate. The data was analyzed and processed with SoftMax Pro software.

The results of metabolism of CT101 and CT102 in humanized CD4 mice under low-dose intravenous injection are shown in FIG. 12. Curve fitting with the time after antibody injection as the X-axis and serum concentration as Y-axis was performed. The calculated half-life periods t1/2 of antibodies CT101 and CT102 are 30 and 32 hours, respectively.

### Embodiment 8: In vivo experiment of tumor growth inhibition effects of antibody treatment at a high dose

In order to determine the *in vivo* antitumor activity of CT101, CT102, TGF-β1 antibodies, and control antibody at a high dose, MC38 colon cancer cells were inoculated subcutaneously into female humanized CD4 mice aged 8-10 weeks. Then, 200µg of the drug was administered every 5 days by tail vein injection, and for 4 times in total. After drug administration, the length and width of tumor in each group were measured and the tumor volume was calculated.

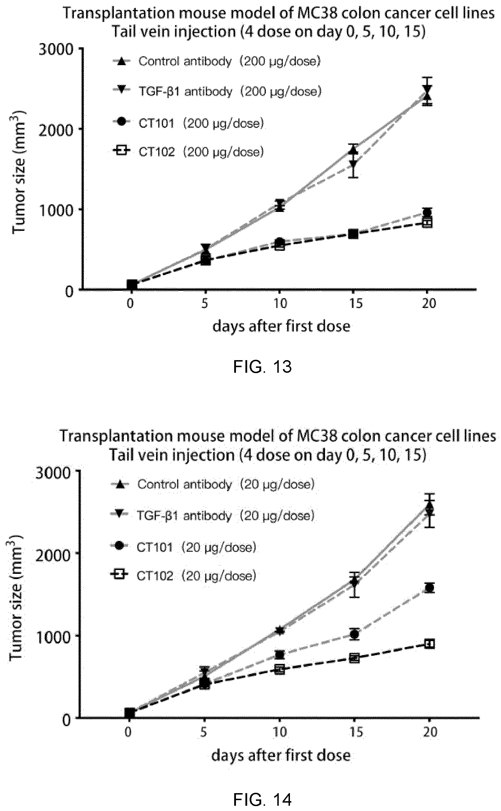

The results are shown in FIG. 13. The results indicate that both CT101 and CT102 inhibit mouse tumor growth more effectively compared with control antibody and TGF-β1 antibody at a high dose, and their inhibiting effects are comparable.

### Embodiment 9: In vivo experiment of tumor growth inhibition effects of antibody treatment at a low dose

In order to determine the *in vivo* antitumor activity of CT101, CT102, TGF-β1 antibodies, and control antibody at a low dose, MC38 colon cancer cells were inoculated subcutaneously into female humanized CD4 mice aged 8-10 weeks. Then, 20µg of the drug was administered every 5 days by tail vein injection, and for 4 times in total. After drug administration, the length and width of tumor in each group were measured and the tumor volume was calculated.

The results are shown in FIG. 14. The results indicate that both CT101 and CT102 inhibit mouse tumor growth more effectively compared with control antibody and TGF-β1 antibody at a low dose, and the anti-tumor effects of CT102 are superior to that of CT101.

### Embodiment 9: In vivo experiment of tumor growth inhibition effects of different bifunctional antibodies

In order to determine the *in vivo* antitumor activity of different bifunctional antibodies, first EMT-6 breast cancer cells were subcutaneously inoculated to female BALB/c mice aged 6-8 weeks. Then, 50 µg of the drug was administered every 5 days by tail vein injection, and for 5 times in total. After drug administration, the length and width of tumor in each group were measured and the tumor volume was calculated.

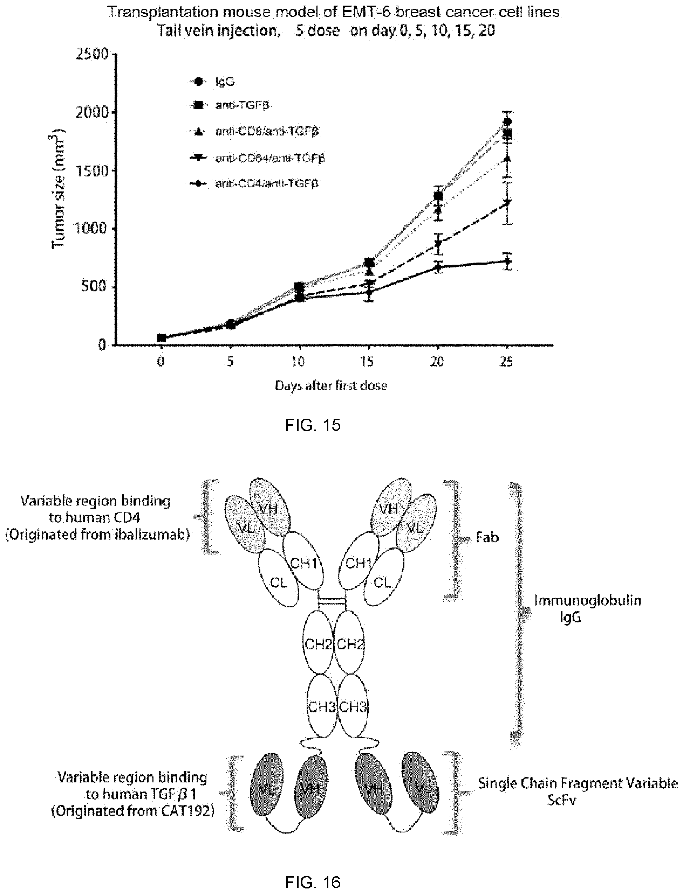

The results are shown in FIG. 15. The results indicate that compared with the control antibody IgG and anti-TGFβ (clone 1D11) antibody, anti-CD4/anti-TGFβ demonstrates the strongest inhibition effect on mouse tumor growth, and anti-CD64/anti-TGFβ shows second strong inhibition effect. Compared with anti-TGFβ antibody, anti-CD8/anti-TGFβ has no obvious improved effect on mouse tumor growth.

### Embodiment 2: Sequence design, preparation, and test of the heavy chain and light chain of bifunctional antibody CT101

### 1. Sequence design

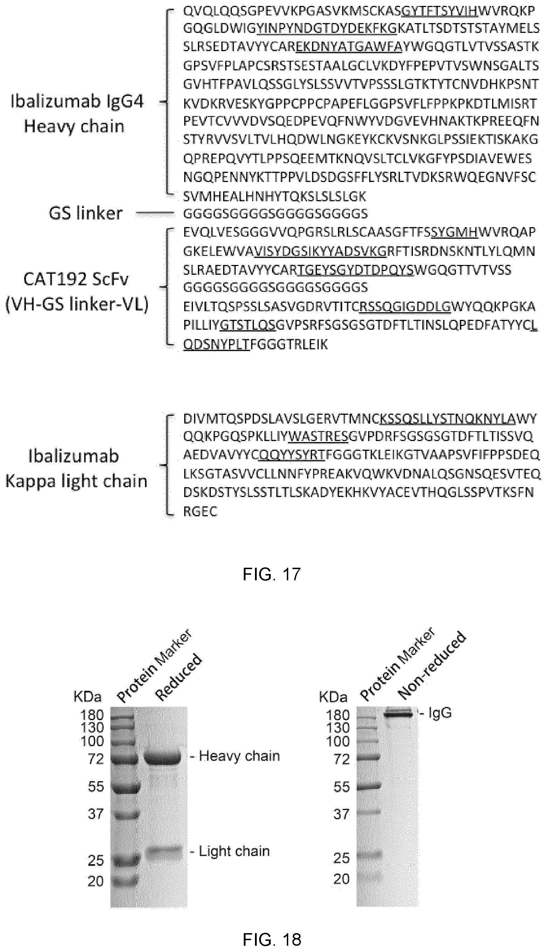

The structure mode of the bifunctional antibody CT101 in the present invention is Morrison mode (IgG-ScFv), that is, the C-terminals of two heavy chains of one IgG antibody are linked with the ScFv fragments of another antibody. The main composition design and sequence information of its heavy chain and light chain are shown in FIG. 16 and FIG. 17.

### 2. Expression and purification of antibody CT101

Heavy chain cDNA sequence and light chain cDNA sequence of CT101 were cloned to pCMV vector. The recombinant plasmid was extracted and used to transfect HEK293F cells. After the cells were cultured for five days, the culture solution was subjected to high-speed centrifugation and supernatant concentration, and loaded to ProteinA/G column, and the protein was eluted using Elution Buffer. The target sample antibody CT101 was recovered and transferred to PBS.

### 2. Test of antibody CT101

The purified antibody CT101 was added into loading buffer of reduced protein electrophoresis and non-reduced protein electrophoresis and boiled to carry out SDS-PAGE electrophoresis detection. The electrophoretogram of CT101 is shown in FIG. 18. The target protein band in reduced protein samples is at 75 kD and 25 kD, and that in non-reduced protein samples (single antibody) is at 200 kD.

### 3. High-performance liquid chromatography analysis of antibody CT101

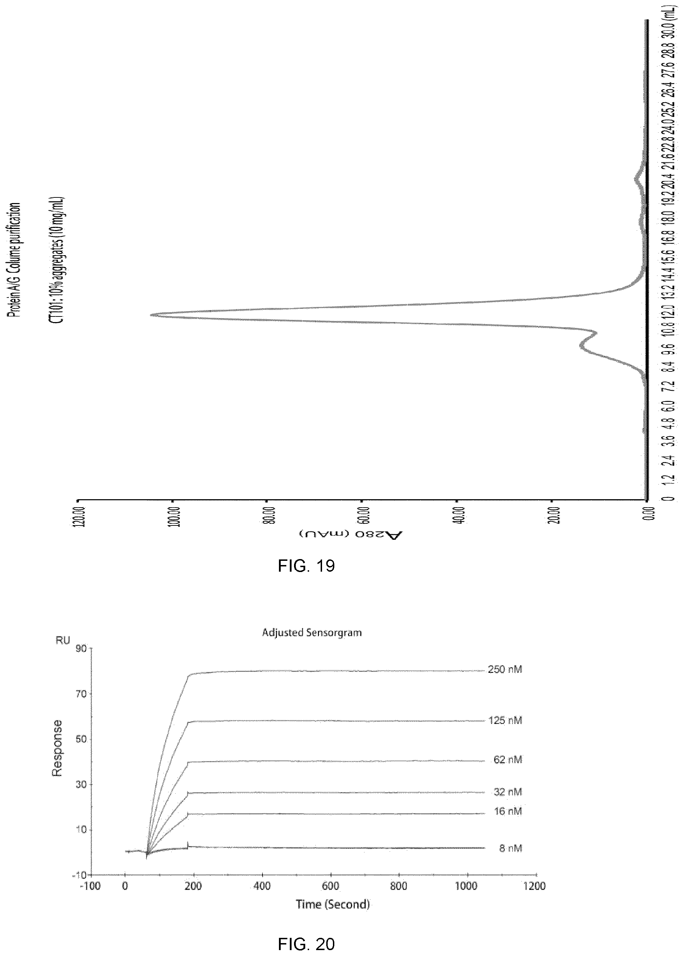

The purified antibody CT101 flowed through a Superdex S200 10/300 GL column (GE) on an AKTA purifier, and PBS solution flowed through the system at 0.5 mL/min. The high-performance liquid chromatography analysis result of antibody CT101 is shown in FIG. 19. CT101 mainly exists in monomer form (90%), and 10% exists in polymer form.

The humanized antibody CT101 used in the following experiment was prepared following the protocol of this example unless specifically stated.

### Embodiment 10: Determination of kinetics parameters of antibody CT101

### 1. Determining the kinetics parameters of bifunctional antibody CT101 binding to CD4

The affinity constants of CT101 with human CD4 were measured with a Biacore system for molecular interaction. HBS-EP served as buffer, and human CD4 was immobilized on the surface of CM5 chip by Bicaore standard amino coupling. Binding of antibody CT101 to human CD4 was performed, where the concentration of antibody CT101 was 8-250 nM (2 fold dilution), the flow rate was 30 µl/min, the binding duration was 120 s, and the dissociation duration was 880 s. The chip was regenerated using 10 mM glycine, where PH was 2.7, the flow rate was 30 µl/min, and the duration was 120 s. The affinity constants were obtained by data fitting analysis with a 1:1 model. Data was collected with Biacore Control 2.0 software and analyzed using Biacore T200 Evaluation 2.0 software. The kinetics parameters of antibodies CT101 and Ibalizumab binding to human CD4 are shown in Table 1, and the test results of specific kinetic parameters of antibody CT101 binding to human CD4 are shown in FIG. 20, respectively.

**Table 1: Kinetics parameters of humanized antibody CT101 and Ibalizumab binding to human CD4 molecule**

| Antibody name | Kon (10⁴/Ms) | Koff (10⁻⁵/s) | Kd (Koff/Kon, nM) |
|---|---|---|---|
| CT101 | 3.8 | 0.03 | 0.01 |
| Ibalizumab | 3.0 | 0.15 | 0.05 |

Kd is the affinity constant; Kon is the binding rate of antigen and antibody; Koff is the dissociation rate of antigen and antibody; Kd= Koff/Kon.

The results show that antibody CT101 demonstrates a high affinity with its antigen, which is slightly higher than that of control antibody Ibalizumab.

### 2. Determining the kinetics parameters of bifunctional antibody CT101 binding to TGF-β1

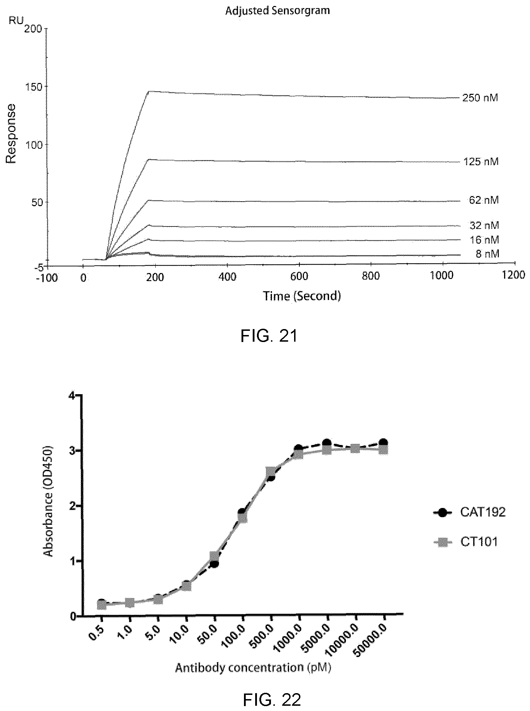

The affinity constants of CT101 with human TGF-β1 were measured with a Biacore system for molecular interaction. HBS-EP served as buffer, human TGF-β1 was immobilized on the surface of CM5 chip by Bicaore standard amino coupling. Binding of antibody CT101 to human TGF-β1 was performed, where the concentration of antibody CT101 was 8-250 nM (2 fold dilution), the flow rate was 30 µl/min, the binding duration was 120 s, and the dissociation duration was 880 s. The chip was regenerated using 10 mM glycine, where PH was 2.7, the flow rate was 30 µl/min, and the duration was 120 s. The affinity constants were obtained by data fitting analysis with a 1:1 model. Data was collected with Biacore Control 2.0 software and analyzed using Biacore T200 Evaluation 2.0 software. The kinetics parameters of antibodies CT102 and CAT192 binding to human TGF-β1 are shown in Table 2, and the test results of specific kinetic parameters of antibody CT101 to human TGF-β1 are shown in FIG. 21.

**Table 2: Kinetics parameters of antibodies CT101 and CAT192 binding to TGF-β1**

| Antibody name | Kon (10⁴/Ms) | Koff (10⁻⁵/s) | Kd (Koff/Kon, nM) |
|---|---|---|---|
| CT101 | 2.8 | 2.1 | 0.75 |
| CAT192 | 3.0 | 2.4 | 0.80 |

The results show that the antibodies CT101 and CAT192 both demonstrate high affinities with antigen TGF-β1

### Embodiment 11: Determination of binding activity of antibody CT101 to antigen by ELISA

1. Determining the binding activity of antibodies CT101 and CAT192 to antigen TGFβ1-His by indirect ELISA.

### The method is detailed as follows:

The ELISA plate was coated with TGF-β1-His and incubated at 37°C for 3 hours. After rinsing the plate, 1%BSA was used for blocking for one hour. After rinsing the plate, serially diluted antibody was added and incubated at 37°C for 60 minutes. After rinsing the plate, enzyme-labeled donkey anti-human IgG secondary antibody working solution was added and incubated at 37°C for 30 minutes. After rinsing the plate, TMB developing solution was added to develop for 5 minutes in a dark place, and termination solution was added to terminate the chromogenic reaction. The ELISA plate was immediately put into an ELISA reader, and the optical wavelength of 450nm was selected to read the OD value of each well of the ELISA plate. The data was analyzed and processed with SoftMax Pro software.

The results of antibody CT101 binding to antigen TGF-β1-His are shown in FIG. 22. Curve fitting with antibody concentration as the X-axis and absorbance value as the Y-axis was performed. The calculated binding constants EC₅₀ of the antibodies are shown in Table 3 below.

**Table 3: Binding of antibodies CT101 and CAT192 to TGF-β1-His (indirect ELISA)**

| | CT101 | CAT192 |
|---|---|---|
| EC₅₀ | 85.2 pM | 84.5 pM |

The results show that both antibodies CT101 and CAT192 can effectively bind to TGF-β1 protein, and the binding efficiency is dose-dependent.

2. Determining the binding activity of antibodies CT101 and Ibalizumab to CD4 by indirect ELISA.

### The method is detailed as follows:

The ELISA plate was coated with CD4-His and incubated at 37°C for 3 hours. After rinsing the plate, 1%BSA was used for blocking for one hour. After rinsing the plate, serially diluted antibody was added and incubated at 37°C for 60 minutes. After rinsing the plate, enzyme-labeled donkey anti-human IgG secondary antibody working solution was added and incubated at 37°C for 30 minutes. After rinsing the plate, TMB developing solution was added to develop for 5min in a dark place, and termination solution was added to terminate the chromogenic reaction. The ELISA plate was immediately put into an ELISA reader, and the optical wavelength of 450nm was selected to read the OD value of each well of the ELISA plate. The data was analyzed and processed with SoftMax Pro software.

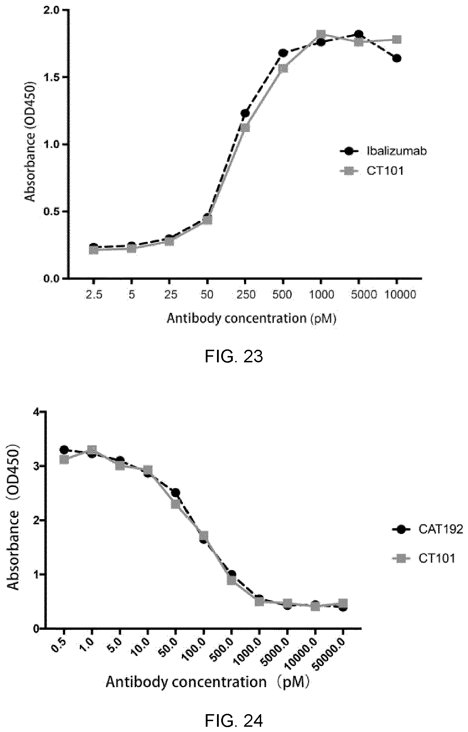

The binding of antibody CT101 to antigen CD4 is shown in FIG. 23. Curve fitting with antibody concentration as the X-axis and absorbance value as the Y-axis was performed. The calculated binding constants of the antibodies are shown in Table 4 below.

**Table 4: Binding of antibodies CT101 and Ibalizumab to CD4-His (indirect ELISA)**

| | CT101 | Ibalizumab |
|---|---|---|
| EC₅₀ | 255.5 pM | 250.4 pM |

The results show that both antibodies CT101 and Ibalizumab can effectively bind to CD4 protein, and the binding efficiency is dose-dependent.

### 3. Determining competitive binding activity of antibodies CT101, CAT192 and TGFβRII to TGF-β1 by competitive ELISA method

### The method is detailed as follows:

The ELISA plate was coated with TGF-β1-His and incubated at 37°C for 3 hours. After rinsing the plate, 1%BSA was used for blocking at 37°C for one hour. After rinsing the plate, serially diluted antibody and human TGFβRII-ECD-Fc-biotin (final concentration 0.1µg/ml) were added and incubated at room temperature for 2 hours. After rinsing the plate, HRP labeled streptavidin SA-HRP (1:2000) working solution was added and incubated at 37°C for 30 minutes. After rinsing the plate, TMB developing solution was added to develop for 5 min in a dark place, and termination solution was added to terminate the chromogenic reaction. The ELISA plate was immediately put into an ELISA reader, and the optical wavelength of 450nm was selected to read the OD value of each well of the ELISA plate. The data was analyzed and processed with SoftMax Pro software.

The results are shown in FIG. 24. Through quantitative analysis of the light absorption intensity of the antibody CT101 being bound, a curve simulation of the binding efficiency of the antibody was performed to obtain the binding constant EC₅₀ (Table 5).

**Table 5: Detection of binding of antibodies, competing with TGFβRII-Fc, to the antigen TGF-β1-His by competitive ELISA**

| | CT101 | CAT192 |
|---|---|---|
| IC₅₀ | 105.2 pM | 105.5 pM |

The results show that both antibodies CT101 and CAT192 can effectively bind to TGF-β1, and inhibit TGFβRII binding to TGF-β1. The inhibition efficiencies are dose-dependent.

### Embodiment 12: Binding of antibody CT101 to cell membrane surface antigen CD4

Firstly, the HEK293 cell lines expressing human CD4 antigen were constructed and then the specific binding ability of the antibody to cell membrane antigen CD4 was analyzed and verified by flow cytometry.

### 1. Constructing the HEK293 cell lines expressing CD4 antigen

HEK293 cells were transfected with the lentiviral vector pHAGE-CD4-IRES-EGFP and helper plasmids psPAX2, pMD2.G. The supernatant was collected 48 hours later, filtered with a 0.45 µM filter membrane, and then infection of fresh HEK293 cells was performed. 48 hours post infection, the EGFP was detected by flow cytometry, and the clonal population of HEK293-CD4 cells stably expressing CD4 was sorted out.

### 2. Determining the binding of antibody CT101 to cell membrane antigen

The HEK293-CD4 cells expressing CD4 antigen obtained in the above steps were digested by conventional trypsin digestion. 2×10⁵ cells were placed in each collecting tube, and antibodies with serially diluted concentrations were prepared using PBS (containing 1 %BSA) and incubated with HEK293-CD4 cells on ice for half an hour. 100 µL of FITC-conjugated goat anti-human IgG (1:200) was added to incubate on ice for half an hour, washed with PBS, 200 µL of PBS was added to resuspend the cells, and the fluorescence signals (MFI, mean fluorescent intensity) were detected using the FITC channel of flow cytometry.

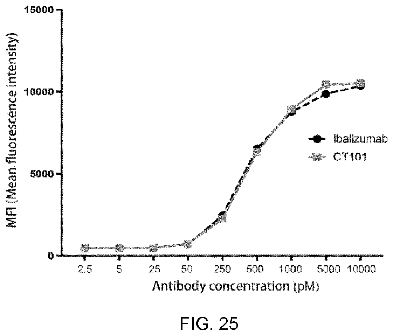

The results are shown in FIG. 25. According to fluorescence quantitative analysis and curve fitting of the CT101 antibody and Ibalizumab that are bound, the calculated EC₅₀ of CT101 antibody and Ibalizumab are shown in Table 6.

**Table 6: Fluorescence intensity analysis of CT101 binding to HEK293-CD4 surface antigen by FACS**

| | CT101 | CAT192 |
|---|---|---|
| EC₅₀ | 310.5 pM | 312.5 pM |

The results show that CT101 antibody can effectively bind to CD4 antigen on the surface of HEK293-CD4 cells in a dose-dependent manner. The binding activity of Ibalizumab to CD4 antigen on the surface of HEK293-CD4 cells is similar to that of CT101, indicating that the anti-CD4 function of the bifunctional antibody CT101 is not affected by antibody modification.

### Embodiment 13: Inhibition effect of CT101 antibody on activation of TGF-β1-induced TGF-β/Smad signal pathway in HEK93 cells

HEK293 cells were inoculated to a 24-well plate at a concentration of 1 × 10⁵/ml and 500 µL/well, cultured at an incubator under 37°C and 5%CO₂ for 24h, and then were transfected with TGF-β/Smad promoter luciferase reporter gene plasmid (pSMAD-Luc) and renilla luciferase control plasmid (pRL-TK). 24 hours after transfection, CT110 antibody and CAT192 antibody with different concentrations were added and cultured for 12 hours. After 12 hours, the culture medium was discarded and the cells in each well were lysed with 100µL 1 × Passive Lysis Buffer (purchased from Promega). 70 µL of lysate was placed into an ELISA plate, 30 µL of substrate 1 solution was added, the ELISA plate was immediately put into the ELISA reader, and Luminescence was selected to read the value of each well of the ELISA plate as An. 30 µL of substrate 2 solution was added, the ELISA plate was immediately put into the ELISA reader, and Luminescence was selected to read the value of each well of the ELISA plate as Bn. The data was analyzed and processed with SoftMax Pro software. The ratio of An/Bn was the activation fold of TGF-β/SMAD signal pathway.

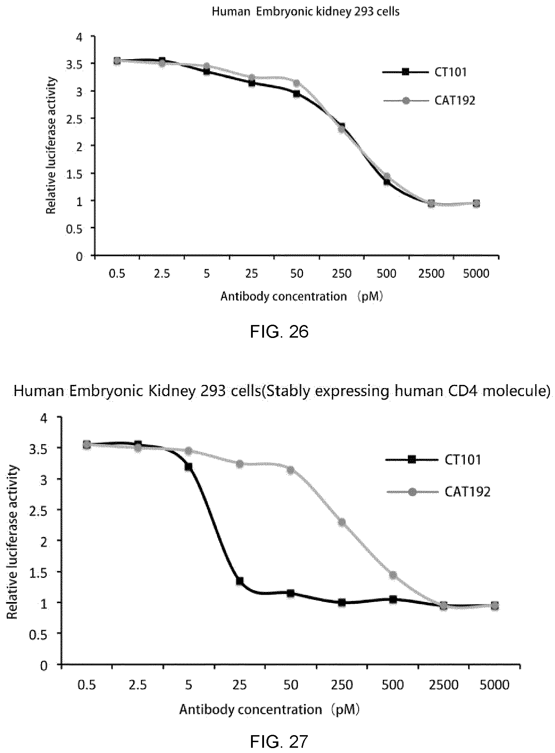

The results are shown in FIG. 26. The results show that antibodies CT101 andCAT192 can effectively inhibit the activation of TGFβ/Smad signal pathway induced by TGF-β1 in a dose-dependent manner, at the same dose, the pharmacological activity of CT101 on the inhibition of TGFβ/Smad signal pathway induced by TGF-TGF-β1 is similar to that of CAT192, with IC₅₀ about 250 pM.

### Embodiment 14: Inhibition effects of CT101 antibody on activation of TGF-β1-induced TGF-β/Smad signal pathway in HEK293-CD4 cells

HEK293-CD4 cells were inoculated to a 24-well plate at a concentration of 1 × 10⁵/ml and 500 µL/well, cultured at an incubator under 37°C and 5%CO₂ for 24h, and then were transfected with TGF-β/Smad promoter luciferase reporter gene plasmid (pSMAD-Luc) and renilla luciferase control plasmid (pRL-TK). 24 hours after transfection, CT110 antibody and CAT192 antibody with different concentrations were added and cultured for 12 hours. After 12 hours, the culture medium was discarded and the cells in each well were lysed with 100 µL 1 × Passive Lysis Buffer (purchased from Promega). 70 µL of lysate was placed into an ELISA plate, 30 µL of substrate 1 solution was added, the ELISA plate was immediately put into the ELISA reader, and Luminescence was selected to read the value of each well of the ELISA plate as An. 30 µL of substrate 2 solution was added, the ELISA plate was immediately put into the ELISA reader, and Luminescence was selected to read the value of each well of the ELISA plate as Bn. The data was analyzed and processed with SoftMax Pro software. The ratio of An/Bn was the activation fold of TGFβ/Smad signal pathway.

The results are shown in FIG. 27. The results show that antibodies CT101 and CAT192 can effectively inhibit the activation of TGFβ/Smad signal pathway induced by TGF-β1 in a dose-dependent manner, at the same dose, the pharmacological activity of CT101 on the inhibition of TGFβ/Smad signal pathway induced by TGF-β1 is significantly superior to that of CAT192. The IC₅₀ of CT101 and CAT192 are 15 pM and 250 pM, respectively.

### Embodiment 15: In vivo experiment of tumor growth inhibition of CT101

In order to detect the *in vivo* antitumor activity of CT101, first MC38 colon cancer cells were subcutaneously inoculated to female human CD4 transgenic mice aged 6-8 weeks. Then, 50 µg of the drug was administered every 5 days by tail vein injection, and for 4 times in total. After drug administration, the length and width of tumor in each group were measured and the tumor volume was calculated.

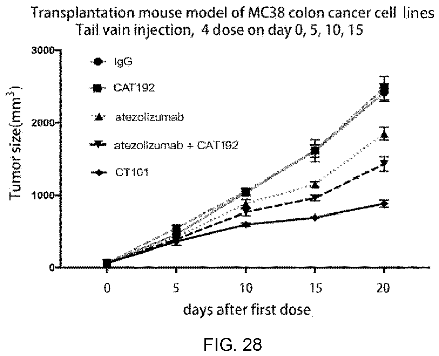

The results are shown in FIG. 28. The results show that compared with the isotype control antibodies IgG, CAT192, atezolizumab (PDL1 antibody), and combination of atezolizumab and CAT192, CT101 has stronger inhibition effect on mouse tumor growth.

The sequences involved in the present invention are as follows:
VH region of the first function region (SEQ ID NO: 1) 
QVQLQQSGPEVVKPGASVKMSCKASGYTFTSYVIHWVRQKPGQ
GLDWIGYINPYNDGTDYDEKFKGKATLTSDTSTSTAYMELSSLRSEDTAV
YYCAREKDNYATGAWFAYWGQGTLVTVSS
VL region of the first function region (SEQ ID NO: 2) 
DIVMTQSPDSLAVSLGERVTMNCKSSQSLLYSTNQKNYLAWYQQ
KPGQSPKLLIYWASTRESGVPDRFSGSGSGTDFTLTISSVQAEDVAVYY
CQQYYSYRTFGGGTKLEIK
Type 1 VH region of the second function region (SEQ ID NO: 3) 
EVQLVESGGGVVQPGRSLRLSCAASGFTFSSYGMHWVRQAPGK
ELEWVAVISYDGSIKYYADSVKGRFTISRDNSKNTLYLQMNSLRAEDTAV
YYCARTGEYSGYDTDPQYSWGQGTTVTVSS
Type 2 VH region of the second function region (SEQ ID NO: 4) 
EVQLVESGGGVVQPGRSLRLSCAASGFTFSSYGMHWVRQAPGK
ELEWVAVISYDGSIKYYADSVKGRFTISRDNSKNTLYLQMNSLRAEDTAV
YYCARTGEYSGYDTSGVELWGQGTTVTVSS
Type 3 VH region of the second function region (SEQ ID NO: 5) 
EVQLVESGGGVVQPGRSLRLSCAASGFTFSSYGMHWVRQAPGK
ELEWVAVISYDGSIKYYADSVKGRFTISRDNSKNTLYLQMNSLRAEDTAV
YYCARTGFYSGYDTPASPDWGQGTTVTVSS
VL region of the second function region (SEQ ID NO: 6) 
EIVLTQSPSSLSASVGDRVTITCRASQGIGDDLGWYQQKPGKAPI
LLIYGTSTLQSGVPSRFSGSGSGTDFTLTINSLQPEDFATYYCLQDSNYP
LTFGGGTRLEIK
Linker fragment (SEQ ID NO:7)
   GGGGSGGGGSGGGGS
Unit of linker fragment (SEQ ID NO:8)
   GGGGS
CL region of Human IgG4 κ light chain (SEQ ID NO: 9) 
GTVAAPSVFIFPPSDEQLKSGTASVVCLLNNFYPREAKVQWKVD
NALQSGNSQESVTEQDSKDSTYSLSSTLTLSKADYEKHKVYACEVTHQG
LSSPVTKSFNRGEC
CH1 region of Human IgG4 (SEQ ID NO:10) 
ASTKGPSVFPLAPCSRSTSESTAALGCLVKDYFPEPVTVSWNSG
ALTSGVHTFPAVLQSSGLYSLSSVVTVPSSSLGTKTYTCNVDHKPSNTK
VDKRVES
Hinge region (S228P) of Human IgG4 (SEQ ID NO: 11)
   KYGPPCPPCPAPEFLGGP
Fc region of Human IgG4 (SEQ ID NO: 12) 
SVFLFPPKPKDTLMISRTPEVTCVVVDVSQEDPEVQFNWYVDGV
EVHNAKTKPREEQFNSTYRVVSVLTVLHQDWLNGKEYKCKVSNKGLPS
SIEKTISKAKGQPREPQVYTLPPSQEEMTKNQVSLTCLVKGFYPSDIAVE
WESNGQPENNYKTTPPVLDSDGSFFLYSRLTVDKSRWQEGNVFSCSV
MHEALHNHYTQKSLSLSLGK
The first polypeptide chain of bifunctional antibody CT101 (SEQ ID NO: 13) 
DIVMTQSPDSLAVSLGERVTMNCKSSQSLLYSTNQKNYLAWYQQ
KPGQSPKLLIYWASTRESGVPDRFSGSGSGTDFTLTISSVQAEDVAVYY
CQQYYSYRTFGGGTKLEIKGTVAAPSVFIFPPSDEQLKSGTASVVCLLNN
FYPREAKVQWKVDNALQSGNSQESVTEQDSKDSTYSLSSTLTLSKADY
EKHKVYACEVTHQGLSSPVTKSFNRGEC
The second polypeptide chain of bifunctional antibody CT101 (SEQ ID NO: 14) 
QVQLQQSGPEVVKPGASVKMSCKASGYTFTSYVIHWVRQKPGQ
GLDWIGYINPYNDGTDYDEKFKGKATLTSDTSTSTAYMELSSLRSEDTAV
YYCAREKDNYATGAWFAYWGQGTLVTVSSASTKGPSVFPLAPCSRSTS
ESTAALGCLVKDYFPEPVTVSWNSGALTSGVHTFPAVLQSSGLYSLSSV
VTVPSSSLGTKTYTCNVDHKPSNTKVD
 
KRVESKYGPPCPPCPAPEFLGGPSVFLFPPKPKDTLMISRTPEVT
CVVVDVSQEDPEVQFNWYVDGVEVHNAKTKPREEQFNSTYRVVSVLTV
LHQDWLNGKEYKCKVSNKGLPSSIEKTISKAKGQPREPQVYTLPPSQEE
MTKNQVSLTCLVKGFYPSDIAVEWESNGQPENNYKTTPPVLDSDGSFFL
YSRLTVDKSRWQEGNVFSCSVMHEALHNHYTQKSLSLSLGKGGGGSG
 
GGGSGGGGSEVQLVESGGGVVQPGRSLRLSCAASGFTFSSYGMHWV
RQAPGKELEWVAVISYDGSIKYYADSVKGRFTISRDNSKNTLYLQMNSL
RAEDTAVYYCARTGEYSGYDTDPQYSWGQGTTVTVSSGGGGSGGGG
SGGGGSEIVLTQSPSSLSASVGDRVTITCRASQGIGDDLGWYQQKPGKA
PILLIYGTSTLQSGVPSRFSGSGSGTDFTLTINSLQPEDFATYYCLQDSNY
PLTFGGGTRLEIK
The first polypeptide chain of bifunctional antibody CT102 (SEQ ID NO: 15) 
DIVMTQSPDSLAVSLGERVTMNCKSSQSLLYSTNQKNYLAWYQQ
KPGQSPKLLIYWASTRESGVPDRFSGSGSGTDFTLTISSVQAEDVAVYY
CQQYYSYRTFGGGTKLEIKGTVAAPSVFIFPPSDEQLKSGTASVVCLLNN
FYPREAKVQWKVDNALQSGNSQESVTEQDSKDSTYSLSSTLTLSKADY
EKHKVYACEVTHQGLSSPVTKSFNRGECGGGGSGGGGSGGGGSEVQL
VESGGGVVQPGRSLRLSCAASGFTFSSYGMHWVRQAPGKELEWVAVI
SYDGSIKYYADSVKGRFTISRDNSKNTLYLQMNSLRAEDTAVYYCARTG
EYSGYDTDPQYSWGQGTTVTVSSGGGGSGGGGSGGGGSEIVLTQSPS
SLSASVGDRVTITCRASQGIGDDLGWYQQKPGKAPILLIYGTSTLQSGVP
SRFSGSGSGTDFTLTINSLQPEDFATYYCLQDSNYPLTFGGGTRLEIK
The second polypeptide chain of bifunctional antibody CT102 (SEQ ID NO: 16) 
QVQLQQSGPEVVKPGASVKMSCKASGYTFTSYVIHWVRQKPGQ
GLDWIGYINPYNDGTDYDEKFKGKATLTSDTSTSTAYMELSSLRSEDTAV
YYCAREKDNYATGAWFAYWGQGTLVTVSSASTKGPSVFPLAPCSRSTS
ESTAALGCLVKDYFPEPVTVSWNSGALTSGVHTFPAVLQSSGLYSLSSV
VTVPSSSLGTKTYTCNVDHKPSNTKVD
 
KRVESKYGPPCPPCPAPEFLGGPSVFLFPPKPKDTLMISRTPEVT
CVVVDVSQEDPEVQFNWYVDGVEVHNAKTKPREEQFNSTYRVVSVLTV
LHQDWLNGKEYKCKVSNKGLPSSIEKTISKAKGQPREPQVYTLPPSQEE
MTKNQVSLTCLVKGFYPSDIAVEWESNGQPENNYKTTPPVLDSDGSFFL
YSRLTVDKSRWQEGNVFSCSVMHEALHNHYTQKSLSLSLGK
The first polypeptide chain of bifunctional antibody CT103 (SEQ ID NO: 17) 
DIVMTQSPDSLAVSLGERVTMNCKSSQSLLYSTNQKNYLAWYQQ
KPGQSPKLLIYWASTRESGVPDRFSGSGSGTDFTLTISSVQAEDVAVYY
CQQYYSYRTFGGGTKLEIKGTVAAPSVFIFPPSDEQLKSGTASVVCLLNN
FYPREAKVQWKVDNALQSGNSQESVTEQDSKDSTYSLSSTLTLSKADY
EKHKVYACEVTHQGLSSPVTKSFNRGEC
The second polypeptide chain of bifunctional antibody CT103 (SEQ ID NO: 18) 
EVQLVESGGGVVQPGRSLRLSCAASGFTFSSYGMHWVRQAPGK
ELEWVAVISYDGSIKYYADSVKGRFTISRDNSKNTLYLQMNSLRAEDTAV
YYCARTGEYSGYDTDPQYSWGQGTTVTVSSGGGGSGGGGSGGGGSE
IVLTQSPSSLSASVGDRVTITCRASQGIGDDLGWYQQKPGKAPILLIYGTS
TLQSGVPSRFSGSGSGTDFTLTINSLQPEDFATYYCLQDSNYPLTFGGG
TRLEIKGGGGSGGGGSGGGGSQVQLQQSGPEVVKPGASVKMSCKASG
YTFTSYVIHWVRQKPGQGLDWIGYINPYNDGTDYDEKFKGKATLTSDTS
TSTAYMELSSLRSEDTAVYYCAREKDNYATGAWFAYWGQGTLVTVSSA
STKGPSVFPLAPCSRSTSESTAALGCLVKDYFPEPVTVSWNSGALTSGV
HTFPAVLQSSGLYSLSSVVTVPSSSLGTKTYTCNVDHKPSNTKVDKRVE
SKYGPPCPPCPAPEFLGGPSVFLFPPKPKDTLMISRTPEVTCVVVDVSQ
 
EDPEVQFNWYVDGVEVHNAKTKPREEQFNSTYRVVSVLTVLHQDWLN
GKEYKCKVSNKGLPSSIEKTISKAKGQPREPQVYTLPPSQEEMTKNQVS
LTCLVKGFYPSDIAVEWESNGQPENNYKTTPPVLDSDGSFFLYSRLTVD
KSRWQEGNVFSCSVMHEALHNHYTQKSLSLSLGK
The first polypeptide chain of bifunctional antibody CT104 (SEQ ID NO: 19) 
EVQLVESGGGVVQPGRSLRLSCAASGFTFSSYGMHWVRQAPGK
ELEWVAVISYDGSIKYYADSVKGRFTISRDNSKNTLYLQMNSLRAEDTAV
YYCARTGEYSGYDTDPQYSWGQGTTVTVSSGGGGSGGGGSGGGGSE
IVLTQSPSSLSASVGDRVTITCRASQGIGDDLGWYQQKPGKAPILLIYGTS
TLQSGVPSRFSGSGSGTDFTLTINSLQPEDFATYYCLQDSNYPLTFGGG
TRLEIKGGGGSGGGGSGGGGSDIVMTQSPDSLAVSLGERVTMNCKSSQ
SLLYSTNQKNYLAWYQQKPGQSPKLLIYWASTRESGVPDRFSGSGSGT
DFTLTISSVQAEDVAVYYCQQYYSYRTFGGGTKLEIKGTVAAPSVFIFPP
SDEQLKSGTASVVCLLNNFYPREAKVQWKVDNALQSGNSQESVTEQDS
KDSTYSLSSTLTLSKADYEKHKVYACEVTHQGLSSPVTKSFNRGEC
The second polypeptide chain of bifunctional antibody CT1 04 (SEQ ID NO: 20) 
QVQLQQSGPEVVKPGASVKMSCKASGYTFTSYVIHWVRQKPGQ
GLDWIGYINPYNDGTDYDEKFKGKATLTSDTSTSTAYMELSSLRSEDTAV
YYCAREKDNYATGAWFAYWGQGTLVTVSSASTKGPSVFPLAPCSRSTS
ESTAALGCLVKDYFPEPVTVSWNSGALTSGVHTFPAVLQSSGLYSLSSV
VTVPSSSLGTKTYTCNVDHKPSNTKVD
 
KRVESKYGPPCPPCPAPEFLGGPSVFLFPPKPKDTLMISRTPEVT
CVVVDVSQEDPEVQFNWYVDGVEVHNAKTKPREEQFNSTYRVVSVLTV
LHQDWLNGKEYKCKVSNKGLPSSIEKTISKAKGQPREPQVYTLPPSQEE
MTKNQVSLTCLVKGFYPSDIAVEWESNGQPENNYKTTPPVLDSDGSFFL
YSRLTVDKSRWQEGNVFSCSVMHEALHNHYTQKSLSLSLGK
The first polypeptide chain of bifunctional antibody CT105 (SEQ ID NO: 21) 
EIVLTQSPSSLSASVGDRVTITCRASQGIGDDLGWYQQKPGKAPI
LLIYGTSTLQSGVPSRFSGSGSGTDFTLTINSLQPEDFATYYCLQDSNYP
LTFGGGTRLEIKGGGGSDIVMTQSPDSLAVSLGERVTMNCKSSQSLLYS
TNQKNYLAWYQQKPGQSPKLLIYWASTRESGVPDRFSGSGSGTDFTLTI
SSVQAEDVAVYYCQQYYSYRTFGGGTKLEIKGTVAAPSVFIFPPSDEQL
KSGTASVVCLLNNFYPREAKVQWKVDNALQSGNSQESVTEQDSKDSTY
SLSSTLTLSKADYEKHKVYACEVTHQGLSSPVTKSFNRGEC
The second polypeptide chain of bifunctional antibody CT105 (SEQ ID NO: 22) 
EVQLVESGGGVVQPGRSLRLSCAASGFTFSSYGMHWVRQAPGK
ELEWVAVISYDGSIKYYADSVKGRFTISRDNSKNTLYLQMNSLRAEDTAV
YYCARTGEYSGYDTDPQYSWGQGTTVTVSSGGGGSQVQLQQSGPEV
VKPGASVKMSCKASGYTFTSYVIHWVRQKPGQGLDWIGYINPYNDGTD
YDEKFKGKATLTSDTSTSTAYMELSSLRSEDTAVYYCAREKDNYATGAW
FAYWGQGTLVTVSSASTKGPSVFPLAPCSRSTSESTAALGCLVKDYFPE
PVTVSWNSGALTSGVHTFPAVLQSSGLYSLSSVVTVPSSSLGTKTYTCN
VDHKPSNTKVDKRVESKYGPPCPPCPAPEFLGGPSVFLFPPKPKDTLMI
SRTPEVTCVVVDVSQEDPEVQFNWYVDGVEVHNAKTKPREEQFNSTYR
 
VVSVLTVLHQDWLNGKEYKCKVSNKGLPSSIEKTISKAKGQPREPQVYT
LPPSQEEMTKNQVSLTCLVKGFYPSDIAVEWESNGQPENNYKTTPPVLD
SDGSFFLYSRLTVDKSRWQEGNVFSCSVMHEALHNHYTQKSLSLSLGK
The first polypeptide chain of bifunctional antibody CT106 (SEQ ID NO: 23) 
DIVMTQSPDSLAVSLGERVTMNCKSSQSLLYSTNQKNYLAWYQQ
KPGQSPKLLIYWASTRESGVPDRFSGSGSGTDFTLTISSVQAEDVAVYY
CQQYYSYRTFGGGTKLEIKGTVAAPSVFIFPPSDEQLKSGTASVVCLLNN
FYPREAKVQWKVDNALQSGNSQESVTEQDSKDSTYSLSSTLTLSKADY
EKHKVYACEVTHQGLSSPVTKSFNRGEC
The second polypeptide chain of bifunctional antibody CT1 06 (SEQ ID NO: 24) 
QVQLQQSGPEVVKPGASVKMSCKASGYTFTSYVIHWVRQKPGQ
GLDWIGYINPYNDGTDYDEKFKGKATLTSDTSTSTAYMELSSLRSEDTAV
YYCAREKDNYATGAWFAYWGQGTLVTVSSASTKGPSVFPLAPCSRSTS
ESTAALGCLVKDYFPEPVTVSWNSGALTSGVHTFPAVLQSSGLYSLSSV
VTVPSSSLGTKTYTCNVDHKPSNTKVDKRVESKYGPPCPPCPAPEFLGG
PSVFLFPPKPKDTLMISRTPEVTCVVVDVSQEDPEVQFNWYVDGVEVHN
AKTKPREEQFNSTYRVVSVLTVLHQDWLNGKEYKCKVSNKGLPSSIEKTI
SKAKGQPREPQVYTLPPCQEEMTKNQVSLWCLVKGFYPSDIAVEWESN
GQPENNYKTTPPVLDSDGSFFLYSALTVDKSRWQEGNVFSCSVMHEAL
HNHYTQKSLSLSLGK
The third polypeptide chain of bifunctional antibody CT106 (SEQ ID NO: 25) 
EVQLVESGGGVVQPGRSLRLSCAASGFTFSSYGMHWVRQAPGK
ELEWVAVISYDGSIKYYADSVKGRFTISRDNSKNTLYLQMNSLRAEDTAV
YYCARTGEYSGYDTDPQYSWGQGTTVTVSGGSGTVAAPSVFIFPPSDE
QLKSGTASVVCLLNNFYPREAKVQWKVDNALQSGNSQESVTEQDSKDS
TYSLSSTLTLSKADYEKHKVYACEVTHQ
 
GLSSPVTKSFNRGECSGGGGSEVQLVESGGGVVQPGRSLRLSC
AASGFTFSSYGMHWVRQAPGKELEWVAVISYDGSIKYYADSVKGRFTIS
RDNSKNTLYLQMNSLRAEDTAVYYCARTGEYSGYDTDPQYSWGQGTT
VTVSGGSGTVAAPSVFIFPPSDEQLKSGTASVVCLLNNFYPREAKVQWK
VDNALQSGNSQESVTEQDSKDSTYSLSSTLTLSKADYEKHKVYACEVTH
QGLSSPVTKSFNRGECSKYGPPCPPCPAPEFLGGPSVFLFPPKPKDTLM
ISRTPEVTCVVVDVSQEDPEVQFNWYVDGVEVHNAKTKPREEQFNSTY
RVVSVLTVLHQDWLNGKEYKCKVSNKGLPSSIEKTISKAKGQPREPQVC
TLPPSQEEMTKNQVSLSCAVKGFYPSDIAVEWESNGQPENNYKTTPPVL
DSDGSFKLVSRLTVDKSRWQEGNVFSCSVMHEALHNHYTQKSLSLSLG
K
The fourth polypeptide chain of bifunctional antibody CT1 06 (SEQ ID NO: 26) 
EIVLTQSPSSLSASVGDRVTITCRSSQGIGDDLGWYQQKPGKAPI
LLIYGTSTLQSGVPSRFSGSGSGTDFTLTINSLQPEDFATYYCLQDSNYP
LTFGGGTRLEIKGSSASTKGPSVFPLAPCSRSTSESTAALGCLVKDYFPE
PVTVSWNSGALTSGVHTFPAVLQSSGLYSLSSVVTVPSSSLGTKTYTCN
VDHKPSNTKVDKRVES
The first polypeptide chain of bifunctional antibody CT107 (SEQ ID NO: 27) 
EIVLTQSPSSLSASVGDRVTITCRSSQGIGDDLGWYQQKPGKAPI
LLIYGTSTLQSGVPSRFSGSGSGTDFTLTINSLQPEDFATYYCLQDSNYP
LTFGGGTRLEIKGSSASTKGPSVFPLAPCSRSTSESTAALGCLVKDYFPE
PVTVSWNSGALTSGVHTFPAVLQSSGLYSLSSVVTVPSSSLGTKTYTCN
VDHKPSNTKVDKRVES
The second polypeptide chain of bifunctional antibody CT107 (SEQ ID NO: 28) 
EVQLVESGGGVVQPGRSLRLSCAASGFTFSSYGMHWVRQAPGK
ELEWVAVISYDGSIKYYADSVKGRFTISRDNSKNTLYLQMNSLRAEDTAV
YYCARTGEYSGYDTDPQYSWGQGTTVTVSGGSGTVAAPSVFIFPPSDE
QLKSGTASVVCLLNNFYPREAKVQWKVDNALQSGNSQESVTEQDSKDS
TYSLSSTLTLSKADYEKHKVYACEVTHQ
 
GLSSPVTKSFNRGECSKYGPPCPPCPAPEFLGGPSVFLFPPKPK
DTLMISRTPEVTCVVVDVSQEDPEVQFNWYVDGVEVHNAKTKPREEQF
NSTYRVVSVLTVLHQDWLNGKEYKCKVSNKGLPSSIEKTISKAKGQPRE
PQVYTLPPCQEEMTKNQVSLWCLVKGFYPSDIAVEWESNGQPENNYKT
TPPVLDSDGSFFLYSALTVDKSRWQEGNVFSCSVMHEALHNHYTQKSL
SLSLGK
The third polypeptide chain of bifunctional antibody CT107 (SEQ ID NO: 29) 
QVQLQQSGPEVVKPGASVKMSCKASGYTFTSYVIHWVRQKPGQ
GLDWIGYINPYNDGTDYDEKFKGKATLTSDTSTSTAYMELSSLRSEDTAV
YYCAREKDNYATGAWFAYWGQGTLVTVSSASTKGPSVFPLAPCSRSTS
 
ESTAALGCLVKDYFPEPVTVSWNSGALTSGVHTFPAVLQSSGLYSLSSV
VTVPSSSLGTKTYTCNVDHKPSNTKVD
 
KRVESGGGGSEVQLVESGGGVVQPGRSLRLSCAASGFTFSSYG
MHWVRQAPGKELEWVAVISYDGSIKYYADSVKGRFTISRDNSKNTLYLQ
MNSLRAEDTAVYYCARTGEYSGYDTDPQYSWGQGTTVTVSGGSGTVA
APSVFIFPPSDEQLKSGTASVVCLLNNFYPREAKVQWKVDNALQSGNSQ
ESVTEQDSKDSTYSLSSTLTLSKADYEKHKVYACEVTHQGLSSPVTKSF
NRGECSKYGPPCPPCPAPEFLGGPSVFLFPPKPKDTLMISRTPEVTCVV
VDVSQEDPEVQFNWYVDGVEVHNAKTKPREEQFNSTYRVVSVLTVLHQ
DWLNGKEYKCKVSNKGLPSSIEKTISKAKGQPREPQVCTLPPSQEEMTK
NQVSLSCAVKGFYPSDIAVEWESNGQPENNYKTTPPVLDSDGSFKLVSR
LTVDKSRWQEGNVFSCSVMHEALHNHYTQKSLSLSLGK
The fourth polypeptide chain of bifunctional antibody CT1 07 (SEQ ID NO: 30) 
DIVMTQSPDSLAVSLGERVTMNCKSSQSLLYSTNQKNYLAWYQQ
KPGQSPKLLIYWASTRESGVPDRFSGSGSGTDFTLTISSVQAEDVAVYY
CQQYYSYRTFGGGTKLEIKGTVAAPSVFIFPPSDEQLKSGTASVVCLLNN
FYPREAKVQWKVDNALQSGNSQESVTEQDSKDSTYSLSSTLTLSKADY
EKHKVYACEVTHQGLSSPVTKSFNRGEC
The first polypeptide chain of bifunctional antibody CT108 (SEQ ID NO: 31) 
DIVMTQSPDSLAVSLGERVTMNCKSSQSLLYSTNQKNYLAWYQQ
KPGQSPKLLIYWASTRESGVPDRFSGSGSGTDFTLTISSVQAEDVAVYY
CQQYYSYRTFGGGTKLEIKGTVAAPSVFIFPPSDEQLKSGTASVVCLLNN
 
FYPREAKVQWKVDNALQSGNSQESVTEQDSKDSTYSLSSTLTLSKADY
EKHKVYACEVTHQGLSSPVTKSFNRGEC
The second polypeptide chain of bifunctional antibody CT1 08 (SEQ ID NO: 32) 
EIVLTQSPSSLSASVGDRVTITCRSSQGIGDDLGWYQQKPGKAPI
LLIYGTSTLQSGVPSRFSGSGSGTDFTLTINSLQPEDFATYYCLQDSNYP
LTFGGGTRLEIKGSSASTKGPSVFPLAPCSRSTSESTAALGCLVKDYFPE
PVTVSWNSGALTSGVHTFPAVLQSSGLYSLSSVVTVPSSSLGTKTYTCN
VDHKPSNTKVDKRVES
The third polypeptide chain of bifunctional antibody CT108 (SEQ ID NO: 33) 
QVQLQQSGPEVVKPGASVKMSCKASGYTFTSYVIHWVRQKPGQ
GLDWIGYINPYNDGTDYDEKFKGKATLTSDTSTSTAYMELSSLRSEDTAV
YYCAREKDNYATGAWFAYWGQGTLVTVSSASTKGPSVFPLAPCSRSTS
ESTAALGCLVKDYFPEPVTVSWNSGALTSGVHTFPAVLQSSGLYSLSSV
VTVPSSSLGTKTYTCNVDHKPSNTKVD
 
KRVESGGGGSEVQLVESGGGVVQPGRSLRLSCAASGFTFSSYG
MHWVRQAPGKELEWVAVISYDGSIKYYADSVKGRFTISRDNSKNTLYLQ
MNSLRAEDTAVYYCARTGEYSGYDTDPQYSWGQGTTVTVSGGSGTVA
APSVFIFPPSDEQLKSGTASVVCLLNNFYPREAKVQWKVDNALQSGNSQ
ESVTEQDSKDSTYSLSSTLTLSKADYEKHKVYACEVTHQGLSSPVTKSF
NRGECSKYGPPCPPCPAPEFLGGPSVFLFPPKPKDTLMISRTPEVTCVV
VDVSQEDPEVQFNWYVDGVEVHNAKTKPREEQFNSTYRVVSVLTVLHQ
DWLNGKEYKCKVSNKGLPSSIEKTISKAKGQPREPQVYTLPPSQEEMTK
 
NQVSLTCLVKGFYPSDIAVEWESNGQPENNYKTTPPVLDSDGSFFLYSR
LTVDKSRWQEGNVFSCSVMHEALHNHYTQKSLSLSLGK
The first polypeptide chain of bifunctional antibody CT109 (SEQ ID NO: 34) 
DIVMTQSPDSLAVSLGERVTMNCKSSQSLLYSTNQKNYLAWYQQ
KPGQSPKLLIYWASTRESGVPDRFSGSGSGTDFTLTISSVQAEDVAVYY
CQQYYSYRTFGGGTKLEIKGTVAAPSVFIFPPSDEQLKSGTASVVCLLNN
FYPREAKVQWKVDNALQSGNSQESVTEQDSKDSTYSLSSTLTLSKADY
EKHKVYACEVTHQGLSSPVTKSFNRGEC
The second polypeptide chain of bifunctional antibody CT1 09 (SEQ ID NO: 35) 
EIVLTQSPSSLSASVGDRVTITCRSSQGIGDDLGWYQQKPGKAPI
LLIYGTSTLQSGVPSRFSGSGSGTDFTLTINSLQPEDFATYYCLQDSNYP
LTFGGGTRLEIKGSSASTKGPSVFPLAPCSRSTSESTAALGCLVKDYFPE
PVTVSWNSGALTSGVHTFPAVLQSSGLYSLSSVVTVPSSSLGTKTYTCN
VDHKPSNTKVDKRVES
The third polypeptide chain of bifunctional antibody CT109 (SEQ ID NO: 36) 
QVQLQQSGPEVVKPGASVKMSCKASGYTFTSYVIHWVRQKPGQ
GLDWIGYINPYNDGTDYDEKFKGKATLTSDTSTSTAYMELSSLRSEDTAV
YYCAREKDNYATGAWFAYWGQGTLVTVSSASTKGPSVFPLAPCSRSTS
ESTAALGCLVKDYFPEPVTVSWNSGALTSGVHTFPAVLQSSGLYSLSSV
VTVPSSSLGTKTYTCNVDHKPSNTKVD
 
KRVESKYGPPCPPCPAPEFLGGPSVFLFPPKPKDTLMISRTPEVT
CVVVDVSQEDPEVQFNWYVDGVEVHNAKTKPREEQFNSTYRVVSVLTV
LHQDWLNGKEYKCKVSNKGLPSSIEKTISKAKGQPREPQVYTLPPSQEE
MTKNQVSLTCLVKGFYPSDIAVEWESNGQPENNYKTTPPVLDSDGSFFL
YSRLTVDKSRWQEGNVFSCSVMHEALHNHYTQKSLSLSLGKGGGGSG
GGGSGGGGSEVQLVESGGGVVQPGRSLRLSCAASGFTFSSYGMHWV
RQAPGKELEWVAVISYDGSIKYYADSVKGRFTISRDNSKNTLYLQMNSL
RAEDTAVYYCARTGEYSGYDTDPQYSWGQGTTVTVSGGSGTVAAPSV
FIFPPSDEQLKSGTASVVCLLNNFYPREAKVQWKVDNALQSGNSQESVT
EQDSKDSTYSLSSTLTLSKADYEKHKVYACEVTHQGLSSPVTKSFNRGE
C
The first polypeptide chain of bifunctional antibody CT110 (SEQ ID NO: 37) 
DIVMTQSPDSLAVSLGERVTMNCKSSQSLLYSTNQKNYLAWYQQ
KPGQSPKLLIYWASTRESGVPDRFSGSGSGTDFTLTISSVQAEDVAVYY
CQQYYSYRTFGGGTKLEIKGTVAAPSVFIFPPSDEQLKSGTASVVCLLNN
FYPREAKVQWKVDNALQSGNSQESVTEQDSKDSTYSLSSTLTLSKADY
EKHKVYACEVTHQGLSSPVTKSFNRGEC
The second polypeptide chain of bifunctional antibody CT110 (SEQ ID NO: 38) 
QVQLQQSGPEVVKPGASVKMSCKASGYTFTSYVIHWVRQKPGQ
GLDWIGYINPYNDGTDYDEKFKGKATLTSDTSTSTAYMELSSLRSEDTAV
YYCAREKDNYATGAWFAYWGQGTLVTVSSASTKGPSVFPLAPCSRSTS
ESTAALGCLVKDYFPEPVTVSWNSGALTSGVHTFPAVLQSSGLYSLSSV
VTVPSSSLGTKTYTCNVDHKPSNTKVDKRVESKYGPPCPPCPAPEFLGG
 
PSVFLFPPKPKDTLMISRTPEVTCVVVDVSQEDPEVQFNWYVDGVEVHN
AKTKPREEQFNSTYRVVSVLTVLHQDWLNGKEYKCKVSNKGLPSSIEKTI
SKAKGQPREPQVYTLPPCQEEMTKNQVSLWCLVKGFYPSDIAVEWESN
GQPENNYKTTPPVLDSDGSFFLYSALTVDKSRWQEGNVFSCSVMHEAL
HNHYTQKSLSLSLGKGGGGSEVQLVESGGGVVQPGRSLRLSCAASGFT
FSSYGMHWVRQAPGKELEWVAVISYDGSIKYYADSVKGRFTISRDNSKN
TLYLQMNSLRAEDTAVYYCARTGEYSGYDTDPQYSWGQGTTVTVSS
The third polypeptide chain of bifunctional antibody CT110 (SEQ ID NO: 39) 
QVQLQQSGPEVVKPGASVKMSCKASGYTFTSYVIHWVRQKPGQ
GLDWIGYINPYNDGTDYDEKFKGKATLTSDTSTSTAYMELSSLRSEDTAV
YYCAREKDNYATGAWFAYWGQGTLVTVSSASTKGPSVFPLAPCSRSTS
ESTAALGCLVKDYFPEPVTVSWNSGALTSGVHTFPAVLQSSGLYSLSSV
VTVPSSSLGTKTYTCNVDHKPSNTKVDKRVESKYGPPCPPCPAPEFLGG
PSVFLFPPKPKDTLMISRTPEVTCVVVDVSQEDPEVQFNWYVDGVEVHN
AKTKPREEQFNSTYRVVSVLTVLHQDWLNGKEYKCKVSNKGLPSSIEKTI
SKAKGQPREPQVCTLPPSQEEMTKNQVSLSCAVKGFYPSDIAVEWESN
GQPENNYKTTPPVLDSDGSFKLVSRLTVDKSRWQEGNVFSCSVMHEAL
HNHYTQKSLSLSLGKGGGGSEIVLTQSPSSLSASVGDRVTITCRASQGIG
DDLGWYQQKPGKAPILLIYGTSTLQSGVPSRFSGSGSGTDFTLTINSLQP
EDFATYYCLQDSNYPLTFGGGTRLEIK
The first polypeptide chain of bifunctional antibody CT111 (SEQ ID NO: 40) 
DIVMTQSPDSLAVSLGERVTMNCKSSQSLLYSTNQKNYLAWYQQ
KPGQSPKLLIYWASTRESGVPDRFSGSGSGTDFTLTISSVQAEDVAVYY
 
CQQYYSYRTFGGGTKLEIKGTVAAPSVFIFPPSDEQLKSGTASVVCLLNN
FYPREAKVQWKVDNALQSGNSQESVTEQDSKDSTYSLSSTLTLSKADY
EKHKVYACEVTHQGLSSPVTKSFNRGEC
The second polypeptide chain of bifunctional antibody CT111 (SEQ ID NO: 41) 
QVQLQQSGPEVVKPGASVKMSCKASGYTFTSYVIHWVRQKPGQ
GLDWIGYINPYNDGTDYDEKFKGKATLTSDTSTSTAYMELSSLRSEDTAV
YYCAREKDNYATGAWFAYWGQGTLVTVSSASTKGPSVFPLAPCSRSTS
ESTAALGCLVKDYFPEPVTVSWNSGALTSGVHTFPAVLQSSGLYSLSSV
VTVPSSSLGTKTYTCNVDHKPSNTKVD
 
KRVESKYGPPCPPCPAPEFLGGPSVFLFPPKPKDTLMISRTPEVT
CVVVDVSQEDPEVQFNWYVDGVEVHNAKTKPREEQFNSTYRVVSVLTV
LHQDWLNGKEYKCKVSNKGLPSSIEKTISKAKGQPREPQVYTLPPCQEE
MTKNQVSLWCLVKGFYPSDIAVEWESNGQPENNYKTTPPVLDSDGSFF
LYSALTVDKSRWQEGNVFSCSVMHEALHNHYTQKSLSLSLGK
The third polypeptide chain of bifunctional antibody CT111 (SEQ ID NO: 42) 
EVQLVESGGGVVQPGRSLRLSCAASGFTFSSYGMHWVRQAPGK
ELEWVAVISYDGSIKYYADSVKGRFTISRDNSKNTLYLQMNSLRAEDTAV
YYCARTGEYSGYDTDPQYSWGQGTTVTVSSGGGGSGGGGSGGGGSE
IVLTQSPSSLSASVGDRVTITCRASQGIGDDLGWYQQKPGKAPILLIYGTS
TLQSGVPSRFSGSGSGTDFTLTINSLQPEDFATYYCLQDSNYPLTFGGG
TRLEIKYGPPCPPCPAPEFLGGPSVFLFPPKPKDTLMISRTPEVTCVVVD
VSQEDPEVQFNWYVDGVEVHNAKTKPREEQFNSTYRVVSVLTVLHQD
 
WLNGKEYKCKVSNKGLPSSIEKTISKAKGQPREPQVCTLPPSQEEMTKN
QVSLSCAVKGFYPSDIAVEWESNGQPENNYKTTPPVLDSDGSFKLVSRL
TVDKSRWQEGNVFSCSVMHEALHNHYTQKSLSLSLGK
The first polypeptide chain of bifunctional antibody CT112 (SEQ ID NO: 43) 
DIVMTQSPDSLAVSLGERVTMNCKSSQSLLYSTNQKNYLAWYQQ
KPGQSPKLLIYWASTRESGVPDRFSGSGSGTDFTLTISSVQAEDVAVYY
CQQYYSYRTFGGGTKLEIKGTVAAPSVFIFPPSDEQLKSGTASVVCLLNN
FYPREAKVQWKVDNALQSGNSQESVTEQDSKDSTYSLSSTLTLSKADY
EKHKVYACEVTHQGLSSPVTKSFNRGEC
The second polypeptide chain of bifunctional antibody CT112 (SEQ ID NO: 44) 
QVQLQQSGPEVVKPGASVKMSCKASGYTFTSYVIHWVRQKPGQ
GLDWIGYINPYNDGTDYDEKFKGKATLTSDTSTSTAYMELSSLRSEDTAV
YYCAREKDNYATGAWFAYWGQGTLVTVSSASTKGPSVFPLAPCSRSTS
ESTAALGCLVKDYFPEPVTVSWNSGALTSGVHTFPAVLQSSGLYSLSSV
VTVPSSSLGTKTYTCNVDHKPSNTKVD
 
KRVESKYGPPCPPCPAPEFLGGPSVFLFPPKPKDTLMISRTPEVT
CVVVDVSQEDPEVQFNWYVDGVEVHNAKTKPREEQFNSTYRVVSVLTV
LHQDWLNGKEYKCKVSNKGLPSSIEKTISKAKGQPREPQVYTLPPCQEE
MTKNQVSLWCLVKGFYPSDIAVEWESNGQPENNYKTTPPVLDSDGSFF
LYSALTVDKSRWQEGNVFSCSVMHEALHNHYTQKSLSLSLGK
The third polypeptide chain of bifunctional antibody CT112 (SEQ ID NO: 45) 
EVQLVESGGGVVQPGRSLRLSCAASGFTFSSYGMHWVRQAPGK
ELEWVAVISYDGSIKYYADSVKGRFTISRDNSKNTLYLQMNSLRAEDTAV
YYCARTGEYSGYDTDPQYSWGQGTTVTVSGGSGTVAAPSVFIFPPSDE
QLKSGTASVVCLLNNFYPREAKVQWKVDNALQSGNSQESVTEQDSKDS
TYSLSSTLTLSKADYEKHKVYACEVTHQ
 
GLSSPVTKSFNRGECSKYGPPCPPCPAPEFLGGPSVFLFPPKPK
DTLMISRTPEVTCVVVDVSQEDPEVQFNWYVDGVEVHNAKTKPREEQF
NSTYRVVSVLTVLHQDWLNGKEYKCKVSNKGLPSSIEKTISKAKGQPRE
PQVCTLPPSQEEMTKNQVSLSCAVKGFYPSDIAVEWESNGQPENNYKT
TPPVLDSDGSFKLVSRLTVDKSRWQEGNVFSCSVMHEALHNHYTQKSL
SLSLGK
The fourth polypeptide chain of bifunctional antibody CT112 (SEQ ID NO: 46) 
EIVLTQSPSSLSASVGDRVTITCRSSQGIGDDLGWYQQKPGKAPI
LLIYGTSTLQSGVPSRFSGSGSGTDFTLTINSLQPEDFATYYCLQDSNYP
LTFGGGTRLEIKGSSASTKGPSVFPLAPCSRSTSESTAALGCLVKDYFPE
PVTVSWNSGALTSGVHTFPAVLQSSGLYSLSSVVTVPSSSLGTKTYTCN
VDHKPSNTKVDKRVES
Polypeptide chain of TGF-β1 antibody (ScFv-IgG4) (SEQ ID NO: 47) 
EVQLVESGGGVVQPGRSLRLSCAASGFTFSSYGMHWVRQAPGK
ELEWVAVISYDGSIKYYADSVKGRFTISRDNSKNTLYLQMNSLRAEDTAV
YYCARTGEYSGYDTDPQYSWGQGTTVTVSSGGGGSGGGGSGGGGSE
IVLTQSPSSLSASVGDRVTITCRASQGIGDDLGWYQQKPGKAPILLIYGTS
TLQSGVPSRFSGSGSGTDFTLTINSLQPEDFATYYCLQDSNYPLTFGGG
 
TRLEIKSGSKYGPPCPPCPAPEFLGGPSVFLFPPKPKDTLMISRTPEVTC
VVVDVSQEDPEVQFNWYVDGVEVHNAKTKPREEQFNSTYRVVSVLTVL
HQDWLNGKEYKCKVSNKGLPSSIEKTISKAKGQPREPQVYTLPPSQEEM
TKNQVSLTCLVKGFYPSDIAVEWESNGQPENNYKTTPPVLDSDGSFFLY
SRLTVDK
SRWQEGNVFSCSVMHEALHNHYTQKSLSLSLGK
Polypeptide chain of control antibody (ScFv-IgG4) (SEQ ID NO: 48) 
EVQLVESGGGLVQPGGSLRLSCAASGYTFTNYGMNWVRQAPGK
GLEWVGWINTYTGEPTYAADFKRRFTFSLDTSKSTAYLQMNSLRAEDTA
VYYCAKYPHYYGSSHWYFDVWGQGTLVTVSSGGGGSGGGGSGGGGS
DIQMTQSPSSLSASVGDRVTITCSASQDISNYLNWYQQKPGKAPKVLIYF
TSSLHSGVPSRFSGSGSGTDFTLTISSLQ
 
PEDFATYYCQQYSTVPWTFGQGTKVEIKSGSKYGPPCPPCPAPE
FLGGPSVFLFPPKPKDTLMISRTPEVTCVVVDVSQEDPEVQFNWYVDGV
EVHNAKTKPREEQFNSTYRVVSVLTVLHQDWLNGKEYKCKVSNKGLPS
SIEKTISKAKGQPREPQVYTLPPSQEEMTKNQVSLTCLVKGFYPSDIAVE
WESNGQPENNYKTTPPVLDSDGSFFLYSRLTVDKSRWQEGNVFSCSV
MHEALHNHYTQKSLSLSLGK
Recombinant CD4-His protein sequence (SEQ ID NO: 49) 
KKVVLGKKGDTVELTCTASQKKSIQFHWKNSNQIKILGNQGSFLT
KGPSKLNDRADSRRSLWDQGNFPLIIKNLKIEDSDTYICEVEDQKEEVQL
LVFGLTANSDTHLLQGQSLTLTLESPPGSSPSVQCRSPRGKNIQGGKTL
SVSQLELQDSGTWTCTVLQNQKKVEFKIDIVVLAFQKASSIVYKKEGEQV
EFSFPLAFTVEKLTGSGELWWQAERASSSKSWITFDLKNKEVSVKRVTQ
 
DPKLQMGKKLPLHLTLPQALPQYAGSGNLTLALEAKTGKLHQEVNLVVM
RATQLQKNLTCEVWGPTSPKLMLSLKLENKEAKVSKREKAVWVLNPEA
GMWQCLLSDSGQVLLESNIKVLPTWHHHHHH
Nucleotide sequence encoding recombinant CD4-His protein (SEQ ID NO: 50) 
AAGAAAGTGGTGCTGGGCAAAAAAGGGGATACAGTGGAACTG
ACCTGTACAGCTTCCCAGAAGAAGAGCATACAATTCCACTGGAAAAA
CTCCAACCAGATAAAGATTCTGGGAAATCAGGGCTCCTTCTTAACTAA
AGGTCCATCCAAGCTGAATGATCGCGCTGACTCAAGAAGAAGCCTTT
GGGACCAAGGAAACTTTCCCCTGATCATCAAGAATCTTAAGATAGAA
GACTCAGATACTTACATCTGTGAAGTGGAGGACCAGAAGGAGGAGGT
GCAATTGCTAGTGTTCGGATTGACTGCCAACTCTGACACCCACCTGC
TTCAGGGGCAGAGCCTGACCCTGACCTTGGAGAGCCCCCCTGGTAG
TAGCCCCTCAGTGCAATGTAGGAGTCCAAGGGGTAAAAACATACAGG
GGGGGAAGACCCTCTCCGTGTCTCAGCTGGAGCTCCAGGATAGTGG
CACCTGGACATGCACTGTCTTGCAGAACCAGAAGAAGGTGGAGTTCA
AAATAGACATCGTGGTGCTAGCTTTCCAGAAGGCCTCCAGCATAGTC
TATAAGAAAGAGGGGGAACAGGTGGAGTTCTCCTTCCCACTCGCCTT
TACAGTTGAAAAGCTGACGGGCAGTGGCGAGCTGTGGTGGCAGGCG
GAGAGGGCTTCCTCCTCCAAGTCTTGGATCACCTTTGACCTGAAGAA
CAAGGAAGTGTCTGTAAAACGGGTTACCCAGGACCCTAAGCTCCAGA
TGGGCAAGAAGCTCCCGCTCCACCTCACCCTGCCCCAGGCCTTGCC
TCAGTATGCTGGCTCTGGAAACCTCACCCTGGCCCTTGAAGCGAAAA
CAGGAAAGTTGCATCAGGAAGTGAACCTGGTGGTGATGAGAGCCACT
CAGCTCCAGAAAAATTTGACCTGTGAGGTGTGGGGACCCACCTCCCC
TAAGCTGATGCTGAGTTTGAAACTGGAGAACAAGGAGGCAAAGGTCT
 
CGAAGCGGGAGAAGGCGGTGTGGGTGCTGAACCCTGAGGCGGGGA
TGTGGCAGTGTCTGCTGAGTGACTCGGGACAGGTCCTGCTGGAATC
CAACATCAAGGTTCTGCCCACATGGCATCATCATCATCATCAT
TGF-β1-His protein sequence (SEQ ID NO: 51) 
ALDTNYCFSSTEKNCCVRQLYIDFRKDLGWKWIHEPKGYHANFC
LGPCPYIWSLDTQYSKVLALYNQHNPGASAAPCCVPQALEPLPIVYYVG
RKPKVEQLSNMIVRSCKCSHHHHHH
Nucleotide sequence encoding the recombinant TGF-β1-His protein (SEQ ID NO: 52) 
GCCCTGGATACCAACTATTGCTTCAGCTCCACAGAGAAGAACT
GCTGTGTGCGGCAGCTGTACATTGACTTTAGGAAGGACCTGGGTTGG
AAGTGGATCCACGAGCCCAAGGGCTACCATGCCAACTTCTGCCTCG
GGCCCTGCCCCTACATTTGGAGCCTGGACACGCAGTACAGCAAGGT
CCTGGCCCTGTACAACCAGCATAACCCGGGCGCCTCGGCGGCGCCG
TGCTGCGTGCCGCAGGCGCTGGAGCCGCTGCCCATCGTGTACTACG
TGGGCCGCAAGCCCAAGGTGGAGCAGCTGTCCAACATGATCGTGCG
CTCCTGCAAGTGCAGCCATCATCATCATCATCAT
The first function region HCDR1: (SEQ ID NO:53)
   GYTFTSYVIH
The first function region HCDR2: (SEQ ID NO:54)
   YINPYNDGTDYDEKFKG
The first function region HCDR3: (SEQ ID NO:55) EKDNYATGAWFA
The first function region LCDR1: (SEQ ID NO:56)
   KSSQSLLYSTNQKNYLA
The first function region LCDR2:WASTRES(SEQ ID NO: 57)
   WASTRES
The first function region LCDR3: (SEQ ID NO:58)
   QQYYSYRT
The second function region HCDR1: (SEQ ID NO:59)
   SYGMH
The second function region HCDR2: (SEQ ID NO:60)
   VISYDGSIKYYADSVKG
The second function region HCDR3 (type 1): (SEQ ID NO: 61)
   TGEYSGYDTDPQYS
The second function region HCDR3 (type 2): (SEQ ID NO: 62)
   TGEYSGYDTSGVEL
The second function region HCDR3 (type 3): (SEQ ID NO: 63)
   TGFYSGYDTPASPD
Consensus sequence of the second function region HCDR3: (SEQ ID NO:64)
   TGX1YSGYDTX2X3X4X5X6
The second function region LCDR1: (SEQ ID NO: 65)
   RSSQGIGDDLG
The second function region LCDR2: (SEQ ID NO: 66)
   GTSTLQS
The second function region LCDR3: (SEQ ID NO:67)
   LQDSNYPLT
Amino acid sequence encoding TGF-β1 antibody: (475aa) 
EVQLVESGGGVVQPGRSLRLSCAASGFTFSSYGMHWVRQAPGK
ELEWVAVISYDGSIKYYADSVKGRFTISRDNSKNTLYLQMNSLRAEDTAV
YYCARTGEYSGYDTDPQYSWGQGTTVTVSSGGGGSGGGGSGGGGSE
IVLTQSPSSLSASVGDRVTITCRASQGIGDDLGWYQQKPGKAPILLIYGTS
TLQSGVPSRFSGSGSGTDFTLTINSLQPEDFATYYCLQDSNYPLTFGGG
TRLEIKSGSKYGPPCPPCPAPEFLGGPSVFLFPPKPKDTLMISRTPEVTC
VVVDVSQEDPEVQFNWYVDGVEVHNAKTKPREEQFNSTYRVVSVLTVL
HQDWLNGKEYKCKVSNKGLPSSIEKTISKAKGQPREPQVYTLPPSQEEM
TKNQVSLTCLVKGFYPSDIAVEWESNGQPENNYKTTPPVLDSDGSFFLY
SRLTVDKSRWQEGNVFSCSVMHEALHNHYTQKSLSLSLGK（SEQ ID
NO:68）
Amino acid sequence encoding control antibody: (475aa)

EVQLVESGGGLVQPGGSLRLSCAASGYTFTNYGMNWVRQAPGK
GLEWVGWINTYTGEPTYAADFKRRFTFSLDTSKSTAYLQMNSLRAEDTA
VYYCAKYPHYYGSSHWYFDVWGQGTLVTVSSGGGGSGGGGSGGGGS
DIQMTQSPSSLSASVGDRVTITCSASQDISNYLNWYQQKPGKAPKVLIYF
TSSLHSGVPSRFSGSGSGTDFTLTISSLQPEDFATYYCQQYSTVPWTFG
QGTKVEIKSGSKYGPPCPPCPAPEFLGGPSVFLFPPKPKDTLMISRTPEV
TCVVVDVSQEDPEVQFNWYVDGVEVHNAKTKPREEQFNSTYRVVSVLT
VLHQDWLNGKEYKCKVSNKGLPSSIEKTISKAKGQPREPQVYTLPPSQE
EMTKNQVSLTCLVKGFYPSDIAVEWESNGQPENNYKTTPPVLDSDGSFF
LYSRLTVDKSRWQEGNVFSCSVMHEALHNHYTQKSLSLSLGK（SEQ ID
NO:69）

Although the contents presented in the present invention provide a complete and clear description of the specific embodiments disclosed therein, the present invention is not limited thereto. It is possible for those skilled in the art to make modifications and alternatives to the present invention with the guidance of these descriptions, and such modifications and alternatives are included within the scope of the present invention. The full scope of the present invention is provided by the appended claims and any equivalents thereof.

## Claims

1. A bispecific antibody, comprising at least a first polypeptide chain and a second polypeptide chain,
wherein the first polypeptide chain comprises partial or complete structure of a first function region, and/or, partial or complete structure of a second function region;
wherein the first function region targets human protein cluster of differentiation 4 (CD4), and is an anti-CD4 antibody or an antigen-binding fragment thereof;
wherein the second function region targets human protein transformation growth factor-β1 (TGF-β1), and is an anti-TGF-β1 antibody or an antigen-binding fragment thereof.

2. The bispecific antibody of claim 1, wherein a heavy chain variable region (VH) of the first function region comprises HCDR1, HCDR2, and HCDR3 having an amino acid sequence of SEQ ID NO:53, SEQ ID NO:54, and SEQ ID NO:55, respectively, and a light chain variable region (VL) of the first function region comprises LCDR1, LCDR2, and LCDR3 having an amino acid sequence of SEQ ID NO:56, SEQ ID NO:57, and SEQ ID NO:58, respectively;
a heavy chain variable region (VH) of the second function region comprises HCDR1 having an amino acid sequence of SEQ ID NO:59, HCDR2 having an amino acid sequence of SEQ ID NO:60, and HCDR3 having an amino acid sequence of any one of SEQ ID NO:61-SEQ ID NO:63, and a light chain variable region (VL) comprises LCDR1, LCDR2, and LCDR3 having amino acid sequence of SEQ ID NO:65, SEQ ID NO:66, and SEQ ID NO:67, respectively.

3. The bispecific antibody of claim 1, wherein the anti-CD4 antibody or antigen-binding fragment thereof is selected from a group consisting of Fab, Fab', F(ab')2, Fd, Fv, dAb, complementarity determining region (CDR), single chain variable fragment (ScFv), humanized antibody, chimeric antibody, and diabody;
and/or, the anti-TGF-β1 antibody or antigen-binding fragment thereof is selected from a group consisting of Fab, Fab', F(ab')2, Fd, Fv, dAb, CDR, ScFv, humanized antibody, chimeric antibody, and diabody.

4. The bispecific antibody of any one of claims 1-3, wherein the first function region is an immunoglobulin comprising a VH and a VL, wherein the VH comprises an amino acid sequence of SEQ ID NO:1, and the VL comprises an amino acid sequence of SEQ ID NO:2;
the second function region is an ScFv or Fab comprising a VH and a VL, wherein the VH comprises an amino acid sequence of any one of SEQ ID NO:3, SEQ ID NO:4, and SEQ ID NO: 5, and the VL comprises an amino acid sequence of SEQ ID NO:6.

5. The bispecific antibody of any one of claims 1-3, wherein the first function region is an immunoglobulin comprising a VH and a VL, wherein the VH comprises an amino acid sequence of SEQ ID NO:76, and the VL comprises an amino acid sequence of SEQ ID NO:78;
and the second function domain is an ScFv or Fab comprising a VH and a VL, wherein the VH comprises an amino acid sequence of SEQ ID NO:80, and the VL comprises an amino acid sequence of SEQ ID NO:82.

6. The bispecific antibody of any one of claims 1-5, wherein the bispecific antibody is in an IgG-ScFv/Fab format or other bispecific antibody formats.

7. The bispecific antibody of any one of claims 1-6, wherein the number of the first function region and second function region is independently 1, 2, or more.

8. The bispecific antibody of any one of claims 1-7, wherein the first and second function regions are directly connected or linked by a peptide linker;
preferably, the peptide linker is (GGGGS)m, wherein m is an integer, such as 1, 2, 3, 4, 5, or 6.

9. The bispecific antibody of any one of claims 1-8, wherein a constant region of the immunoglobin is from human antibody;
preferably, the constant region of the immunoglobin is selected from a group consisting of IgG1, IgG2, IgG3, and IgG4.

10. The bispecific antibody of any one of claims 1-9, wherein the first polypeptide chain, from N-terminal to C-terminal, has a formula of:
light chain variable region-light chain constant region-(peptide linker)m-heavy chain variable region-(peptide linker)n-light chain variable region;
the second polypeptide chain, from N-terminal to C-terminal, has a formula of:
heavy chain variable region-heavy chain constant region-Hinge-Fc region;
preferably, the peptide linker is GGGGS, wherein m is 0 or a positive integer, and n is 0 or a positive integer.

11. The bispecific antibody of claim 10, wherein the light chain variable region-light chain constant region in the first polypeptide chain interacts with the heavy chain variable region-heavy chain constant region in the second polypeptide chain to form a polypeptide chain group, wherein two such polypeptide chain groups form a homodimer.

12. The bispecific antibody of any one of claims 10-11, wherein the VL at N-terminal of the first polypeptide chain comprises an amino acid sequence of SEQ ID NO:2, and the VH of the first polypeptide chain comprises an amino acid sequence of any one of SEQ ID NO:3, SEQ ID NO:4, and SEQ ID NO:5; and the VL at C-terminal of the first polypeptide chain comprises an amino acid sequence of SEQ ID NO:6, and the VH of the second polypeptide chain comprises an amino acid sequence of SEQ ID NO:1.

13. The bispecific antibody of any one of claims 10-12, wherein the first polypeptide chain comprises an amino acid sequence of SEQ ID NO:15; and the second polypeptide chain comprises an amino acid sequence of SEQ ID NO:16.

14. The bispecific antibody of any one of claims 1-13, wherein
a. the first polypeptide chain comprises an amino acid sequence of SEQ ID NO:13, and the second polypeptide chain comprises an amino acid sequence of SEQ ID NO:14;
b. the first polypeptide chain comprises an amino acid sequence of SEQ ID NO:17, and the second polypeptide chain comprises an amino acid sequence of SEQ ID NO:18;
c. the first polypeptide chain comprises an amino acid sequence of SEQ ID NO:19, and the second polypeptide chain comprises an amino acid sequence of SEQ ID NO:20;
d. the first polypeptide chain comprises an amino acid sequence of SEQ ID NO:21, and the second polypeptide chain comprises an amino acid sequence of SEQ ID NO:22;
e. the first polypeptide chain comprises an amino acid sequence of SEQ ID NO:23, and the second polypeptide chain comprises the amino acid sequence of SEQ ID NO:24, wherein the bispecific antibody further comprises a third polypeptide chain and a fourth polypeptide chain, the third polypeptide chain comprises an amino acid sequence of SEQ ID NO:25, and the fourth polypeptide chain comprises an amino acid sequence of SEQ ID NO:26;
f. the first polypeptide chain comprises an amino acid sequence of SEQ ID NO:27, and the second polypeptide chain comprises an amino acid sequence of SEQ ID NO:28, wherein the bispecific antibody further comprises a third polypeptide chain and a fourth polypeptide chain, the third polypeptide chain comprises an amino acid sequence of SEQ ID NO:29, and the fourth polypeptide chain comprises an amino acid sequence of SEQ ID NO:30;
g. the first polypeptide chain comprises an amino acid sequence of SEQ ID NO:31, and the second polypeptide chain comprises an amino acid sequence of SEQ ID NO:32, wherein the bispecific antibody further comprises a third polypeptide chain, and the third polypeptide chain comprises an amino acid sequence of SEQ ID NO:33;
h. the first polypeptide chain comprises an amino acid sequence of SEQ ID NO:34, and the second polypeptide chain comprises an amino acid sequence of SEQ ID NO:35, wherein the bispecific antibody further comprises a third polypeptide chain, and the third polypeptide chain comprises an amino acid sequence of SEQ ID NO:36;
i. the first polypeptide chain comprises an amino acid sequence of SEQ ID NO:37, and the second polypeptide chain comprises an amino acid sequence of SEQ ID NO:38, wherein the bispecific antibody further comprises a third polypeptide chain, and the third polypeptide chain comprises an amino acid sequence of SEQ ID NO:39;
j. the first polypeptide chain comprises an amino acid sequence of SEQ ID NO:40, and the second polypeptide chain comprises an amino acid sequence of SEQ ID NO:41, wherein the bispecific antibody further comprises a third polypeptide chain, and the third polypeptide chain comprises an amino acid sequence of SEQ ID NO:42;
k. the first polypeptide chain comprises an amino acid sequence of SEQ ID NO: 43, and the second polypeptide chain comprises an amino acid sequence of SEQ ID NO: 44; wherein the bispecific antibody further comprises a third polypeptide chain and a fourth polypeptide chain, the third polypeptide chain comprises an amino acid sequence of SEQ ID NO: 45, and the fourth polypeptide chain comprises an amino acid sequence of SEQ ID NO: 46.

15. The bispecific antibody of any one of claims 1-14, wherein the bispecific antibody binds to human CD4 and TGF-β1 molecule selectively.

16. The bispecific antibody of any one of claims 1-15, wherein the bispecific antibody binds to human CD4 protein and/or TGF-β1 protein with a Kd less than 10⁻⁹ M; preferably, the Kd is acquired through a Biacore Molecular Interaction System.

17. The bispecific antibody of any one of claims 1-16, wherein the bispecific antibody neutralizes human TGF-β1 with an IC₅₀ less than 5 nM; wherein the IC₅₀ is acquired through TGF-β1 luciferase reporter gene activation measurement assay.

18. The bispecific antibody of claim 17, wherein the bispecific antibody neutralizes human TGF-β1 with the IC₅₀ less than 5 pM; wherein the IC₅₀ is acquired through TGF-β1 luciferase reporter gene activation measurement assay.

19. An isolated polynucleotide, comprising a nucleotide sequence encoding the bispecific antibody of any one of claims 1-18 or a fragment thereof.

20. A vector, comprising the isolated polynucleotide of claim 19.

21. A host cell, comprising a polynucleotide or the vector of claim 20.

22. A method for preparing the bispecific antibody of any one of claims 1-18, comprising culturing the host cells of claim 21 under an appropriate condition, and extracting the bispecific antibody from a cell culture.

23. A conjugation, comprising the bispecific antibody of any one of claims 1-18 and a conjugate reagent, wherein the conjugate reagent is a detectable mark; preferably, the conjugate reagent is a chemical molecule, a fluorescent substance, a luminescent substance, a colored substance, or an enzyme.

24. A pharmaceutical composition, comprising the bispecific antibody of any one of claims 1-18 or the conjugation of claim 23, wherein the pharmaceutical composition further comprises pharmaceutically acceptable excipients.

25. Use of the bispecific antibody of any one of claims 1-18 or the conjugation of claim 23 in the preparation of a medicament for prevention or treatment of a disease, wherein the disease is selected from a group consisting of cancer, immune-mediated diseases, fibrotic diseases, viral infectious diseases, neurodegenerative diseases, bone remodeling, kidney diseases, and a combination thereof.

26. Use of the bispecific antibody of any one of claims 1-18 or the conjugation of claim 23 in the preparation of a medicament for prevention or treatment of malignant tumor; preferably, the malignant tumor is selected from one or more of colon cancer, rectal cancer, lung cancer, kidney cancer, breast cancer, ovarian cancer, prostate cancer, bladder cancer, pancreatic cancer, gastrointestinal cancer, brain cancer, liver cancer, melanoma, and blood cancer.
